(19) Europäisches Patentamt
European Patent Office
Office européen des brevets

(11) **EP 4 372 383 A2**

(12) **EUROPEAN PATENT APPLICATION**

(43) Date of publication:
**22.05.2024 Bulletin 2024/21**

(51) International Patent Classification (IPC):
*G01N 33/68* (2006.01)

(21) Application number: **24169318.3**

(52) Cooperative Patent Classification (CPC):
**C40B 30/04; G01N 33/54353; G01N 33/6803; G16B 35/20**

(22) Date of filing: **20.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2017 US 201762575976 P**

(62) Document number(s) of the earlier application(s) in accordance with Art. 76 EPC:
**18871210.3 / 3 701 066**

(71) Applicant: **Nautilus Subsidiary, Inc.**
**Seattle WA 98102 (US)**

(72) Inventor: **The designation of the inventor has not yet been filed**

(74) Representative: **Marks & Clerk LLP**
**15 Fetter Lane**
**London EC4A 1BW (GB)**

Remarks:
This application was filed on 09-04-2024 as a divisional application to the application mentioned under INID code 62.

(54) **METHODS AND SYSTEMS FOR PROTEIN IDENTIFICATION**

(57) Methods and systems are provided for accurate and efficient identification and quantification of proteins. In an aspect, disclosed herein is a method for iteratively identifying candidate proteins within a sample of unknown proteins, the method comprising receiving information of binding measurements of each of a plurality of affinity reagent probes to the unknown proteins, each affinity reagent probe configured to selectively bind to one or more candidate proteins; comparing at least a portion of the information of binding measurements against a database comprising a plurality of protein sequences, each protein sequence corresponding to a candidate protein; and iteratively generating a probability that each of one or more candidate proteins is present in the sample based on the comparison of the information of binding measurements of the candidate proteins against the database comprising the plurality of protein sequences.

EP 4 372 383 A2

Receive information of binding measurements of each a plurality of affinity reagent probes to the unknown proteins in the sample — 105

↓

Compare information of binding measurements against a database of candidate protein sequences — 110

↓

For each candidate protein, calculate a probability that the candidate protein is present in the sample — 115

100

**FIG. 1**

## Description

### CROSS-REFERENCE

This application claims priority to U.S. Provisional Patent Application No. 62/575,976, filed October 23, 2017, which is entirely incorporated herein by reference.

### BACKGROUND

Current techniques for protein identification typically rely upon either the binding and subsequent readout of highly specific and sensitive affinity reagents (such as antibodies) or upon peptide-read data (typically on the order of 12-30 amino acids long) from a mass spectrometer. Such techniques may be applied to unknown proteins in a sample to determine the presence, absence or quantity of candidate proteins based on analysis of binding measurements of the highly specific and sensitive affinity reagents to the protein of interest.

### SUMMARY

Recognized herein is a need for improved identification and quantification of proteins within a sample of unknown proteins. Methods and systems provided herein can significantly reduce or eliminate errors in identifying proteins in a sample and thereby improve the quantification of said proteins. Such methods and systems may achieve accurate and efficient identification of candidate proteins within a sample of unknown proteins. Such identification may be based on iterative calculations using information of binding measurements of affinity reagent probes configured to selectively bind to one or more candidate proteins. In some embodiments, a sample of unknown proteins may be iteratively exposed to individual affinity reagent probes, pooled affinity reagent probes, or a combination of individual affinity reagent probes and pooled affinity reagent probes. The identification may comprise estimation of a confidence level that each of one or more candidate proteins is present in the sample.

In an aspect, disclosed herein is a computer-implemented method for iteratively identifying each candidate protein within a sample of unknown proteins, the method comprising: (a) receiving, by said computer, information of binding measurements of each of a plurality of affinity reagent probes to said unknown proteins in said sample, each affinity reagent probe configured to selectively bind to one or more candidate proteins among a plurality of candidate proteins; (b) comparing, by said computer, at least a portion of said information of binding measurements against a database comprising a plurality of protein sequences, each protein sequence corresponding to a candidate protein among said plurality of candidate proteins; and (c) for each of one or more candidate proteins in said plurality of candidate proteins, iteratively generating, by said computer, a probability that said each of one or more candidate proteins is present in said sample based on said comparison of said at least a portion of said information of binding measurements of said each of one or more candidate proteins against said database comprising said plurality of protein sequences.

In some embodiments, generating said plurality of probabilities further comprises iteratively receiving additional information of binding measurements of each of a plurality of additional affinity reagent probes, each additional affinity reagent probe configured to selectively bind to one or more candidate proteins among said plurality of candidate proteins. In some embodiments, the method further comprises generating, for said each of one or more candidate proteins, a confidence level that said candidate protein matches one of said unknown proteins in said sample.

In some embodiments, generating said probability comprises taking into account a detector error rate associated with said information of binding measurements. In some embodiments, said detector error rate is obtained from specifications of one or more detectors used to acquire said information of binding measurements. In some embodiments, said detector error rate is set to an estimated detector error rate. In some embodiments, said estimated detector error rate is set by a user of said computer. In some embodiments, said estimated detector error rate is about 0.001. Such an error rate may encompass a physical detector error, which is described elsewhere herein. Alternatively, such an error rate may be attributable to a failure of a probe to "land on" a protein, e.g., when a probe is stuck in the system and not washing out properly, or when a probe binds to a protein that was not expected based on previous qualification and testing of the probes. Hence, the detector error rate may comprise one or more of: physical detector error rate, off-target binding rate, or an error rate due to stuck probes.

In some embodiments, iteratively generating said plurality of probabilities further comprises removing one or more candidate proteins from said plurality of candidate proteins from subsequent iterations, thereby reducing a number of iterations necessary to perform said iterative generation of said probabilities. In some embodiments, removing said one or more candidate proteins is based at least on a predetermined criterion of said binding measurements associated with said candidate proteins. In some embodiments, said predetermined criterion comprises said one or more candidate proteins having binding measurements to a first plurality among said plurality of affinity reagent probes below a predetermined threshold.

In some embodiments, each of said probabilities is normalized to a length of said candidate protein. In some embodiments, each of said probabilities are normalized to a total sum of probabilities of said plurality of candidate proteins. In some embodiments, said plurality of affinity reagent probes comprises no more than 50 affinity reagent probes. In some embodiments, said plurality of affinity reagent probes comprises no more than 100 affinity reagent probes. In some embodiments, said plurality of affinity reagent probes comprises no more than 500 affinity reagent probes.

Recognizing that length of said candidate protein is an approximate proxy for the number of epitopes available in a candidate protein for binding to a particular affinity reagent ("Binding Sites"), in some embodiments, each of the said probabilities is normalized to the total number of Binding Sites available in each of said candidate proteins. In some embodiments, the number of Binding Sites available for each of said candidate proteins is empirically determined with a qualification process. In some embodiments, said qualification process repeatedly measures the binding of an affinity reagent to a particular protein. In some embodiments, said qualification process is performed under condition similar to or identical to the conditions present during said methods and systems of protein identification described herein.

In some embodiments, said probabilities are iteratively generated until a predetermined condition is satisfied. In some embodiments, said predetermined condition comprises generating each of the plurality of probabilities with a confidence of at least 90%. In some embodiments, said predetermined condition comprises generating each of said plurality of probabilities with a confidence of at least 95%. In some embodiments, said predetermined condition comprises generating each of said plurality of probabilities with a confidence of at least 99%.

In some embodiments, the method further comprises generating a paper or electronic report identifying one or more unknown proteins in said sample. In some embodiments, said sample comprises a biological sample. In some embodiments, said biological sample is obtained from a subject. In some embodiments, the method further comprises identifying a disease state in said subject based at least on said plurality of probabilities.

In some embodiments, the method further comprises quantifying proteins in said biological sample by counting the number of identifications made for each protein candidate. In some embodiments, raw protein counts are normalized to correct for sources of error and bias including, but not limited to, detector error, fluorophore intensity, off-target binding by affinity reagents, and protein detectability.

In another aspect, disclosed herein is a computer-implemented method for identifying candidate proteins within a sample of unknown proteins, the method comprising: (a) receiving, by said computer, information of binding measurements of each of a plurality of affinity reagent probes to said unknown proteins in said sample, each affinity reagent probe configured to selectively bind to one or more candidate proteins among a plurality of candidate proteins; (b) comparing, by said computer, at least a portion of said information of binding measurements against a database comprising a plurality of protein sequences, each protein sequence corresponding to a candidate protein among said plurality of candidate proteins; and (c) removing one or more candidate proteins from said plurality of candidate proteins based at least on said comparison of said at least a portion of said information of binding measurements against said database comprising said plurality of protein sequences.

In some embodiments, removing said one or more candidate proteins is based at least on a predetermined criterion of said binding measurements associated with said candidate proteins. In some embodiments, said predetermined criterion comprises said one or more candidate proteins having binding measurements to a first plurality among said plurality of affinity reagent probes below a predetermined threshold. In some embodiments, said plurality of affinity reagent probes comprises no more than 50 affinity reagent probes. In some embodiments, said plurality of affinity reagent probes comprises no more than 100 affinity reagent probes. In some embodiments, said plurality of affinity reagent probes comprises no more than 500 affinity reagent probes.

In some embodiments, the method further comprises generating a paper or electronic report identifying one or more unknown proteins in said sample. In some embodiments, said sample comprises a biological sample. In some embodiments, said biological sample is obtained from a subject. In some embodiments, the method further comprises identifying a disease state in said subject based at least on said identified candidate proteins.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

FIG. 1 illustrates an example flowchart of protein identification of unknown proteins in a biological sample, in accordance with some embodiments.

FIG. 2 illustrates a computer control system that is programmed or otherwise configured to implement methods provided herein.

FIG. 3 illustrates the performance of a censored protein identification vs. an uncensored protein identification approach, in accordance with some embodiments.

FIG. 4 illustrates the tolerance of censored protein identification and uncensored protein identification approaches to random "false negative" binding outcomes, in accordance with some embodiments.

FIG. 5 illustrates the tolerance of censored protein identification and uncensored protein identification approaches to random "false positive" binding outcomes, in accordance with some embodiments.

FIG. 6 illustrates the performance of censored protein identification and uncensored protein identification approaches with overestimated or underestimated affinity reagent binding probabilities, in accordance with some embodiments.

FIG. 7 illustrates the performance of censored protein identification and uncensored protein identification approaches using affinity reagents with unknown binding epitopes, in accordance with some embodiments.

FIG. 8 illustrates the performance of censored protein identification and uncensored protein identification approaches using affinity reagents with missing binding epitopes, in accordance with some embodiments.

FIG. 9 illustrates the performance of censored protein identification and uncensored protein identification approaches using affinity reagents targeting the top 300 most abundant trimers in the proteome, 300 randomly selected trimers in the proteome, or the 300 least abundant trimers in the proteome, in accordance with some embodiments.

FIG. 10 illustrates the performance of censored protein identification and uncensored protein identification approaches using affinity reagents with random or biosimilar off-target sites, in accordance with some embodiments.

FIG. 11 illustrates the performance of censored protein identification and uncensored protein identification approaches using a set of optimal affinity reagents (probes), in accordance with some embodiments.

FIG. 12 illustrates the performance of censored protein identification and uncensored protein identification approaches using unmixed candidate affinity reagents and mixtures of candidate affinity reagents, in accordance with some embodiments.

FIG. 13 illustrates two hybridization steps in reinforcing a binding between an affinity reagent and a protein, in accordance with some embodiments.

## DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

The term "sample," as used herein, generally refers to a biological sample (e.g., a sample containing protein). The samples may be taken from tissue or cells or from the environment of tissue or cells. In some examples, the sample may comprise, or be derived from, a tissue biopsy, blood, blood plasma, extracellular fluid, dried blood spots, cultured cells, culture media, discarded tissue, plant matter, synthetic proteins, bacterial and/or viral samples, fungal tissue, archaea, or protozoans. The sample may have been isolated from the source prior to collection. Samples may comprise forensic evidence. Non-limiting examples include a finger print, saliva, urine, blood, stool, semen, or other bodily fluids isolated from the primary source prior to collection. In some examples, the protein is isolated from its primary source (cells, tissue, bodily fluids such as blood, environmental samples etc) during sample preparation. The sample may be derived from an extinct species including but not limited to samples derived from fossils. The protein may or may not be purified or otherwise enriched from its primary source. In some cases the primary source is homogenized prior to further processing. In some cases, cells are lysed using a buffer such as RIPA buffer. Denaturing buffers may also be used at this stage. The sample may be filtered or centrifuged to remove lipids and particulate matter. The sample may also be purified to remove nucleic acids, or may be treated with RNases and DNases. The sample may contain intact proteins, denatured proteins, protein fragments or partially degraded proteins.

The sample may be taken from a subject with a disease or disorder. The disease or disorder may be an infectious disease, an immune disorder or disease, a cancer, a genetic disease, a degenerative disease, a lifestyle disease, an injury, a rare disease or an age related disease. The infectious disease may be caused by bacteria, viruses, fungi and/or parasites. Non-limiting examples of cancers include Bladder cancer, Lung cancer, Brain cancer, Melanoma, Breast cancer, Non-Hodgkin lymphoma, Cervical cancer, Ovarian cancer, Colorectal cancer, Pancreatic cancer, Esophageal cancer, Prostate cancer, Kidney cancer, Skin cancer, Leukemia, Thyroid cancer, Liver cancer, and Uterine cancer. Some examples of genetic diseases or disorders include, but are not limited to, cystic fibrosis, Charcot-Marie-Tooth disease, Huntington's disease, Peutz-Jeghers syndrome, Down syndrome, Rheumatoid arthritis, and Tay-Sachs disease. Non-limiting examples of lifestyle diseases include obesity, diabetes, arteriosclerosis, heart disease, stroke, hypertension, liver cirrhosis, nephritis, cancer, chronic obstructive pulmonary disease (copd), hearing problems, and chronic backache. Some examples of injuries include, but are not limited to, abrasion, brain injuries, bruising, burns, concussions, congestive heart failure, construction injuries, dislocation, flail chest, fracture, hemothorax, herniated disc, hip pointer, hypothermia, lacerations, pinched nerve, pneumothorax, rib fracture, sciatica, spinal cord injury, tendons ligaments fascia injury, traumatic brain injury, and whiplash. The sample may be taken before and/or after treatment of a subject with a disease or disorder. Samples may be taken before and/or after a treatment. Samples may be taken during a treatment or a treatment regime. Multiple samples may be taken from a subject to monitor the effects of the treatment over time. The sample may be taken from a subject known or suspected of having an infectious disease for which diagnostic antibodies are not available.

The sample may be taken from a subject suspected of having a disease or a disorder. The sample may be taken from a subject experiencing unexplained symptoms, such as fatigue, nausea, weight loss, aches and pains, weakness, or memory loss. The sample may be taken from a subject having explained symptoms. The sample may be taken from a subject at risk of developing a disease or disorder due to factors such as familial history, age, environmental exposure, lifestyle risk factors, or presence of other known risk factors.

The sample may be taken from an embryo, fetus, or pregnant woman. In some examples, the sample may comprise of proteins isolated from the mother's blood plasma. In some examples, proteins isolated from circulating fetal cells in the mother's blood.

The sample may be taken from a healthy individual. In some cases, samples may be taken longitudinally from the same individual. In some cases, samples acquired longitudinally may be analyzed with the goal of monitoring individual health and early detection of health issues. In some embodiments, the sample may be collected at a home setting or at a point-of-care setting and subsequently transported by a mail delivery, courier delivery, or other transport method prior to analysis. For example, a home user may collect a blood spot sample through a finger prick, which blood spot sample may be dried and subsequently transported by mail delivery prior to analysis. In some cases, samples acquired longitudinally may be used to monitor response to stimuli expected to impact healthy, athletic performance, or cognitive performance. Non-limiting examples include response to medication, dieting or an exercise regimen.

Proteins of the sample may be treated to remove modifications that may interfere with epitope binding. For example, the protein may be glycosidase treated to remove post translational glycosylation. The protein may be treated with a reducing agent to reduce disulfide binds within the protein. The protein may be treated with a phosphatase to remove phosphate groups. Other non-limiting examples of post translational modifications that may be removed include acetate, amide groups, methyl groups, lipids, ubiquitin, myristoylation, palmitoylation, isoprenylation or prenylation (e.g., farnesol and geranylgeraniol), farnesylation, geranylgeranylation, glypiation, lipoylation, flavin moiety attachment, phosphopantetheinylation, and retinylidene Schiff base formation. Samples may also be treated to retain posttranslational protein modifications. In some examples, phosphatase inhibitors may be added to the sample. In some examples, oxidizing agents may be added to protect disulfide bonds.

Proteins of the sample may be denatured in full or in part. In some embodiments, proteins can be fully denatured. Proteins may be denatured by application of an external stress such as a detergent, a strong acid or base, a concentrated inorganic salt, an organic solvent (e.g., alcohol or chloroform), radiation or heat. Proteins may be denatured by addition of a denaturing buffer. Proteins may also be precipitated, lyophilized and suspended in denaturing buffer. Proteins may be denatured by heating. Methods of denaturing that are unlikely to cause chemical modifications to the proteins may be preferred.

Proteins of the sample may be treated to produce shorter polypeptides, either before or after conjugation. Remaining proteins may be partially digested with an enzyme such as ProteinaseK to generate fragments or may be left intact. In further examples the proteins may be exposed to proteases such as trypsin. Additional examples of proteases may include serine proteases, cysteine proteases, threonine proteases, aspartic proteases, glutamic proteases, metalloproteases, and asparagine peptide lyases.

In some cases, it may be useful to remove extremely large and small proteins (e.g., Titin), such proteins may be removed by filtration or other appropriate methods. In some examples, extremely large proteins may include proteins that are over 400 kilodalton (kD), 450 kD, 500 kD, 600 kD, 650kD, 700kD, 750kD, 800kD, or 850kD. In some examples, extremely large proteins may include proteins that are over about 8,000 amino acids, about 8,500 amino acids, about 9,000 amino acis, about 9,500 amino acids, about 10,000 amino acids, about 10,500 amino acids, about 11,000 amino acids or about 15,000 amino acids. In some examples, small proteins may include proteins that are less than about 10kD, 9kD, 8kD, 7kD, 6kD, 5kD, 4kD, 3kD, 2kD or 1 kD. In some examples, small proteins may include proteins that are less than about 50 amino acids, 45 amino acids, 40 amino acids, 35 amino acids or about 30 amino acids. Extremely large or small proteins can be removed by size exclusion chromatography. Extremely large proteins may be isolated by size exclusion chromatography, treated with proteases to produce moderately sized polypeptides and recombined with the moderately size proteins of the sample.

Proteins of the sample may be tagged, e.g., with identifiable tags, to allow for multiplexing of samples. Some non-limiting examples of identifiable tags include: fluorophores, magnetic nanoparticles, or DNA barcoded base linkers. Fluorophores used may include fluorescent proteins such as GFP, YFP, RFP, eGFP, mCherry, tdtomato, FITC, Alexa Fluor 350, Alexa Fluor 405, Alexa Fluor 488, Alexa Fluor 532, Alexa Fluor 546, Alexa Fluor 555, Alexa Fluor 568, Alexa Fluor 594, Alexa Fluor 647, Alexa Fluor 680, Alexa Fluor 750, Pacific Blue, Coumarin, BODIPY FL, Pacific Green, Oregon Green, Cy3, Cy5, Pacific Orange, TRITC, Texas Red, Phycoerythrin, Allophcocyanin, or other fluorophores known in the art.

Any number of protein samples may be multiplexed. For example, a multiplexed reaction may contain proteins from 2, 3, 4, 5, 6, 7, 8, 9, 10, 11, 12, 13, 14, 15, 16, 17, 18, 19, about 20, about 25, about 30, about 35, about 40, about 45, about 50, about 55, about 60, about 65, about 70, about 75, about 80, about 85, about 90, about 95, about 100 or more than 100 initial samples. The identifiable tags may provide a way to interrogate each protein as to its sample of origin, or may direct proteins from different samples to segregate to different areas or a solid support. In some embodiments, the proteins are then applied to a functionalized substrate to chemically attach proteins to the substrate.

Any number of protein samples may be mixed prior to analysis without tagging or multiplexing. For example, a multiplexed reaction may contain proteins from 2, 3, 4, 5, 6, 7, 8, 9, 10, 11, 12, 13, 14, 15, 16, 17, 18, 19, about 20, about 25, about 30, about 35, about 40, about 45, about 50, about 55, about 60, about 65, about 70, about 75, about 80, about 85, about 90, about 95, about 100 or more than 100 initial samples. For example, diagnostics for rare conditions may be performed on pooled samples. Analysis of individual samples could then be performed only from samples in a pool that tested positive for the diagnostic. Samples may be multiplexed without tagging using a combinatorial pooling design in which samples are mixed into pools in a manner that allows signal from individual samples to be resolved from the analyzed pools using computational demultiplexing.

The term "substrate," as used herein, generally refers to a substrate capable of forming a solid support. Substrates, or solid substrates, can refer to any solid surface to which proteins can be covalently or non-covalently attached. Non-limiting examples of solid substrates include particles, beads, slides, surfaces of elements of devices, membranes, flow cells, wells, chambers, macrofluidic chambers, microfluidic chambers, channels, microfluidic channels, or any other surfaces. Substrate surfaces can be flat or curved, or can have other shapes, and can be smooth or textured. Substrate surfaces may contain microwells. In some embodiments, the substrate can be composed of glass, carbohydrates such as dextrans, plastics such as polystyrene or polypropylene, polyacrylamide, latex, silicon, metals such as gold, or cellulose, and may be further modified to allow or enhance covalent or non-covalent attachment of the proteins. For example, the substrate surface may be functionalized by modification with specific functional groups, such as maleic or succinic moieties, or derivatized by modification with a chemically reactive group, such as amino, thiol, or acrylate groups, such as by silanization. Suitable silane reagents include aminopropyltrimethoxysilane, aminopropyltriethoxysilane and 4-aminobutyltriethoxysilane. The substrate may be functionalized with N-Hydroxysuccinimide (NHS) functional groups. Glass surfaces can also be derivatized with other reactive groups, such as acrylate or epoxy, using, e.g., epoxysilane, acrylatesilane or acrylamidesilane. The substrate and process for protein attachment are preferably stable for repeated binding, washing, imaging and eluting steps. In some examples, the substrate may be a slide, a flow cell, or a microscaled or nanoscaled structure (e.g., an ordered structure such as microwells, micropillars, single molecule arrays, nanoballs, nanopillars, or nanowires).

The spacing of the functional groups on the substrate may be ordered or random. An ordered array of functional groups may be created by, for example, photolithography, Dip-Pen nanolithography, nanoimprint lithography, nanosphere lithography, nanoball lithography, nanopillar arrays, nanowire lithography, scanning probe lithography, thermochemical lithography, thermal scanning probe lithography, local oxidation nanolithography, molecular self-assembly, stencil lithography, or electron-beam lithography. Functional groups in an ordered array may be located such that each functional group is less than 200 nanometers (nm), or about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 675 nm, about 700 nm, about 725 nm, about 750 nm, about 775 nm, about 800 nm, about 825 nm, about 850 nm, about 875 nm, about 900 nm, about 925 nm, about 950 nm, about 975 nm, about 1000 nm, about 1025 nm, about 1050 nm, about 1075 nm, about 1100 nm, about 1125 nm, about 1150 nm, about 1175 nm, about 1200 nm, about 1225 nm, about 1250 nm, about 1275 nm, about 1300 nm, about 1325 nm, about 1350 nm, about 1375 nm, about 1400 nm, about 1425 nm, about 1450 nm, about 1475 nm, about 1500nm, about 1525 nm, about 1550 nm, about 1575 nm, about 1600 nm, about 1625 nm, about 1650 nm, about 1675 nm, about 1700 nm, about 1725 nm, about 1750 nm, about 1775 nm, about 1800 nm, about 1825 nm, about 1850 nm, about 1875 nm, about 1900 nm, about 1925 nm, about 1950 nm, about 1975 nm, about 2000 nm, or more than 2000 nm from any other functional group. Functional groups in a random spacing may be provided at a concentration such that functional groups are on average at least about 50 nm, about 100 nm, about 150 nm, about 200 nm, about 250 nm, about 300 nm, about 350 nm, about 400 nm, about 450 nm, about 500 nm, about 550 nm, about 600 nm, about 650 nm, about 700 nm, about 750 nm, about 800 nm, about 850 nm, about 900 nm, about 950 nm, about 1000 nm, or more than 100 nm from any other functional group.

The substrate may be indirectly functionalized. For example, the substrate may be PEGylated and a functional group may be applied to all or a subset of the PEG molecules. The substrate may be functionalized using techniques suitable for microscaled or nanoscaled structures (e.g., an ordered structure such as microwells, micropillars, single molecular arrays, nanoballs, nanopillars, or nanowires).

The substrate may comprise any material, including metals, glass, plastics, ceramics or combinations thereof. In some preferred embodiments, the solid substrate can be a flow cell. The flow cell can be composed of a single layer or multiple layers. For example, a flow cell can comprise a base layer (e.g., of boro silicate glass), a channel layer (e.g., of etched silicon) overlaid upon the base layer, and a cover, or top, layer. When the layers are assembled together, enclosed channels can be formed having inlet/outlets at either end through the cover. The thickness of each layer can vary, but is preferably less than about 1700 $\mu$m. Layers can be composed of any suitable material known in the art, including but not limited to photosensitive glasses, borosilicate glass, fused silicate, PDMS or silicon. Different layers can be composed of the same material or different materials.

In some embodiments, flow cells can comprise openings for channels on the bottom of the flow cell. A flow cell can comprise millions of attached target conjugation sites in locations that can be discretely visualized. In some embodiments, various flow cells of use with embodiments of the invention can comprise different numbers of channels (e.g., 1 channel, 2 or more channels, 3 or more channels, 4 or more channels, 6 or more channels, 8 or more channels, 10 or more channels, 12 or more channels, 16 or more channels, or more than 16 channels). Various flow cells can comprise channels of different depths or widths, which may be different between channels within a single flow cell, or different between channels of different flow cells. A single channel can also vary in depth and/or width. For example, a channel can be less than about 50 $\mu$m deep, about 50 $\mu$m deep, less than about 100 $\mu$m deep, about 100 $\mu$m deep, about 100 $\mu$m about 500 $\mu$m deep, about 500 $\mu$m deep, or more than about 500 $\mu$m deep at one or more points within the channel. Channels can have any cross sectional shape, including but not limited to a circular, a semi-circular, a rectangular, a trapezoidal, a triangular, or an ovoid cross-section.

The proteins may be spotted, dropped, pipetted, flowed, washed or otherwise applied to the substrate. In the case of a substrate that has been functionalized with a moiety such as an NHS ester, no modification of the protein is required. In the case of a substrate that has been functionalized with alternate moieties (e.g., a sulfhydryl, amine, or linker DNA), a crosslinking reagent (e.g., disuccinimidyl suberate, NHS, sulphonamides) may be used. In the case of a substrate that has been functionalized with linker DNA the proteins of the sample may be modified with complementary DNA tags. In some cases, the protein may be functionalized so that it may bind to the substrate by electrostatic interaction.

Photo-activatable cross linkers may be used to direct cross linking of a sample to a specific area on the substrate. Photo-activatable cross linkers may be used to allow multiplexing of protein samples by attaching each sample in a known region of the substrate. Photo-activatable cross linkers may allow the specific attachment of proteins which have been successfully tagged, for example, by detecting a fluorescent tag before cross linking a protein. Examples of photo-activatable cross linkers include, but are not limited to, N-5-azido-2-nitrobenzoyloxysuccinimide, sulfosuccinimidyl 6-(4'-azido-2'-nitrophenylamino)hexanoate, succinimidyl 4,4'-azipentanoate, sulfosuccinimidyl 4,4'-azipentanoate, succinimidyl 6-(4,4'-azipentanamido)hexanoate, sulfosuccinimidyl 6-(4,4'-azipentanamido)hexanoate, succinimidyl 2-((4,4'-azipentanamido)ethyl)-1,3'-dithiopropionate, and sulfosuccinimidyl 2-((4,4'-azipentanamido)ethyl)-1,3'-dithiopropionate.

The polypeptides may be attached to the substrate by one or more residues. In some examples, the polypeptides may be attached via the N terminal, C terminal, both terminals, or via an internal residue.

In addition to permanent crosslinkers, it may be appropriate for some applications to use photo-cleavable linkers and that doing so enables proteins to be selectively extracted from the substrate following analysis. In some cases photo-cleavable cross linkers may be used for several different multiplexed samples. In some cases photo-cleavable cross linkers may be used from one or more samples within a multiplexed reaction. In some cases a multiplexed reaction may comprise control samples cross linked to the substrate via permanent crosslinkers and experimental samples cross linked to the substrate via photo-cleavable crosslinkers.

Each conjugated protein may be spatially separated from each other conjugated protein such that each conjugated protein is optically resolvable. Proteins may thus be individually labeled with a unique spatial address. In some embodiments, this can be accomplished by conjugation using low concentrations of protein and low density of attachment sites on the substrate so that each protein molecule is spatially separated from each other protein molecule. In examples where photo-activatable crosslinkers are used a light pattern may be used such that proteins are affixed to predetermined locations.

In some embodiments, each protein may be associated with a unique spatial address. For example, once the proteins are attached to the substrate in spatially separated locations, each protein can be assigned an indexed address, such as by coordinates. In some examples, a grid of pre-assigned unique spatial addresses may be predetermined. In some embodiments the substrate may contain easily identifiable fixed marks such that placement of each protein can be determined relative to the fixed marks of the substrate. In some examples, the substrate may have grid lines and/or and "origin" or other fiducials permanently marked on the surface. In some examples, the surface of the substrate may be permanently or semi-permanently marked to provide a reference by which to locate cross linked proteins. The shape of the patterning itself, such as the exterior border of the conjugated polypeptides may also be used as fiducials for determining the unique location of each spot.

The substrate may also contain conjugated protein standards and controls. Conjugated protein standards and controls may be peptides or proteins of known sequence which have been conjugated in known locations. In some examples, conjugated protein standards and controls may serve as internal controls in an assay. The proteins may be applied to the substrate from purified protein stocks, or may be synthesized on the substrate through a process such as Nucleic Acid-Programmable Protein Array (NAPPA).

In some examples, the substrate may comprise fluorescent standards. These fluorescent standards may be used to calibrate the intensity of the fluorescent signals from assay to assay. These fluorescent standards may also be used to correlate the intensity of a fluorescent signal with the number of fluorophores present in an area. Fluorescent standards may comprise some or all of the different types of fluorophores used in the assay.

Once the substrate has been conjugated with the proteins from the sample, multi-affinity reagent measurements can be performed. The measurement processes described herein may utilize various affinity reagents. In some embodiments, multiple affinity reagents may be mixed together and measurements may be performed on the binding of the affinity reagent mixture to the protein-substrate conjugate.

The term "affinity reagent," as used herein, generally refers to a reagent that binds proteins or peptides with reproducible specificity. For example, the affinity reagents may be antibodies, antibody fragments, aptamers, mini-protein binders, or peptides. In some embodiments, mini-protein binders may comprise protein binders that may be between 30-210 amino acids in length. In some embodiments, mini-protein binders may be designed. In some embodiments, monoclonal antibodies may be preferred. In some examples, antibody fragments such as Fab fragments may be preferred. In some cases, the affinity reagents may be commercially available affinity reagents, such as commercially available antibodies. In some cases, the desired affinity reagents may be selected by screening commercially available affinity reagents to identify those with useful characteristics.

The affinity reagents may have high, moderate, or low specificity. In some examples, the affinity reagents may recognize several different epitopes. In some examples, the affinity reagents may recognize epitopes present in two or more different proteins. In some examples, the affinity reagents may recognize epitopes present in many different proteins. In some cases, an affinity reagent used in the methods of this disclosure may be highly specific for a single epitope. In some cases, an affinity reagent used in the methods of this disclosure may be highly specific for a single epitope containing a post-translational modification. In some cases, affinity reagents may have highly similar epitope specificity. In some cases, affinity reagents with highly similar epitope specificity may be designed specifically to resolve highly similar protein candidate sequences (e.g. candidates with single amino acid variants or isoforms). In some cases, affinity reagents may have highly diverse epitope specificity to maximize protein sequence coverage. In some embodiments, experiments may be performed in replicate with the same affinity probe with the expectation that the results may differ, and thus provide additional information for protein identification, due to the stochastic nature of probe binding to the protein-substrate.

In some cases, the specific epitope or epitopes recognized by an affinity reagent may not be fully known. For example, affinity reagents may be designed or selected for binding specific to one or more whole proteins, protein complexes, or protein fragments without knowledge of a specific binding epitope. Through a qualification process, the binding profile of this reagent may have been elaborated. Even though the specific binding epitope(s) are unknown, binding measurements using said affinity reagent may be used to determine protein identity. For example, a commercially-available antibody or aptamer designed for binding to a protein target may be used as an affinity reagent. Following qualification under assay conditions (e.g., fully folded, partially denaturing, or fully denaturing), binding of this affinity reagent to an unknown protein may provide information about the identity of the unknown protein. In some cases, a collection of protein-specific affinity reagents (e.g., commercially-available antibodies or aptamers) may be used to generate protein identifications, either with or without knowledge of the specific epitopes they target. In some cases, the collection of protein-specific affinity reagents may comprise 50, 100, 200, 300, 400, 500, 600, 700, 800, 900, 1000, 2000, 3000, 4000, 5000, 10000, 20000, or more than 20000 affinity reagents. In some cases, the collection of affinity reagents may comprise all commercially-available affinity reagents demonstrating target-reactivity in a specific organism. For example, a collection of protein-specific affinity reagents may be assayed in series, with binding measurements for each affinity reagent made individually. In some cases, subsets of the protein-specific affinity reagents may be mixed prior to binding measurement. For example, for each binding measurement pass, a new mixture of affinity reagents may be selected comprising a subset of the affinity reagents selected at random from the complete set. For example, each subsequent mixture may be generated in the same random manner, with the expectation that many of the affinity reagents will be present in more than one of the mixtures. In some cases, protein identifications may be generated more rapidly using mixtures of protein-specific affinity reagents. In some cases, such mixtures of protein-specific affinity reagents may increase the percentage of unknown proteins for which an affinity reagent binds in any individual pass. Mixtures of affinity reagents may comprise 1%, 5%, 10%, 20%, 30%, 40%, 50%, 60%, 70%, 80%, 90%, or more than 90% of all available affinity reagents. Mixtures of affinity reagents assessed in a single experiment may or may not share individual affinity reagents in common. In some cases, there may be multiple different affinity reagents within a collection that bind to the same protein. In some cases, each affinity reagent in the collection may bind to a different protein. In cases where multiple affinity reagents with affinity for the same protein bind to a single unknown protein, confidence in the identity of the unknown protein being the common target of said affinity reagents may increase. In some cases, using multiple protein affinity reagents targeting the same protein may provide redundancy in cases where the multiple affinity reagents bind different epitopes on the same protein, and binding of only a subset of the affinity reagents targeting that protein may be interfered with by post-translational modifications or other steric hinderance of a binding epitope. In some cases, binding of affinity reagents for which the binding epitope is unknown may be used in conjunction with binding measurements of affinity reagents for which the binding epitope is known to generate protein identifications.

In some examples, one or more affinity reagents may be chosen to bind amino acid motifs of a given length, such as 2, 3, 4, 5, 6, 7, 8, 9, 10, or more than 10 amino acids. In some examples, one or more affinity reagents may be chosen to bind amino acid motifs of a range of different lengths from 2 amino acids to 40 amino acids.

In some cases, the affinity reagents may be labeled with DNA barcodes. In some examples, DNA barcodes may be used to purify affinity reagents after use. In some examples, DNA barcodes may be used to sort the affinity reagents for repeated uses. In some cases, the affinity reagents may be labeled with fluorophores which may be used to sort the affinity reagents after use.

The family of affinity reagents may comprise one or more types of affinity reagents. For example, the methods of the present disclosure may use a family of affinity reagents comprising one or more of antibodies, antibody fragments, Fab fragments, aptamers, peptides, and proteins.

The affinity reagents may be modified. Modifications include, but are not limited to, attachment of a detection moiety. Detection moieties may be directly or indirectly attached. For example, the detection moiety may be directly covalently attached to the affinity reagent, or may be attached through a linker, or may be attached through an affinity reaction such as complementary DNA tags or a biotin streptavidin pair. Attachment methods that are able to withstand gentle washing and elution of the affinity reagent may be preferred.

Affinity reagents may be tagged, e.g., with identifiable tags, to allow for identification or quantification of binding events (e.g., with fluorescence detection of binding events). Some non-limiting examples of identifiable tags include: fluorophores, fluorescent nanoparticles, quantum dots, magnetic nanoparticles, or DNA barcoded base linkers. Fluorophores used may include fluorescent proteins such as GFP, YFP, RFP, eGFP, mCherry, tdtomato, FITC, Alexa Fluor 350, Alexa Fluor 405, Alexa Fluor 488, Alexa Fluor 532, Alexa Fluor 546, Alexa Fluor 555, Alexa Fluor 568, Alexa Fluor 594, Alexa Fluor 647, Alexa Fluor 680, Alexa Fluor 750, Pacific Blue, Coumarin, BODIPY FL, Pacific Green, Oregon Green, Cy3, Cy5, Pacific Orange, TRITC, Texas Red, Phycoerythrin, Allophcocyanin, or other fluorophores known in the art. Alternatively, affinity reagents may be untagged, such as when binding events are directly detected, e.g., with SPR detection of binding events.

Detection moieties may include, but are not limited to, fluorophores, bioluminescent proteins, DNA segments including a constant region and barcode region, or chemical tethers for linking to a nanoparticle such as a magnetic particle. Detection moieties may include several different fluorophores with different patterns of excitation or emission.

The detection moiety may be cleavable from the affinity reagent. This can allow for a step in which the detection moieties are removed from affinity reagents that are no longer of interest to reduce signal contamination.

In some cases, the affinity reagents are unmodified. For example, if the affinity reagent is an antibody then the presence of the antibody may be detected by atomic force microscopy. The affinity reagents may be unmodified and may be detected, for example, by having antibodies specific to one or more of the affinity reagents. For example, if the affinity reagent is a mouse antibody then the mouse antibody may be detected by using an anti-mouse secondary antibody. Alternately the affinity reagent may be an aptamer which is detected by an antibody specific for the aptamer. The secondary antibody may be modified with a detection moiety as described above. In some cases, the presence of the secondary antibody may be detected by atomic force microscopy.

In some examples, the affinity reagents may comprise the same modification, for example, a conjugated green fluorescent protein, or may comprise two or more different types of modification. For example, each affinity reagent may be conjugated to one of several different fluorescent moieties, each with a different wavelength of excitation or emission. This may allow multiplexing of the affinity reagents as several different affinity reagents may be combined and/or distinguished. In one example, a first affinity reagent may be conjugated to a green fluorescent protein, a second affinity reagent may be conjugated to a yellow fluorescent protein and a third affinity reagent may be conjugated to a red fluorescent protein, thus the three affinity reagents can be multiplexed and identified by their fluorescence. In a further example a first, fourth and seventh affinity reagent may be conjugated to a green fluorescent protein, a second, fifth and eighth affinity reagent may be conjugated to a yellow fluorescent protein and a third, sixth and ninth affinity reagent may be conjugated to a red fluorescent protein; in this case the first, second and third affinity reagents may be multiplexed together while the second, fourth and seventh, and third, sixth and ninth affinity reagents form two further multiplexing reactions. The number of affinity reagents which can be multiplexed together may depend on the detection moieties used to differentiate them. For example, the multiplexing of affinity reagents labeled with fluorophores may be limited by the number of unique fluorophores available. For further example, the multiplexing of affinity reagents labeled with DNA tags may be determined by the length of the DNA bar code.

The specificity of each affinity reagent can be determined prior to use in an assay. The binding specificity of the affinity reagents can be determined in a control experiment using known proteins. Any appropriate experimental methods may be used to determine the specificity of the affinity reagent. In one example a substrate may be loaded with known protein standards at known locations and used to assess the specificity of a plurality of affinity reagents. In another example, a substrate may contain both experimental samples and a panel of controls and standards such that the specificity of each affinity reagent can be calculated from the binding to the controls and standards and then used to identify the experimental samples. In some cases, affinity reagents with unknown specificity may be included along with affinity reagents of known specificity, data from the known specificity affinity reagents may be used to identify proteins, and the pattern of binding of the unknown specificity affinity reagents to the identified proteins may be used to determine their binding specificity. It is also possible to reconfirm the specificity of any individual affinity reagent by using the known binding data of other affinity reagents to assess which proteins the individual affinity reagent bound. In some cases, the frequency of binding of the affinity reagent to each known protein conjugated to the substrate may be used to derive a probability of binding to any of the proteins on the substrate. In some cases, the frequency of binding to known proteins containing an epitope (e.g., an amino acid sequence or post-translational modification) may be used to determine the probability of binding of the affinity reagent to a particular epitope. Thus with multiple uses of an affinity reagent panel the specificities of the affinity reagents may be increasingly refined with each iteration. While affinity reagents that are uniquely specific to particular proteins may be used, methods described herein may not require them. Additionally, methods may be effective on a range of specificities. In some examples, methods described herein may be particularly efficient when affinity reagents are not specific to any particular protein, but are instead specific to amino acid motifs (e.g., the tri-peptide AAA).

In some examples, the affinity reagents may be chosen to have high, moderate, or low binding affinities. In some cases, affinity reagents with low or moderate binding affinities may be preferred. In some cases, the affinity reagents may have dissociation constants of about $10^{-3}$ M, $10^{-4}$ M, $10^{-5}$ M, $10^{-6}$ M, $10^{-7}$ M, $10^{-8}$ M, $10^{-9}$ M, $10^{-10}$M, or less than $10^{-10}$M. In some cases the affinity reagents may have dissociation constants of greater than about $10^{-10}$M, $10^{-9}$ M, $10^{-8}$ M, $10^{-7}$ M, $10^{-6}$ M, $10^{-5}$ M, $10^{-4}$ M, $10^{-3}$ M, $10^{-2}$ M, or greater than $10^{-2}$ M. In some cases, affinity reagents with low or moderate $k_{off}$ rates or moderate or high $k_{on}$ rates may be preferred.

Some of the affinity reagents may be chosen to bind modified amino acid sequences, such as phosphorylated or ubiquitinated amino acid sequences. In some examples, one or more affinity reagents may be chosen to be broadly specific for a family of epitopes that may be contained by one or more proteins. In some examples, one or more affinity reagents may bind two or more different proteins. In some examples, one or more affinity reagents may bind weakly to their target or targets. For example, affinity reagents may bind less than 10%, less than 10%, less than 15%, less than 20%, less than 25%, less than 30%, or less than 35% to their target or targets. In some examples, one or more affinity reagents may bind moderately or strongly to their target or targets. For example, affinity reagents may bind more than 35%, more than 40%, more than 45%, more than 60%, more than 65%, more than 70%, more than 75%, more than 80%, more than 85%, more than 90%, more than 91%, more than 92%, more than 93%, more than 94%, more than 95%, more than 96%, more than 97%, more than 98%, or more than 99% to their target or targets.

To compensate for weak binding, an excess of the affinity reagent may be applied to the substrate. The affinity reagent may be applied at about a 1:1, 2:1, 3:1, 4:1, 5:1, 6:1, 7:1, 8:1, 9:1 or 10:1 excess relative to the sample proteins. The affinity reagent may be applied at about a 1:1, 2:1, 3:1, 4:1, 5:1, 6:1, 7:1, 8:1, 9:1 or 10:1 excess relative to the expected incidence of the epitope in the sample proteins.

To compensate for high affinity reagent dissociation rates, a linker moiety may be attached to each affinity reagent and used to reversibly link bound affinity reagents to the substrate or unknown protein to which it binds. For example, a DNA tag could be attached to the end of each affinity reagent and a different DNA tag attached to the substrate or each unknown protein. After the affinity reagent is hybridized with the unknown proteins, a linker DNA complementary to the affinity reagent-associated DNA tag on one end and the substrate-associated tag on the other could be washed over the chip to bind the affinity reagent to the substrate and prevent the affinity reagent from dissociating prior to measurement. After binding, the linked affinity reagent may be released by washing in the presence of heat or high salt concentration to disrupt the DNA linker bond.

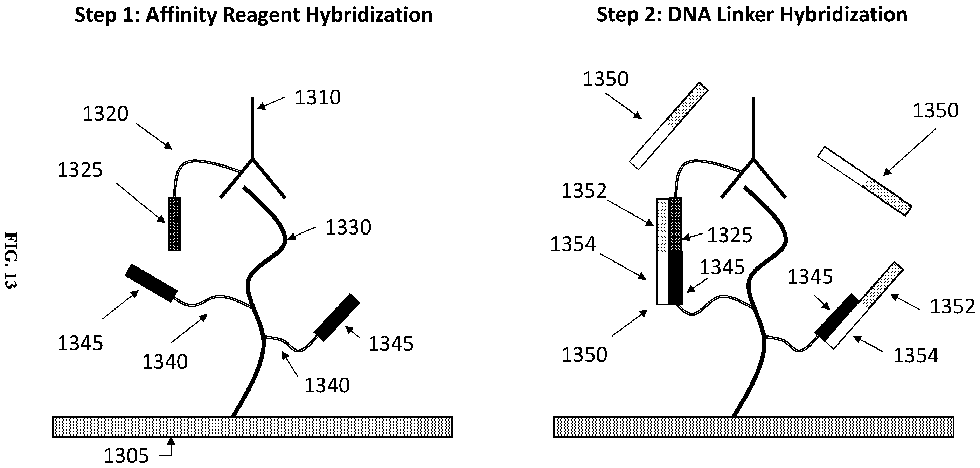

FIG. 13 illustrates two hybridization steps in reinforcing a binding between an affinity reagent and a protein, in accordance with some embodiments. In particular, Step 1 of FIG. 13 illustrates an affinity reagent hybridization. As seen in Step 1, affinity reagent 1310 hybridizes to protein 1330. Protein 1330 is bound to a slide 1305. As seen in Step 1, affinity reagent 1310 has a DNA tag 1320 attached. In some embodiments, an affinity reagent may have more than one DNA tag attached. In some embodiments, an affinity reagent may have 1, 2, 3, 4, 5, 6, 7, 8, 9, 10, 11, 12, 13, 14, 15, 16, 17, 18, 19, 20, or more than 20 DNA tags attached. DNA tag 1320 comprises an ssDNA tag having a recognition sequence 1325. Additionally, protein 1330 has two DNA tags 1340. In some embodiments, DNA tags may be added using chemistry that reacts with cysteines in a protein. In some embodiments, a protein may have more than one DNA tag attached. In some embodiments, a protein may have 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, or more than 100 DNA tags attached. Each DNA tag 1340 comprises an ssDNA tag having a recognition sequence 1345.

As seen in Step 2, DNA linker 1350 hybridizes to DNA tags 1320 and 1340 attached to affinity reagent 1310 and protein 1330, respectively. DNA linker 1350 comprises ssDNA having complementary sequences to recognition sequences 1325 and 1345, respectively. Further, recognition sequences 1325 and 1345 are situated on DNA linker 1350 so as to allow for DNA linker 1350 to bind to both DNA tags 1320 and 1340 at the same time, as illustrated in Step 2. In particular, a first region 1352 of DNA linker 1350 selectively hybridizes to recognition sequence 1325 and a second region 1354 of DNA linker 1350 selectively hybridizes to recognition sequence 1345. In some embodiments, first region 1352 and second region 1354 may be spaced apart from each other on the DNA linker. In particular, in some embodiments a first region of a DNA linker and a second region of a DNA linker may be spaced apart with a non-hybridizing spacer sequence between the first region and the second region. Further, in some embodiments, a sequence of recognition sequence may be less than fully complementary to a DNA linker and may still bind to the DNA linker sequence. In some embodiments a length of a recognition sequence may be less than 5 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, 30 nucleotides, or more than 30 nucleotides. In some embodiments, a recognition sequence may have one or more mismatches to a complementary DNA tag sequence. In some embodiments, approximately 1 in 10 nucleotides of a recognition sequence may be mismatched with a complementary DNA tag sequence and may still hybridize with the complementary DNA tag sequence. In some embodiments, less than 1 in 10 nucleotides of a recognition sequence may be mismatched with a complementary DNA tag sequence and may still hybridize with the complementary DNA tag sequence. In some embodiments, approximately 2 in 10 nucleotides of a recognition sequence may be mismatched with a complementary DNA tag sequence and may still hybridize with the complementary DNA tag sequence. In some embodiments, more than 2 in 10 nucleotides of a recognition sequence may be mismatched with a complementary DNA tag sequence and may still hybridize with the complementary DNA tag sequence.

The affinity reagents may also comprise a magnetic component. The magnetic component may be useful for manipulating some or all bound affinity reagents into the same imaging plane or z stack. Manipulating some or all affinity reagents into the same imaging plane may improve the quality of the imaging data and reduce noise in the system.

The term "detector," as used herein, generally refers to a device that is capable of detecting a signal, including a signal indicative of the presence or absence of a binding event of an affinity reagent to a protein. The signal may be a direct signal indicative of the presence or absence of a binding event, such as a surface plasmon resonance (SPR) signal. The signal may be an indirect signal indicative of the presence or absence of a binding event, such as a fluorescent signal. In some cases, a detector can include optical and/or electronic components that can detect signals. The term "detector" may be used in detection methods. Non-limiting examples of detection methods include optical detection, spectroscopic detection, electrostatic detection, electrochemical detection, magnetic detection, fluorescence detection, surface plasmon resonance (SPR), and the like. Optical detection methods include, but are not limited to, fluorimetry and UV-vis light absorbance. Spectroscopic detection methods include, but are not limited to, mass spectrometry, nuclear magnetic resonance (NMR) spectroscopy, and infrared spectroscopy. Electrostatic detection methods include, but are not limited to, gel based techniques, such as, for example, gel electrophoresis. Electrochemical detection methods include, but are not limited to, electrochemical detection of amplified product after high-performance liquid chromatography separation of the amplified products.

## Protein identification in a sample

Proteins are vital building blocks of cells and tissues of living organisms. A given organism produces a large set of different proteins, typically referred to as the proteome. The proteome may vary with time and as a function of various stages (e.g., cell cycle stages or disease states) that a cell or organism undergoes. A large-scale study (e.g., experimental analysis) of proteomes may be referred to as proteomics. In proteomics, multiple methods exist to identify proteins, including immunoassays (e.g., enzyme-linked immunosorbent assay (ELISA) and Western blot), mass spectroscopy-based methods (e.g., matrix-assisted laser desorption/ionization (MALDI) and electrospray ionization (ESI)), hybrid methods (e.g., mass spectrometric immunoassay (MSIA)), and protein microarrays. For example, single-molecule proteomics methods may attempt to infer the identity of protein molecules in a sample by diverse approaches, ranging from direct functionalization of amino acids to using affinity reagents. The information or measurements gathered from such approaches are typically analyzed by a suitable algorithm to identify the proteins present in the sample.

Accurate quantification of proteins may also encounter challenges owing to lack of sensitivity, lack of specificity, and detector noise. In particular, accurate quantification of proteins in a sample may encounter challenges owing to random and unpredictable systematic variations in signal level of detectors, which can cause errors in identifying and quantifying proteins. In some cases, instrument and detection systematics can be calibrated and removed by monitoring instrument diagnostics and common-mode behavior. However, binding of proteins (e.g., by affinity reagent probes) is inherently a probabilistic process with less than ideal sensitivity and specificity of binding.

The present disclosure provides methods and systems for accurate and efficient identification of proteins. Methods and systems provided herein can significantly reduce or eliminate errors in identifying proteins in a sample. Such methods and systems may achieve accurate and efficient identification of candidate proteins within a sample of unknown proteins. The protein identification may be based on iterative calculations using information of binding measurements of affinity reagent probes configured to selectively bind to one or more candidate proteins. The protein identification may be optimized to be computable within a minimal memory footprint. The protein identification may comprise generating a confidence level that each of one or more candidate proteins is present in the sample.

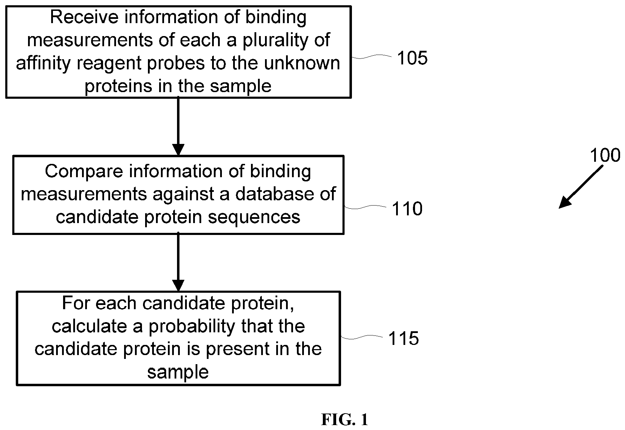

In an aspect, disclosed herein is a computer-implemented method **100** for iteratively identifying candidate proteins within a sample of unknown proteins (e.g., as illustrated in **FIG. 1**). The method may comprise receiving, by the computer, information of binding measurements of each of a plurality of affinity reagent probes to the unknown proteins in the sample (e.g., step **105**). In some embodiments, a plurality of affinity reagent probes may comprise a pool of a plurality of individual affinity reagent probes. For example, a pool of affinity reagent probes may comprise 2, 3, 4, 5, 6, 7, 8, 9, 10, or more than 10 types of affinity reagent probes. In some embodiments, a pool of affinity reagent probes may comprise 2 types of affinity reagent probes that combined make up a majority of the composition of the affinity reagent probes in the pool of affinity reagent probes. In some embodiments, a pool of affinity reagent probes may comprise 3 types of affinity reagent probes that combined make up a majority of the composition of the affinity reagent probes in the pool of affinity reagent probes. In some embodiments, a pool of affinity reagent probes may comprise 4 types of affinity reagent probes that combined make up a majority of the composition of the affinity reagent probes in the pool of affinity reagent probes. In some embodiments, a pool of affinity reagent probes may comprise 5 types of affinity reagent probes that combined make up a majority of the composition of the affinity reagent probes in the pool of affinity reagent probes. In some embodiments, a pool of affinity reagent probes may comprise more than 5 types of affinity reagent probes that combined make up a majority of the composition of the affinity reagent probes in the pool of affinity reagent probes. Each of the affinity reagent probes may be configured to selectively bind to one or more candidate proteins among the plurality of candidate proteins. The affinity reagent probes may be k-mer affinity reagent probes. In some embodiments, each k-mer affinity reagent probe is configured to selectively bind to one or more candidate proteins among a plurality of candidate proteins. The information of binding measurements may comprise a set of probes that are believed to have bound to an unknown protein.

Next, at least a portion of the information of binding measurements may be compared, by the computer, against a database comprising a plurality of protein sequences (e.g., step **110**). Each of the protein sequences may correspond to a candidate protein among the plurality of candidate proteins. The plurality of candidate proteins may comprise at least 10, at least 20, at least 30, at least 40, at least 50, at least 60, at least 70, at least 80, at least 90, at least 100, at least 150, at least 200, at least 250, at least 300, at least 350, at least 400, at least 450, at least 500, at least 600, at least 700, at least 800, at least 900, at least 1000, or more than 1000 different candidate proteins.

Next, for each of one or more candidate proteins in the plurality of candidate proteins, a probability that the candidate protein is present in the sample may be calculated or generated, by the computer (e.g., step **115**). The calculation or generation may be performed iteratively. Alternatively, the calculation or generation may be performed non-iteratively. The probability may be iteratively generated based on the comparison of the information of binding measurements of the candidate proteins against the database comprising the plurality of protein sequences. Thus, the input to the algorithm may comprise a database of protein sequences and a set of probes that are believed to have bound to an unknown protein. The output of the algorithm may comprise the probability that each protein in the database may be present in the sample.

In some embodiments, the output probability calculated in step **115** may be expressed as: P(protein_i | probes[1, 2, ..., n], length(protein_i)). This value gives the probability that a given protein (protein_i) is present in the sample, given the set of probes [1, 2, ..., n] that bound to protein_i and the length of protein_i (e.g., in number of peptides).

In some embodiments, calculating the output probability may comprise finding a product of probabilities that one or more affinity reagents (probes) landed on the protein. For example, if n probes have been detected to be bound to the protein, then the probability of each different probe landing on the protein may be expressed as P_landing_probe_1, P_landing_probe_2, ..., P_landing_probe_n. Thus, the product of probabilities that one or more affinity reagents (probes) landed on the protein may be expressed as Product(P_landing_probe_1, P_landing_probe_2, ..., P_landing_probe_n).

In some embodiments, calculating the output probability may comprise normalizing the product of probabilities that one or more affinity reagents (probes) landed on the protein by a length factor. The length factor may take into account an assumption that lengthy (e.g., longer) proteins are more likely at random to have a larger number of affinity reagents that bind (e.g., land on), compared to less lengthy (e.g., shorter) proteins. The length factor may be expressed as an n-combination of a set of cardinality $Len\_i$ (denoting the length of protein_i), or the binomial coefficient "$Len\_i$ choose n", which may be denoted by $Choose(Len\_i, n)$. The length factor represents the number of different ways to choose a subset of size n elements (e.g., a number of probes that land on the protein), disregarding their order, from a set of $Len\_i$ elements (e.g., a protein of length i). Thus, the product of probabilities that one or more affinity reagents (probes) landed on the protein, normalized or divided by the length factor, may be expressed as: $[Product(P\_landing\_probe\_1, P\_landing\_probe\_2, ..., P\_landing\_probe\_n) / Choose(Len\_i, n)]$. This value may also be referred to as the un-normalized probability of protein_i being present in the sample.

Recognizing that length of said candidate protein is an approximate proxy for the number of epitopes available in a candidate protein for binding to a particular affinity reagent ("Binding Sites"), in some embodiments, calculating the output probability may comprise normalizing of each said probabilities to the total number of Binding Sites available in each of said candidate proteins. In some embodiments, the number of Binding Sites available for each of said candidate proteins is empirically determined with a qualification process. In some embodiments, said qualification process repeatedly measures the binding of an affinity reagent to a particular protein. In some embodiments, said qualification process is performed under condition similar to or identical to the conditions present during said methods and systems of protein identification described herein.

In some embodiments, calculating the output probability may comprise normalizing the un-normalized probability of protein_i being present in the sample. The normalization may comprise dividing by a sum of all un-normalized probabilities across all proteins in the database (e.g., the plurality of candidate proteins). For example, the sum of all un-normalized probabilities across all proteins j in the database (e.g., the plurality of candidate proteins) may be expressed as $SUM(P(protein\_j | probes[1, ..., n], length(proteinj))$. Thus, the normalized probability of protein i being present in the sample may be expressed as:

$$P(protein\_i | probes[1,2 ..., n], length(protein\_i)) = [Product(P\_landing\_probe\_1, P\_landing\_probe\_2, ..., P\_landing\_probe\_n) / Choose(Len\_i, n)] / SUM(P(protein\_j | probes[1, ..., n], length(protein\_j)))$$

In some embodiments, generating the plurality of probabilities further comprises iteratively receiving additional information of binding measurements of each of a plurality of additional affinity reagent probes. Each of the additional affinity reagent probes may be configured to selectively bind to one or more candidate proteins among the plurality of candidate proteins. For example, a first value of output probability may be generated for each candidate protein based on two landing probes, as given by:

$$P(protein\_i | probes[1, 2], length(protein\_i)) = [Product(P\_landing\_probe\_1, P\_landing\_probe\_2) / Choose(Len\_i, 2)] / SUM(P(protein\_j | probes[1,2], length(protein\_j))).$$

Next, additional information of binding measurements of each of a plurality of additional affinity reagent probes may be iteratively received and iteratively calculated as a subsequent iterated value of output probability, thereby generating a second value of output probability. For example, the second value of output probability may be generated for each candidate protein based on the first two landing probes (probes 1 and 2) and the second two landing probes (probes 3 and 4), as given by:

$$P(protein\_i | probes[1, 2, 3, 4], length(protein\_i)) = [Product(P\_landing\_probe\_1, P\_landing\_probe\_2, P\_landing\_probe\_3, P\_landing\_probe\_4) / Choose(Len\_i, 4)] / SUM(P(protein\_j | probes[1, 2, 3, 4], length(protein\_j)))$$

In some embodiments, the output probability calculated or generated in step 115 is a probability that a binding measurement on the candidate protein would generate an observed measurement outcome. The term "binding measurement outcome," as used herein, refers to the information observed on performing a binding measurement. For example, the binding measurement outcome of an affinity reagent binding experiment may be either binding or non-binding of the reagent. Additionally, or alternatively, for each of one or more candidate proteins in the plurality of candidate proteins, a probability that a binding measurement on the candidate protein would not generate an observed measurement outcome, may be calculated or generated by the computer. Additionally, or alternatively, a probability that a binding measurement on the candidate protein would generate an unobserved measurement outcome, may be calculated or generated by the computer. Additionally, or alternatively, a probability that a series of binding measurements on the candidate protein would generate an outcome set may be calculated or generated, by the computer.

"Binding outcome set," as used herein, refers to a plurality of independent Binding measurement outcomes for a protein. For example, a series of empirical affinity reagent binding measurements may be performed on an unknown protein. The binding measurement of each individual affinity reagent comprises a binding measurement outcome, and the set of all binding measurement outcomes is the binding outcome set. In some cases, the binding outcome set may be a subset of all observed binding outcomes. In some cases, the binding outcome set may comprise binding measurement outcomes that were not empirically observed.

Additionally or alternatively, for each of one or more candidate proteins in the plurality of candidate proteins, a probability that the unknown protein is the candidate protein, may be calculated or generated, by the computer.

The probabilities in step **115** may be generated based on the comparison of the binding measurement outcomes of the unknown proteins against the database comprising the plurality of protein sequences for all candidate proteins. Thus, the input to the algorithm may comprise a database of candidate protein sequences and a set of binding measurements (e.g., probes that are believed to have bound to an unknown protein). In some cases, the input to the algorithm may comprise parameters relevant to estimating the probability of any of the affinity reagents generating any binding measurement for any of the candidate proteins (e.g. trimer-level binding probabilities for each affinity reagent). The output of the algorithm may comprise a probability that a binding measurement outcome or binding outcome set is observed, given a hypothesized candidate protein identity. Additionally or alternatively, the output of the algorithm may comprise the most probable identity, selected from the set of candidate proteins, for the unknown protein and the probability of that identification being correct given a binding measurement outcome or binding outcome set. Additionally or alternatively, the output of the algorithm may comprise a group of high-probability candidate protein identities and an associated probability that the unknown protein is one of the proteins in the group. The probability that the binding measurement outcome is observed, given that a candidate protein is the protein being measured, may be expressed as: P(binding measurement outcome | protein).

In some embodiments, P(binding measurement outcome | protein) is calculated completely *in silico.* In some embodiments, P(binding measurement outcome | protein) is calculated based on, or derived from, features of the amino acid sequence of the protein. In some embodiments, P(binding measurement outcome | protein) is calculated independent of knowledge of the amino acid sequence of the protein. For example, P(binding measurement outcome | protein) may be determined empirically by acquiring the binding measurements in replicate experiments on an isolate of the protein candidate, and calculating the P(binding measurement outcome | protein) from the frequency: (number of binding measurements with outcome, divided by the total number of binding measurements). In some embodiments, P(binding measurement outcome | protein) is calculated based on, or derived from, a database of past binding measurements on the protein. In some embodiments P(binding measurement outcome | protein) is calculated based on, or derived from, generating a set of confident protein identifications from a collection of unknown proteins with the results of the binding measurement censored, and then calculating the frequency of the binding measurement outcome among the set of unknown proteins that were confidently identified as the candidate protein.

In some embodiments, a collection of unknown proteins may be identified using a seed value of P(binding measurement outcome | protein), and the seed value may be refined based on the frequency of the binding measurement outcome among unknown proteins confidently matched to the candidate protein. In some embodiments, this process is repeated, with new identifications generated based on updated binding measurement outcome probabilities, and then new binding measurement outcome probabilities may be generated from the updated set of confident identifications. In some embodiments, the parameters of an *in silico* model to predict binding measurement outcome probability for one or more proteins are learned or updated based on observed binding measurement outcomes among unknown proteins that are confidently identified. In some embodiments, this process is repeated, with new identifications generated based on the updated *in silico* model, and then new measurement outcome probabilities may be generated from the updated *in silico* model.

The probability that the binding measurement outcome is not observed, given that a candidate protein is the protein being measured, may be expressed as:

$$P(\text{not binding measurement outcome} \mid \text{protein}) = 1 - P(\text{binding measurement outcome} \mid \text{protein}).$$

The probability that a binding measurement outcome set consisting of N individual binding measurement outcomes is observed, given that a candidate protein is the protein being measured, may be expressed as a product of the probabilities for each individual binding measurement outcome:

P(binding outcome set | protein) = P(binding measurement outcome 1 | protein) * P(binding measurement outcome 2 | protein) * . . . * P(binding measurement outcome N | protein)

The probability of the unknown protein being a candidate protein (protein$_i$), may be calculated based on the probability of the binding outcome set for each possible candidate protein.

In some embodiments, the probability of the unknown protein being a candidate protein (protein$_i$), is calculated as the fraction of the summed probability of observing the binding outcome set for each candidate protein j of the complete set of N candidate proteins:

$$P(protein_i \mid binding\ outcome\ set) = \frac{P(binding\ outcome\ set \mid protein_i)}{\sum_{j=1}^{j=N} P(binding\ outcome\ set \mid protein_j)}$$

In some embodiments, the binding measurement outcome set comprises binding of affinity reagent probes. In some embodiments, the binding measurement outcome set comprises non-specific binding of affinity reagent probes.

In some embodiments, the method further comprises applying the method to all unknown proteins measured in the sample. In some embodiments, the method further comprises generating, for each of the one or more candidate proteins, a confidence level that the candidate protein matches one of the unknown proteins in the sample. The confidence level may comprise a probability value. Alternatively, the confidence level may comprise a probability value with an error. Alternatively, the confidence level may comprise a range of probability values, optionally with a confidence (about 90%, about 95%, about 96%, about 97%, about 98%, about 99%, about 99.9%, about 99.99%, about 99.999%, about 99.9999%, about 99.99999%, about 99.999999%, about 99.9999999%, about 99.99999999%, about 99.999999999%, about 99.9999999999%, about 99.99999999999%, about 99.999999999999%, about 99.9999999999999% confidence or above 99.9999999999999% confidence).

In some embodiments, the method further comprises generating protein identifications, and associated probabilities, independently for each unknown protein in the sample, and generating a list of all unique proteins identified in the sample. In some embodiments, the method further comprises counting the number of identifications generated for each unique candidate protein to determine the quantity of each candidate protein in the sample. In some embodiments, a collection of protein identifications and associated probabilities may be filtered to only contain identifications of a high score, high confidence, and/or low false discovery rate.

In some embodiments, binding probabilities may be generated for affinity reagents to full-length candidate proteins. In some embodiments, binding probabilities may be generated for affinity reagents to protein fragments (e.g., a subsequence of the complete protein sequence). For example, if unknown proteins were processed and conjugated to the substrate in a manner such that only the first 100 amino acids of each unknown protein were conjugated, binding probabilities may be generated for each protein candidate such that all binding probabilities for epitope binding beyond the first 100 amino acids are set to zero, or alternatively to a very low probability representing an error rate. A similar approach may be used if the first 10, 20, 50, 100, 150, 200, 300, 400, or more than 400 amino acids of each protein are conjugated to the substrate. A similar approach may be used if the last 10, 20, 50, 100, 150, 200, 300, 400, or more than 400 amino acids are conjugated to the substrate.

In some embodiments, where proteins may have been treated to generate fragments prior to or after conjugation, the fragmentation of each protein may not be deterministic. For example, proteins may be physically sheared prior to substrate conjugation. In such cases, binding probabilities of affinity reagents may be jointly modeled with protein fragment identity (e.g., the start and the stop of the subsequence of the complete protein candidate comprising the fragment). For example, an expectation maximization approach may be used when generating binding probabilities for each protein candidate, which iteratively refines the estimation of the most likely fragment generated by the protein candidate based on the observed binding measurements, and in turn updates the probability of binding of each affinity reagent to the modeled protein fragment.

In some cases, modeling of the protein fragment may incorporate prior knowledge on the likelihood of generating particular fragments from a protein candidate. For example, a prior knowledge on the expected length distribution of protein fragments may be imposed. As another example, a prior knowledge favoring protein fragments flanked by lysine or arginine may be imposed if the intact proteins were treated with the trypsin enzyme prior to conjugation. In some embodiments, the database of candidate protein sequences against which binding measurements are compared may comprise protein fragments. For example, if a peptide mixture resulting from a tryptic digest of the source sample were conjugated to the substrate, the protein candidate list may comprise every fully tryptic peptide generated from an *in silico* digest of a database of intact protein sequences. In such cases, the results from affinity reagent binding measurements may be used to identify the most likely tryptic peptide for each unknown protein fragment in the sample. In such cases, the resulting peptide identities and/or quantities may be converted to protein-level measurements using protein inference approaches, of which numerous examples exist, e.g., in the field of mass spectrometry.

In some embodiments, in cases where a single protein candidate match cannot be assigned to an unknown protein, a group of potential protein candidate matches may be assigned to the unknown candidate. A confidence level may be assigned to the unknown protein being one of any of the protein candidates in the group. The confidence level may comprise a probability value. Alternatively, the confidence level may comprise a probability value with an error. Alternatively, the confidence level may comprise a range of probability values, optionally with a confidence (e.g. about 90%, about 95%, about 96%, about 97%, about 98%, or about 99% confidence). For example, an unknown protein may match strongly with two protein candidates. The two protein candidates may have high sequence similarity (e.g. protein isoforms, proteins with single amino acid variants compared to a canonical sequence). In these cases, no individual protein candidate may be assigned with high confidence, but a high confidence may be ascribed to the unknown protein matching to a single, but unknown, member of the "protein group" comprising the two strongly matching protein candidates.

In some embodiments, efforts may be made to detect cases where unknown proteins are not optically-resolved. For example, on rare occasion, two or more proteins may bind in the same "well" or location of a substrate despite efforts to prevent this from happening. In some cases, the conjugated proteins may be treated with a non-specific dye and the signal from the dye measured. In cases where two or more proteins are not optically-resolved, the signal resulting from the dye will be higher than locations containing a single protein and be used to flag locations with multiple bound proteins.

In some embodiments, the plurality of candidate proteins is generated or modified by sequencing or analyzing the DNA or RNA of the human or organism from which the sample of unknown proteins is obtained or derived.

In some embodiments, the method further comprises deriving information on post-translational modifications of the unknown protein. The information on post-translational modifications may comprise the presence of a post-translational modifications without knowledge of the nature of the specific modification. The database may be considered to be an exponential product of PTMs. For example, once a protein candidate sequence has been assigned to an unknown protein, the pattern of affinity reagent binding for the assayed protein may be compared to a database containing binding measurements for the affinity reagents to the same candidate from previous experiments. For example, a database of binding measurements may be derived from binding to a Nucleic Acid Programmable Protein Array (NAPPA) containing unmodified proteins of known sequence at known locations.

Alternatively, a database of binding measurements may be derived from previous experiments in which protein candidate sequences were confidently assigned to unknown proteins. Discrepancies in binding measurements between the assayed protein and the database of existing measurements may provide information on the likelihood of post-translation modification. For example, if an affinity agent has a high frequency of binding to the candidate protein in the database, but does not bind the assayed protein, there is a higher likelihood of a post-translational modification being present somewhere on the protein. If the binding epitope is known for the affinity reagent for which there is a binding discrepancy, the location of the post translational modification may be localized to at or near the binding epitope of the affinity reagent. In some embodiments, information on specific post-translational modifications may be derived by performing repeated affinity reagent measurements before and after treatment of the protein-substrate conjugate with an enzyme that specifically removes the particular post translational modification. For example, binding measurements may be acquired for a sequence of affinity reagents prior to treatment of the substrate with a phosphatase, and then repeated after treatment with a phosphatase. Affinity reagents which bind an unknown protein prior to phosphatase treatment but not after phosphatase treatment (differential binding) provide evidence of phosphorylation. If the epitope recognized by the differentially binding affinity reagent is known, the phosphorylation may be localized to at or near the binding epitope for the affinity reagent.

In some cases, the count of a particular post-translational modification may be determined using binding measurements with an affinity reagent against a particular post-translational modification. For example, an antibody that recognizes phosphorylation events may be used as an affinity reagent. The binding of this reagent may indicate the presence of at least one phosphorylation on the unknown protein. In some cases, the number of discrete post-translational modifications of a particular type on an unknown protein may be determined by counting the number of binding events measured for an affinity reagent specific to the particular post-translational modification. For example, a phosphorylation specific antibody may be conjugated to a fluorescent reporter. In this case, the intensity of the fluorescent signal may be used to determine the number of phosphorylation-specific affinity reagents bound to an unknown protein. The number of phosphorylation-specific affinity reagents bound to the unknown protein may in turn be used to determine the number of phosphorylation sites on the unknown protein. In some embodiments, evidence from affinity reagent binding experiments may be combined with pre-existing knowledge of amino acid sequence motifs or specific protein locations likely to be post-translationally modified (e.g., from dbPTM, PhosphoSitePlus, or UniProt) to derive more accurate count, identification, or localization of post-translational modification. For example, if the location of a post-translational modification is not exactly determined from affinity measurements alone, a location containing an amino acid sequence motif frequently associated with the post translational modification of interest may be favored.

In some embodiments, generating the probability comprises taking into account a detector error rate associated with the information of binding measurements. The detector error rate may comprise a true landing rate. For example, the detector error rate may be attributable to a failure of a probe to "land on" a protein, e.g., when a probe is stuck in the system and not washing out properly, or when a probe binds to a protein that was not expected based on previous qualification and testing of the probes. Alternatively, the detector error rate may be attributable to the detector's physical error, and may be obtained from specifications of one or more detectors used to acquire the information of binding measurements. The detector error rate may comprise one or more of: physical detector error rate, off-target binding rate, or an error rate due to stuck probes. In some embodiments, the detector error rate is set to an estimated detector error rate. Alternatively, the estimated detector error rate may be set by a user of the computer. In some embodiments, the estimated detector error rate is about 0.0001, about 0.0002, about 0.0003, about 0.0004, about 0.0005, about 0.0006, about 0.0007, about 0.0008, about 0.0009, about 0.001, about 0.002, about 0.003, about 0.004, about 0.005, about 0.006, about 0.007, about 0.008, about 0.009, about 0.01, about 0.02, about 0.03, about 0.04, about 0.05, about 0.06, about 0.07, about 0.08, about 0.09, about 0.1, or greater than about 0.1.

A hit table may be generated, such that each of the columns of the hit table represents a different protein (e.g., with a different length) and/or each of the rows of the hit table represents a different probe. Each value of a given element of the hit table (e.g., at row j and column i) may comprise a value indicative of whether or not a given probe j exposed to the sample can bind to a given protein i. For example, the hit table element can be set to 1 (e.g., at row j and column i) if probe j can bind to protein i, and 0 otherwise. This information may arrive incrementally, and therefore the hit table may be computed iteratively.

From the hit table, a probability matrix may be calculated or generated. Each value of a given element of the probability matrix may comprise a value indicative of the probability that a binding measurement is observed, given that probe j is exposed to protein i in the sample. This probability can be expressed as P(protein _i | probe_j). In the case that the corresponding hit table entry is greater than or equal to 1, then the probability matrix entry can be set to the true landing rate (e.g., P_landing_probe_j)). In the case that the corresponding hit table entry is 0, then the probability matrix entry can be set to the detector error rate (e.g., 0.0001). The detector error rate may comprise one or more of: physical detector error rate, off-target binding rate, or an error rate due to stuck probes.

In some embodiments, iteratively generating the plurality of probabilities further comprises removing one or more candidate proteins from the plurality of candidate proteins from subsequent iterations, thereby reducing a number of iterations necessary to perform the iterative generation of the probabilities. In some embodiments, removing the one or more candidate proteins is based at least on a predetermined criterion of the binding measurements associated with the candidate proteins. In some embodiments, the predetermined criterion comprises the one or more candidate proteins having binding measurements to a first plurality among the plurality of affinity reagent probes below a predetermined threshold. A protein may be excluded from consideration, for example, if its P(protein i| probes [1..k]) is less than 0.01, less than 0.001, less than 0.0001, less than 0.00001, less than 0.000001, or less than 0.0000001 after binding of k probes have been measured. A protein may also be excluded from consideration if it has been experimentally removed from the sample.

In some embodiments, each of the probabilities is normalized to a length of the candidate protein, as described elsewhere herein. In some embodiments, each of the probabilities are normalized to a total sum of probabilities of the plurality of candidate proteins, as described elsewhere herein. In some embodiments, the plurality of affinity reagent probes comprises no more than 10, no more than 20, no more than 30, no more than 40, no more than 50, no more than 60, no more than 70, no more than 80, no more than 90, no more than 100, no more than 150, no more than 200, no more than 250, no more than 300, no more than 350, no more than 400, no more than 450, no more than 500, or more than 500 affinity reagent probes.

In some embodiments, the probabilities are iteratively generated until a predetermined condition is satisfied. In some embodiments, the predetermined condition comprises generating each of the plurality of probabilities with a confidence of at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 99.9%.

In some embodiments, the method further comprises generating a paper or electronic report identifying one or more unknown proteins in the sample. The paper or electronic report may further indicate, for each of the candidate proteins, a confidence level for the candidate protein being present in the sample. The confidence level may comprise a probability value. Alternatively, the confidence level may comprise a probability value with an error. Alternatively, the confidence level may comprise a range of probability values, optionally with a confidence (e.g., 90%, 95%, 96%, 97%, 98%, or 99% confidence). The paper or electronic report may further indicate the list of protein candidates identified below an expected false discovery rate threshold (e.g., a false discovery rate below 10%, 5%, 4%, 3%, 2%, 1%, 0.5%, 0.4%, 0.3%, 0.2%, or 0.1%). The false discovery rate may be estimated by first sorting the protein identifications in descending order of confidence. The estimated false discovery rate at any point in the sorted list may then be calculated as 1 - avg_c_prob, where avg_c_prob is the average candidate probability for all proteins at or before (higher confidence) the current point in the list. A list of protein identifications below a desired false discovery rate threshold may then be generated by returning all protein identifications before the earliest point in the sorted list where the false discovery rate is higher than the threshold. Alternatively, a list of protein identifications below a desired false discovery rate threshold may be generated by returning all proteins before, and including, the latest point in the sorted list where the false discovery rate is below or equal to the desired threshold.

In some embodiments, the sample comprises a biological sample. The biological sample may be obtained from a subject. In some embodiments, the method further comprises identifying a disease state or a disorder in the subject based at least on the plurality of probabilities. In some embodiments, the method further comprises quantifying proteins by counting the number of identifications generated for each protein candidate. For example, the absolute quantity (number of protein molecules) of a protein present in the sample can be calculated by counting the number of confident identifications generated from that protein candidate. In some embodiments, the quantity may be calculated as a percentage of the total number of unknown proteins assayed. In some embodiments, the raw identification counts may be calibrated to remove systematic error from the instrument and detection systems. In some embodiments, the quantity may be calibrated to remove biases in quantity caused by variation in detectability of protein candidates. Protein detectability may be assessed from empirical measurements or computer simulation.

The disease or disorder may be an infectious disease, an immune disorder or disease, a cancer, a genetic disease, a degenerative disease, a lifestyle disease, an injury, a rare disease or an age related disease. The infectious disease may be caused by bacteria, viruses, fungi and/or parasites. Non-limiting examples of cancers include Bladder cancer, Lung cancer, Brain cancer, Melanoma, Breast cancer, Non-Hodgkin lymphoma, Cervical cancer, Ovarian cancer, Colorectal cancer, Pancreatic cancer, Esophageal cancer, Prostate cancer, Kidney cancer, Skin cancer, Leukemia, Thyroid cancer, Liver cancer, and Uterine cancer. Some examples of genetic diseases or disorders include, but are not limited to, cystic fibrosis, Charcot-Marie-Tooth disease, Huntington's disease, Peutz-Jeghers syndrome, Down syndrome, Rheumatoid arthritis, and Tay-Sachs disease. Non-limiting examples of lifestyle diseases include obesity, diabetes, arteriosclerosis, heart disease, stroke, hypertension, liver cirrhosis, nephritis, cancer, chronic obstructive pulmonary disease (copd), hearing problems, and chronic backache. Some examples of injuries include, but are not limited to, abrasion, brain injuries, bruising, burns, concussions, congestive heart failure, construction injuries, dislocation, flail chest, fracture, hemothorax, herniated disc, hip pointer, hypothermia, lacerations, pinched nerve, pneumothorax, rib fracture, sciatica, spinal cord injury, tendons ligaments fascia injury, traumatic brain injury, and whiplash.

In another aspect, disclosed herein is a computer-implemented method for identifying candidate proteins within a sample of unknown proteins. The method may comprise receiving, by the computer, information of binding measurements of each of a plurality of affinity reagent probes to the unknown proteins in the sample. The affinity reagent probes may be k-mer affinity reagent probes. In some embodiments, each k-mer affinity reagent probe is configured to selectively bind to one or more candidate proteins among a plurality of candidate proteins. The information of binding measurements may comprise a set of probes that are believed to have bound to an unknown protein.

Next at least a portion of the information of binding measurements may be compared, by the computer, against a database comprising a plurality of protein sequences. Each of the protein sequences may correspond to a candidate protein among the plurality of candidate proteins. The plurality of candidate proteins may comprise at least 10, at least 20, at least 30, at least 40, at least 50, at least 60, at least 70, at least 80, at least 90, at least 100, at least 150, at least 200, at least 250, at least 300, at least 350, at least 400, at least 450, at least 500, at least 600, at least 700, at least 800, at least 900, at least 1000, or more than 1000 different candidate proteins.

Next, one or more candidate proteins from the plurality of candidate proteins may be removed from further consideration (e.g., subsequent computations, iterations, calculations, or generations of probabilities). Removing of the one or more candidate proteins from the plurality of candidate proteins may be based at least on the comparison of the information of binding measurements against the database comprising the plurality of protein sequences.

In some embodiments, removing the one or more candidate proteins is based at least on a predetermined criterion of the binding measurements associated with the candidate proteins. In some embodiments, the predetermined criterion comprises the one or more candidate proteins having binding measurements to a first plurality among the plurality of affinity reagent probes below a predetermined threshold. In some embodiments a candidate protein may be excluded from consideration, for example, if its P(protein i| probes [1..k]) is less than 0.01, less than 0.001, less than 0.0001, less than 0.00001, less than 0.000001, or less than 0.0000001 after binding of k probes have been measured. A protein may also be excluded from consideration if it has been experimentally removed from the sample.

In some embodiments, the plurality of affinity reagent probes comprises no more than 10, no more than 20, no more than 30, no more than 40, no more than 50, no more than 60, no more than 70, no more than 80, no more than 90, no more than 100, no more than 150, no more than 200, no more than 250, no more than 300, no more than 350, no more than 400, no more than 450, no more than 500, or more than 500 affinity reagent probes.

In some embodiments, the affinity reagent probes for which binding measurements are made is completely determined prior to performing the measurements. In some embodiments, the set or order of affinity reagent probes for which binding measurements are to be made is modified or derived during the experiment, based on iterative computational analysis of the theretofore acquired binding measurements. For example, the ordering of affinity probes may be iteratively optimized to prioritize binding experiments with probes more likely to generate an unambiguous identification for unidentified unknown proteins. Such an optimization may be based on selecting probes that resolve the top two, the top three, the top four, the top five, or more than the top five candidate protein sequences for the theretofore unidentified unknown proteins.

In some embodiments, the method further comprises generating a paper or electronic report identifying one or more unknown proteins in the sample. The paper or electronic report may further indicate, for each of the candidate proteins, a confidence level for the candidate protein being present in the sample. The confidence level may comprise a probability value. Alternatively, the confidence level may comprise a probability value with an error. Alternatively, the confidence level may comprise a range of probability values, optionally with a confidence (e.g., 90%, 95%, 96%, 97%, 98%, 99% confidence). In some embodiments, the sample comprises a biological sample. The biological sample may be obtained from a subject. In some embodiments, the method further comprises identifying a disease state or a disorder in the subject based at least on the plurality of probabilities.

The disease or disorder may be an infectious disease, an immune disorder or disease, a cancer, a genetic disease, a degenerative disease, a lifestyle disease, an injury, a rare disease or an age related disease. The infectious disease may be caused by bacteria, viruses, fungi and/or parasites. Non-limiting examples of cancers include Bladder cancer, Lung cancer, Brain cancer, Melanoma, Breast cancer, Non-Hodgkin lymphoma, Cervical cancer, Ovarian cancer, Colorectal cancer, Pancreatic cancer, Esophageal cancer, Prostate cancer, Kidney cancer, Skin cancer, Leukemia, Thyroid cancer, Liver cancer, and Uterine cancer. Some examples of genetic diseases or disorders include, but are not limited to, cystic fibrosis, Charcot-Marie-Tooth disease, Huntington's disease, Peutz-Jeghers syndrome, Down syndrome, Rheumatoid arthritis, and Tay-Sachs disease. Non-limiting examples of lifestyle diseases include obesity, diabetes, arteriosclerosis, heart disease, stroke, hypertension, liver cirrhosis, nephritis, cancer, chronic obstructive pulmonary disease (copd), hearing problems, and chronic backache. Some examples of injuries include, but are not limited to, abrasion, brain injuries, bruising, burns, concussions, congestive heart failure, construction injuries, dislocation, flail chest, fracture, hemothorax, herniated disc, hip pointer, hypothermia, lacerations, pinched nerve, pneumothorax, rib fracture, sciatica, spinal cord injury, tendons ligaments fascia injury, traumatic brain injury, and whiplash.

In some embodiments, the method comprises identifying and quantifying small molecules (e.g. metabolites) or glycans instead of proteins. For example, affinity reagents such as lectins or antibodies which bind to sugars or combinations of sugars with varying propensity may be used to identify glycans. The affinity reagents propensity to bind various sugars or combinations of sugars may be characterized by analyzing binding to a commercially-available glycan array. Unknown glycans may be conjugated to a functionalized substrate using hydroxyl-reactive chemistry and binding measurements acquired using the glycan-binding affinity reagents. The binding measurements of the affinity reagents to the unknown glycans on the substrate may be used directly to quantify the number of glycans with a particular sugar or combination of sugars. Alternatively, one or more binding measurements may be compared to predicted binding measurements from a database of candidate glycan structures using the inference algorithm described herein to identify the structure of each unknown glycan. In some embodiments, proteins are bound to the substrate and binding measurements with glycan affinity reagents are generated to identify glycans attached to the proteins. Further, binding measurements may be made with both glycan and protein affinity reagents to generate protein backbone sequence and conjugated glycan identifications in a single experiment. As another example, metabolites may be conjugated to a functionalized substrate using chemistry targeted toward coupling groups commonly found in metabolites such as sulfhydryl, carbonyl, amine, or active hydrogen. Binding measurements may be made using affinity reagents with different propensities to particular functional groups, structural motifs, or metabolites. The resulting binding measurements may be compared to predicted binding measurements for a database of candidate small molecules and the inference approach described herein used to identify the metabolite at each location on the substrate.

**Computer Control Systems**

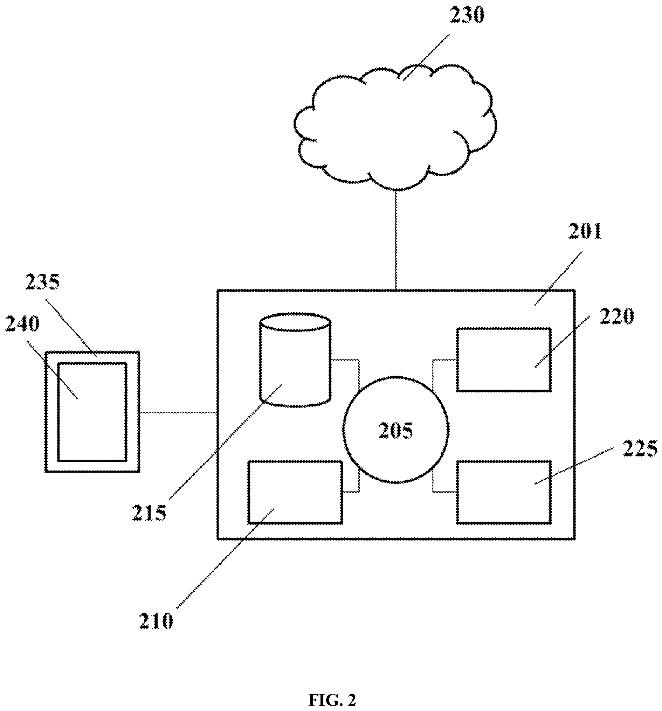

The present disclosure provides computer systems that are programmed to implement methods of the disclosure. FIG. 2 shows a computer system 201 that is programmed or otherwise configured to: receive information of binding measurements of affinity reagent probes to unknown proteins in a sample, compare information of binding measurements against a database comprising a plurality of protein sequences corresponding to candidate proteins, and/or iteratively generate probabilities that candidate proteins are present in the sample.

The computer system 201 can regulate various aspects of methods and systems of the present disclosure, such as, for example, receiving information of binding measurements of affinity reagent probes to unknown proteins in a sample, comparing information of binding measurements against a database comprising a plurality of protein sequences corresponding to candidate proteins, and/or iteratively generating probabilities that candidate proteins are present in the sample.

The computer system 201 can be an electronic device of a user or a computer system that is remotely located with respect to the electronic device. The electronic device can be a mobile electronic device. The computer system 201 includes a central processing unit (CPU, also "processor" and "computer processor" herein) 205, which can be a single core or multi core processor, or a plurality of processors for parallel processing. The computer system 201 also includes memory or memory location 210 (e.g., random-access memory, read-only memory, flash memory), electronic storage unit 215 (e.g., hard disk), communication interface 220 (e.g., network adapter) for communicating with one or more other systems, and peripheral devices 225, such as cache, other memory, data storage and/or electronic display adapters. The memory 210, storage unit 215, interface 220 and peripheral devices 225 are in communication with the CPU 205 through a communication bus (solid lines), such as a motherboard. The storage unit 215 can be a data storage unit (or data repository) for storing data. The computer system 201 can be operatively coupled to a computer network ("network") 230 with the aid of the communication interface 220. The network 230 can be the Internet, an internet and/or extranet, or an intranet and/or extranet that is in communication with the Internet. The network 230 in some cases is a telecommunication and/or data network. The network 230 can include one or more computer servers, which can enable distributed computing, such as cloud computing. The network 230, in some cases with the aid of the computer system 201, can implement a peer-to-peer network, which may enable devices coupled to the computer system 201 to behave as a client or a server.

The CPU 205 can execute a sequence of machine-readable instructions, which can be embodied in a program or software. The instructions may be stored in a memory location, such as the memory 210. The instructions can be directed to the CPU 205, which can subsequently program or otherwise configure the CPU 205 to implement methods of the present disclosure. Examples of operations performed by the CPU 205 can include fetch, decode, execute, and writeback.

The CPU 205 can be part of a circuit, such as an integrated circuit. One or more other components of the system 201 can be included in the circuit. In some cases, the circuit is an application specific integrated circuit (ASIC).

The storage unit 215 can store files, such as drivers, libraries and saved programs. The storage unit 215 can store user data, e.g., user preferences and user programs. The computer system 201 in some cases can include one or more additional data storage units that are external to the computer system 201, such as located on a remote server that is in communication with the computer system 201 through an intranet or the Internet.

The computer system 201 can communicate with one or more remote computer systems through the network 230. For instance, the computer system 201 can communicate with a remote computer system of a user. Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 201 via the network 230.

Methods as described herein can be implemented by way of machine (e.g., computer processor) executable code stored on an electronic storage location of the computer system 201, such as, for example, on the memory 210 or electronic storage unit 215. The machine executable or machine readable code can be provided in the form of software. During use, the code can be executed by the processor 205. In some cases, the code can be retrieved from the storage unit 215 and stored on the memory 210 for ready access by the processor 205. In some situations, the electronic storage unit 215 can be precluded, and machine-executable instructions are stored on memory 210.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 201, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 201 can include or be in communication with an electronic display 235 that comprises a user interface (UI) 240 for providing, for example, user selection of algorithms, binding measurement data, candidate proteins, and databases. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 205. The algorithm can, for example, receive information of binding measurements of affinity reagent probes to unknown proteins in a sample, compare information of binding measurements against a database comprising a plurality of protein sequences corresponding to candidate proteins, and/or iteratively generate probabilities that candidate proteins are present in the sample.

**Example 1- Protein identification with a database of 6 candidate proteins**

Consider a situation where a database contains 6 candidate proteins of lengths: {276, 275, 151, 437, 244, 644}. Additionally, the experiment is performed with 5 probes, each of which has 25% likelihood of binding to a given trimer. The other trimers these reagents bind to are not found in any protein in the database.

A hit table is constructed for the probes to each sequence in the database

(Row = probes #1 to #5, Col = SEQ ID 1 to 6)

|           | 0 | 1 | 2 | 3 | 4 | 5 |
|-----------|---|---|---|---|---|---|
| GAV/0.250 | 1 | 1 |   |   |   | 1 |
| CLD/0.250 | 1 | 1 |   |   |   | 1 |
| TYL/0.250 | 1 | 1 |   |   |   | 2 |
| IAD/0.250 | 1 | 1 |   |   |   | 1 |
| PLE/0.250 | 1 | 1 | 1 |   |   |   |

Notably, this information arrives incrementally, and therefore may be computed iteratively. From the hit table, P(protein_i | probe_j) is evaluated to generate a probability matrix, as shown below. Note that for a given entry, if hit table >= 1, then use P_landing_probe_n = true landing rate = 0.25; else if hit table = 0, use P(detector error) = 0.0001.

| 276  | 275  | 151    | 437    | 244    | 644    |
|------|------|--------|--------|--------|--------|
| 0    | 1    | 2      | 3      | 4      | 5      |
| 0.25 | 0.25 | 0.0001 | 0.0001 | 0.0001 | 0.25   |
| 0.25 | 0.25 | 0.0001 | 0.0001 | 0.0001 | 0.25   |
| 0.25 | 0.25 | 0.0001 | 0.0001 | 0.0001 | 0.25   |
| 0.25 | 0.25 | 0.0001 | 0.0001 | 0.0001 | 0.25   |
| 0.25 | 0.25 | 0.25   | 0.0001 | 0.0001 | 0.0001 |

Note that many of the cells contain a 0.0001 probability. This small probability accounts for possible detector error.

The initial, un-normalized probability of a protein is calculated as the product of the probabilities for each candidate protein:

| | | | | | | |
|---|---|---|---|---|---|---|
| ProductP | 0.000977 | 0.000977 | 2.5E-17 | 1E-20 | 1E-20 | 3.906E-07 |

Next, the length normalization is computed, which refers to the number of ways some number of probes landed on a given protein, as a function of the length of the protein. The length normalization is given by the Choose(Len_i, n) term. For example, the first protein has a length normalization of [276 choose 5] and the second protein has a length normalization of [275 choose 5]. In some embodiments, the length normalization may be calculated as the number of permutations calculated as Len_i! / (len_i! - n!), where the ! operation indicates a factorial.

| | | | | | | |
|---|---|---|---|---|---|---|
| LenNorm | 12868936080 | 12635803180 | 151 | 1 | 1 | 7100332001 |

Next, the product from above (ProductP) is normalized to take into account this length correction, by dividing by the length normalization, which gives:

| | | | | | | |
|---|---|---|---|---|---|---|
| LenNormP | 7.59E-14 | 7.73E-14 | 1.66E-19 | 1E-20 | 1E-20 | 5.50E-17 |

Next, the probabilities are normalized such that the entire set of probabilities over the entire database sums up to one. This is achieved by summing the LenNormP values to 1.53E-13 and then dividing each of the LenNormP by this normalization to achieve the final balanced probabilities:

| | | | | | |
|---|---|---|---|---|---|
| 0.495251 | 0.504389 | 1.081E-06 | 6.526E-08 | 6.526E-08 | 0.000359 |

Note that while 4 of the proteins are extremely unlikely, it is somewhat hard to disambiguate proteins 1 and 2. Looking at the database, this is expected as there is only a single point deletion differentiation between proteins 1 and 2. Also, note that proteins 1 and 2 are split at 50% probability each, while proteins 3-6 have essentially zero probability.

In the experimental technique, probes are detected sequentially; therefore, it is desirable to compute this function iteratively. There are multiple different ways to achieve this an example of which is shown below.

## Example 2 - **Protein identification using mixtures of antibodies**

Consistent with disclosed embodiments, the identification of 1,000 unknown human proteins was benchmarked by acquiring binding measurements using pools of commercially-available antibodies from the Santa Cruz Biotechnology catalog. The 1,000 unknown proteins were randomly selected from the Uniprot protein database comprising about 21,005 proteins. A list of monoclonal antibodies available from the Santa Cruz Biotechnology catalog with reactivity against human proteins was downloaded from an online antibody registry. This list contained 22,301 antibodies, and was filtered to a list of 14,566 antibodies which matched to proteins in the Uniprot human protein database. The complete collection of antibodies modeled in the experiment comprised these 14,566 antibodies. Experimental assessment of binding of antibody mixtures to the 1,000 unknown protein candidates was performed as follows:

First, 50 mixtures of antibodies were modeled. To produce any single mixture, 5,000 antibodies from the total collection of antibodies were selected at random.

Next, for each mixture, a binding probability was determined for the mixture to any of the unknown proteins. Note that, although the proteins are "unknown" in the sense that the goal is to infer their identity, the algorithm is aware of the true identity of each "unknown protein." If the mixture contains an antibody against the unknown protein, a binding probability of 0.99 was assigned. If the mixture does not contain an antibody against the unknown protein, a binding probability of 0.0488 was assigned.

The non-specific binding probability for a mixture was modeled based on the expected probability of any individual antibody binding a protein other than its target, and the number of proteins in the mixture. For this experimental assessment, it was assumed that there is a probability of 0.00001 (1E-5) of a non-specific binding event where an individual antibody binding something other than its target protein. The probability of a non-specific binding event for the mixture of antibodies is the probability of any single antibody in the mixture binding non-specifically. This probability was calculated as one minus the probability of all 5000 antibodies in the mixture not binding non-specifically, or $1 - (1 - 1e-5)^{1000} = 0.0488$.

For each unknown protein, binding was assessed for each antibody mixture measured based on the binding probability of the mixture to the unknown protein. The uniform distribution, with a minimum of 0 and a maximum of 1, was randomly sampled, and if the resulting number is less than the binding probability of the antibody mixture to the unknown protein, the experiment resulted in a binding event for that mixture. Otherwise, the experiment resulted in a non-binding event for that mixture. With all binding events assessed, protein inference is performed as follows:

For each unknown protein, the sequence of assessed binding events (50 total, 1 per mixture) was evaluated against each of the 21,005 protein candidates in the Uniprot database. More specifically, a probability of observing the sequence of binding events was calculated for each candidate. The probability was calculated by multiplying the probability of each individual mixture binding / non-binding event across all 50 mixtures measured. The binding probability was calculated in the same manner as described above, and the probability of non-binding is one minus the binding probability. The protein query candidate with the highest binding probability is the inferred identity for the unknown protein. A probability of the identification being correct for that individual protein was calculated as the probability of the top individual candidate divided by the summed probabilities of all candidates.

With the identity inferred for each of the 1,000 unknown proteins, the unknown proteins were sorted in descending order of their identification probability. An identification probability cutoff was selected such that the percentage of incorrect identifications among all identifications prior in the list was 1%. Overall, 551 of the 1,000 unknown proteins were identified with a 1% incorrect identification rate.

**Example 3: Protein identification using binding measurement outcomes**

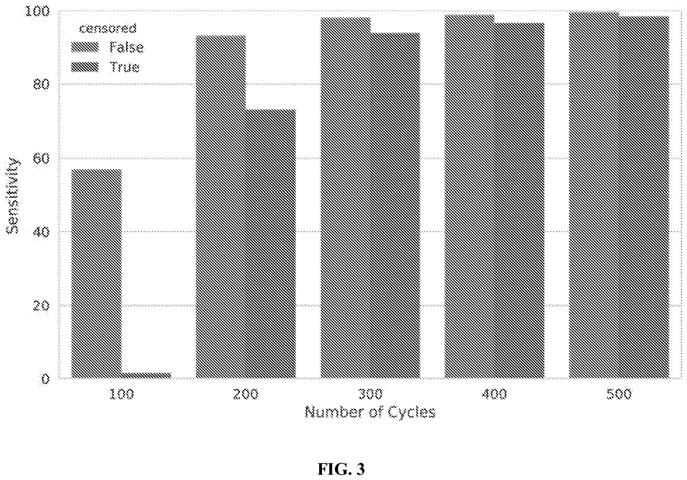

The methods described herein may be applied to different subsets of data associated with the binding and/or non-binding of affinity reagents to unidentified proteins. In some embodiments, methods described herein may be applied to experiments in which a particular subset of the measured binding outcomes is not considered (e.g., non-binding measurement outcomes). These methods where a subset of the measured binding outcomes are not considered may be referred to herein as a "censored" inference approach (e.g., as described in **Example 1**). In the results described in FIG. 3, the protein identifications that result from the censored inference approach are based on assessing occurrences of binding events associated with the particular unidentified proteins. Accordingly, the censored inference approach does not consider non-binding outcomes in determining identities of unknown proteins.

This type of censored inference approach is in contrast to an "uncensored" approach, in which all obtained binding outcomes are considered (e.g., both binding measurement outcomes and non-binding measurement outcomes associated with the particular unidentified proteins). In some embodiments, a censored approach may be applicable in cases where there is an expectation that particular binding measurements or binding measurement outcomes are more error-prone or likely to deviate from the expected binding measurement outcome for the protein (e.g. the probability of that binding measurement outcome being generated by the protein). For example, in an affinity reagent binding experiment, probabilities of binding measurement outcomes and non-binding measurement outcomes may be calculated based on binding to denatured proteins with predominantly linear structure. In these conditions, epitopes may be easily accessible to affinity reagents. However, in some embodiments, binding measurements on the assayed protein sample may be collected under non-denaturing or partially-denaturing conditions where proteins are present in a "folded" state with significant 3-dimensional structure, which can in many cases cause affinity reagent binding epitopes on the protein that are accessible in a linearized form to be inaccessible due to steric hinderance in the folded state. If, for example, the epitopes that the affinity reagent recognizes for a protein are in structurally accessible regions of the folded protein, the expectation may be that empirical binding measurements acquired on the unknown sample will be consistent with the calculated probabilities of binding derived from linearized proteins. However, if, for example, the epitopes recognized by the affinity reagent are structurally inaccessible, the expectation may be that there will be more non-binding outcomes than expected from calculated probabilities of binding derived from linearized proteins. Further, based on the particular conditions surrounding the protein, the 3-dimensional structure may be configured in a number of different possible configurations, and each of the different possible configurations may have an unique expectation for binding a particular affinity reagent based on the degree of accessibility of the desired affinity reagent.

As such, non-binding outcomes may be expected to deviate from the calculated binding probabilities for each protein, and a censored inference approach which only considers binding outcomes may be appropriate. In the "censored" inference approach as provided in FIG. 3, only measured binding outcomes are considered (in other words, either non-binding outcomes are not measured, or measured non-binding outcomes are not considered), such that the probability of a binding outcome set only considers the M measured binding outcomes that resulted in a binding measurement, which is a subset of the N total measured binding outcomes containing both binding and non-binding measurement outcomes. This may be described by the expression:

P(outcome set | protein) = P(binding event 1 | protein) * P(binding event 2 | protein * . . . * P(binding event M | protein)

When applying a censored approach, it may be appropriate to apply a scaling factor to P(binding outcome set | protein) to correct for biases. For example, longer proteins generally have a higher probability of generating a potential binding outcome (e.g.,because they contain more potential binding sites). To correct for this bias, a scaled likelihood SL may be calculated for each candidate protein by dividing the P(binding outcome set | protein) by the number of unique combinations of M binding sites that can be generated from the protein based on the number of potential binding sites on the protein. For a protein of length L, with trimer recognition sites, there may be L-2 potential binding sites (e.g., every possible length L subsequence of the complete protein sequence), such that:

$$SL_{Protein} = \frac{P(outcome\ set\ |\ protein)}{\binom{L-2}{M}} = \frac{P(outcome\ set\ |\ protein)M!\,(L-2-M)!}{(L-2)!}$$

The probability of any candidate protein selected from a collection of Q possible candidate proteins, given the outcome set, may be given by:

$$P(protein_i\ |\ outcome\ set) = \frac{SL_{Protein_i}}{\sum_{j=1}^{Q} SL_{Protein_j}}$$

The performance of an embodiment of a censored protein inference vs. uncensored protein inference approach is plotted in **FIG. 3.** The data plotted in FIG. 3 is provided in Table 1.

**Table 1**

| Censored | Number of Probes | Sensitivity |
|---|---|---|
| TRUE | 100 | 1.52 |
| FALSE | 100 | 56.84 |
| TRUE | 200 | 73.28 |
| FALSE | 200 | 93.18 |
| TRUE | 300 | 93.92 |
| FALSE | 300 | 98.14 |
| TRUE | 400 | 96.68 |
| FALSE | 400 | 98.84 |
| TRUE | 500 | 98.42 |
| FALSE | 500 | 99.6 |

In the comparison shown in FIG. 3, the protein identification sensitivity (e.g., percent of unique proteins identified) is plotted against the number of affinity reagent cycles measured for both censored inference and uncensored inference used on linearized protein substrates. The affinity reagents used are targeted against the top most abundant trimers in the proteome, and each affinity reagent has off-target affinity to four additional random trimers. The uncensored approach outperforms the censored approach by a greater than ten-fold margin when 100 affinity reagent cycles are used. The degree to which uncensored inference outperforms censored inference lessens when more cycles are used.

### Example 4: Tolerance of protein identification to random false negative and false positive affinity reagent binding

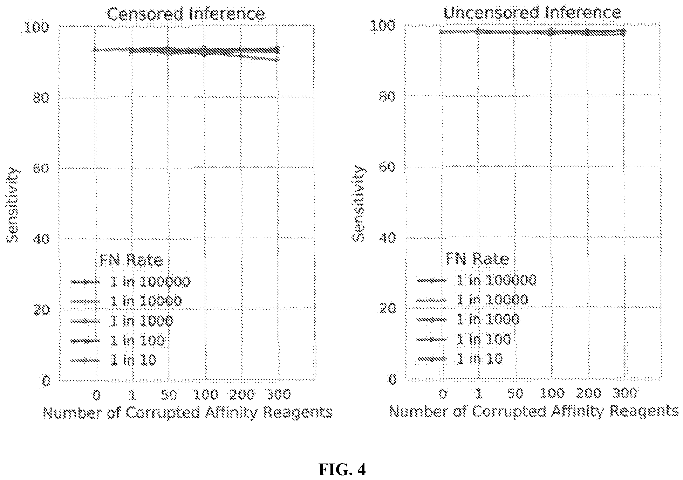

In some cases, there may be a high incidence of false negative binding measurement outcomes for affinity reagent binding. "False negative" binding outcomes manifest as affinity reagent binding measurements occurring less frequently than expected. Such "false negative" outcomes may arise, for example, due to issues with the binding detection method, the binding conditions (for example, temperature, buffer composition, etc.), corruption of the protein sample, or corruption of the affinity reagent stock. To determine the impact of false negative measurements on the censored protein identification and the uncensored protein identification approach, a subset of affinity reagent measurement cycles were purposely corrupted by switching either 1 in 10, 1 in 100, 1 in 1,000, 1 in 10,000, or 1 in 100,000 random observed binding events to non-binding events *in silico*. Either 0, 1, 50, 100, 200, or 300 of the 300 total affinity reagent cycles were corrupted in this manner. As shown by the results plotted in **FIG. 4,** both the censored protein identification approach and the uncensored protein identification approach are tolerant to this type of random false negative binding. The data plotted in FIG. 4 is provided in Table 2.

**Table 2**

| Censored | False Negative Rate | Number of Probes | Number of Probes Impacted | Sensitivity |
|---|---|---|---|---|
| TRUE | 0.1 | 300 | 0 | 93.32 |
| FALSE | 0.1 | 300 | 0 | 98.04 |
| TRUE | 0.1 | 300 | 1 | 93.42 |
| FALSE | 0.1 | 300 | 1 | 98.12 |
| TRUE | 0.01 | 300 | 1 | 92.98 |
| FALSE | 0.01 | 300 | 1 | 98.48 |
| TRUE | 0.001 | 300 | 1 | 92.8 |
| FALSE | 0.001 | 300 | 1 | 97.82 |
| TRUE | 0.0001 | 300 | 1 | 92.82 |
| FALSE | 0.0001 | 300 | 1 | 98.32 |
| TRUE | 0.00001 | 300 | 1 | 93.38 |
| FALSE | 0.00001 | 300 | 1 | 98.02 |
| TRUE | 0.1 | 300 | 50 | 92.26 |
| FALSE | 0.1 | 300 | 50 | 97.96 |
| TRUE | 0.01 | 300 | 50 | 92.7 |
| FALSE | 0.01 | 300 | 50 | 97.76 |
| TRUE | 0.001 | 300 | 50 | 93.72 |
| FALSE | 0.001 | 300 | 50 | 98.04 |
| TRUE | 0.0001 | 300 | 50 | 92.96 |
| FALSE | 0.0001 | 300 | 50 | 97.84 |
| TRUE | 0.00001 | 300 | 50 | 93.7 |
| FALSE | 0.00001 | 300 | 50 | 98.1 |
| TRUE | 0.1 | 300 | 100 | 92.38 |
| FALSE | 0.1 | 300 | 100 | 97.66 |
| TRUE | 0.01 | 300 | 100 | 93.02 |
| FALSE | 0.01 | 300 | 100 | 97.7 |
| TRUE | 0.001 | 300 | 100 | 92.48 |
| FALSE | 0.001 | 300 | 100 | 97.96 |
| TRUE | 0.0001 | 300 | 100 | 93.74 |
| FALSE | 0.0001 | 300 | 100 | 98.34 |
| TRUE | 0.00001 | 300 | 100 | 91.88 |
| FALSE | 0.00001 | 300 | 100 | 97.2 |
| TRUE | 0.1 | 300 | 200 | 91.42 |
| FALSE | 0.1 | 300 | 200 | 97.28 |
| TRUE | 0.01 | 300 | 200 | 93.38 |
| FALSE | 0.01 | 300 | 200 | 98.2 |

(continued)

| Censored | False Negative Rate | Number of Probes | Number of Probes Impacted | Sensitivity |
|---|---|---|---|---|
| TRUE | 0.001 | 300 | 200 | 93.3 |
| FALSE | 0.001 | 300 | 200 | 98.08 |
| TRUE | 0.0001 | 300 | 200 | 92.68 |
| FALSE | 0.0001 | 300 | 200 | 98.12 |
| TRUE | 0.00001 | 300 | 200 | 92.7 |
| FALSE | 0.00001 | 300 | 200 | 98.16 |
| TRUE | 0.1 | 300 | 300 | 90.2 |
| FALSE | 0.1 | 300 | 300 | 97.1 |
| TRUE | 0.01 | 300 | 300 | 92.96 |
| FALSE | 0.01 | 300 | 300 | 98.16 |
| TRUE | 0.001 | 300 | 300 | 93.64 |
| FALSE | 0.001 | 300 | 300 | 98.14 |
| TRUE | 0.0001 | 300 | 300 | 92.92 |
| FALSE | 0.0001 | 300 | 300 | 98.18 |
| TRUE | 0.00001 | 300 | 300 | 92.54 |
| FALSE | 0.00001 | 300 | 300 | 98.14 |

Similarly, tolerance to "false positive" binding outcomes was assessed by switching a subset of binding outcomes from non-binding outcomes to binding outcomes. The results of this assessment are provided in Table 3.

**Table 3**

| Censored | False Positive Rate | Number of Probes | Number of Probes Impacted | Sensitivity |
|---|---|---|---|---|
| TRUE | 0.1 | 300 | 0 | 93.32 |
| FALSE | 0.1 | 300 | 0 | 98.04 |
| TRUE | 0.1 | 300 | 1 | 92.54 |
| FALSE | 0.1 | 300 | 1 | 98.26 |
| TRUE | 0.01 | 300 | 1 | 92.74 |
| FALSE | 0.01 | 300 | 1 | 97.94 |
| TRUE | 0.001 | 300 | 1 | 92.48 |
| FALSE | 0.001 | 300 | 1 | 97.88 |
| TRUE | 0.0001 | 300 | 1 | 92.78 |
| FALSE | 0.0001 | 300 | 1 | 98.26 |
| TRUE | 0.00001 | 300 | 1 | 93.06 |
| FALSE | 0.00001 | 300 | 1 | 98.16 |
| TRUE | 0.1 | 300 | 50 | 68.2 |
| FALSE | 0.1 | 300 | 50 | 89.32 |
| TRUE | 0.01 | 300 | 50 | 91.28 |
| FALSE | 0.01 | 300 | 50 | 97.48 |
| TRUE | 0.001 | 300 | 50 | 92.66 |

(continued)

| Censored | False Positive Rate | Number of Probes | Number of Probes Impacted | Sensitivity |
|---|---|---|---|---|
| FALSE | 0.001 | 300 | 50 | 98.1 |
| TRUE | 0.0001 | 300 | 50 | 93 |
| FALSE | 0.0001 | 300 | 50 | 98.16 |
| TRUE | 0.00001 | 300 | 50 | 93.46 |
| FALSE | 0.00001 | 300 | 50 | 97.68 |
| TRUE | 0.1 | 300 | 100 | 40.98 |
| FALSE | 0.1 | 300 | 100 | 75.02 |
| TRUE | 0.01 | 300 | 100 | 88.56 |
| FALSE | 0.01 | 300 | 100 | 96.94 |
| TRUE | 0.001 | 300 | 100 | 93.34 |
| FALSE | 0.001 | 300 | 100 | 98.26 |
| TRUE | 0.0001 | 300 | 100 | 93.4 |
| FALSE | 0.0001 | 300 | 100 | 97.96 |
| TRUE | 0.00001 | 300 | 100 | 92.62 |
| FALSE | 0.00001 | 300 | 100 | 98.34 |
| TRUE | 0.1 | 300 | 200 | 14.8 |
| FALSE | 0.1 | 300 | 200 | 39.7 |
| TRUE | 0.01 | 300 | 200 | 84.56 |
| FALSE | 0.01 | 300 | 200 | 95.58 |
| TRUE | 0.001 | 300 | 200 | 92.22 |
| FALSE | 0.001 | 300 | 200 | 97.64 |
| TRUE | 0.0001 | 300 | 200 | 93.2 |
| FALSE | 0.0001 | 300 | 200 | 98.12 |
| TRUE | 0.00001 | 300 | 200 | 92.08 |
| FALSE | 0.00001 | 300 | 200 | 98.16 |
| TRUE | 0.1 | 300 | 300 | 3.46 |
| FALSE | 0.1 | 300 | 300 | 17.44 |
| TRUE | 0.01 | 300 | 300 | 79.46 |
| FALSE | 0.01 | 300 | 300 | 93.78 |
| TRUE | 0.001 | 300 | 300 | 92.52 |
| FALSE | 0.001 | 300 | 300 | 97.94 |
| TRUE | 0.0001 | 300 | 300 | 93.36 |
| FALSE | 0.0001 | 300 | 300 | 98.28 |
| TRUE | 0.00001 | 300 | 300 | 93.16 |
| FALSE | 0.00001 | 300 | 300 | 97.78 |

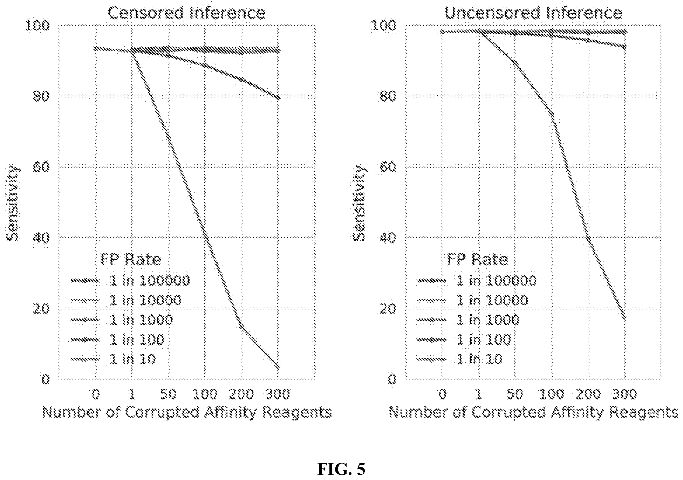

These results, which are plotted in **FIG. 5,** indicate that the performance of a censored protein identification approach degrades more rapidly than the uncensored protein identification approach with increasing incidence of random false positive measurements. However, both approaches tolerate a false positive rate of 1 in 1000 in every affinity reagent cycle or a 1 in 100 rate in a subset of the affinity reagent cycles.

**Example 5: Performance of protein inference with overestimated or underestimated affinity reagent binding probabilities**

Protein identification sensitivity was assessed using protein identification with correctly estimated affinity reagent to trimer binding probabilities, and with overestimated or underestimated binding probabilities. The true binding probability was 0.25. The underestimated binding probabilities were: 0.05, 0.1, and 0.2. The overestimated binding probabilities were 0.30, 0.50, 0.75, and 0.90. In total, 300 cycles of affinity reagent measurements were acquired. None (0), all 300, or a subset (1, 50, 100, 200) of the affinity reagents had the overestimated or underestimated binding probabilities applied. All others had the correct binding probabilities (0.25) used in protein identification. The results of the analysis are provided in Table 4.

**Table 4**

| Censored | Inference Binding Probability | Number of Probes | Number of Probes Impacted | Sensitivity | True Binding Probability |
|---|---|---|---|---|---|
| TRUE | 0.05 | 300 | 0 | 93.32 | 0.25 |
| FALSE | 0.05 | 300 | 0 | 98.04 | 0.25 |
| TRUE | 0.05 | 300 | 1 | 94.04 | 0.25 |
| FALSE | 0.05 | 300 | 1 | 98.6 | 0.25 |
| TRUE | 0.1 | 300 | 1 | 93.22 | 0.25 |
| FALSE | 0.1 | 300 | 1 | 97.8 | 0.25 |
| TRUE | 0.2 | 300 | 1 | 92.64 | 0.25 |
| FALSE | 0.2 | 300 | 1 | 98.14 | 0.25 |
| TRUE | 0.25 | 300 | 1 | 93.24 | 0.25 |
| FALSE | 0.25 | 300 | 1 | 97.86 | 0.25 |
| TRUE | 0.3 | 300 | 1 | 93.3 | 0.25 |
| FALSE | 0.3 | 300 | 1 | 98.24 | 0.25 |
| TRUE | 0.5 | 300 | 1 | 93.28 | 0.25 |
| FALSE | 0.5 | 300 | 1 | 97.96 | 0.25 |
| TRUE | 0.75 | 300 | 1 | 93.38 | 0.25 |
| FALSE | 0.75 | 300 | 1 | 97.94 | 0.25 |
| TRUE | 0.9 | 300 | 1 | 92.84 | 0.25 |
| FALSE | 0.9 | 300 | 1 | 97.32 | 0.25 |
| TRUE | 0.05 | 300 | 50 | 92.22 | 0.25 |
| FALSE | 0.05 | 300 | 50 | 97.8 | 0.25 |
| TRUE | 0.1 | 300 | 50 | 93.14 | 0.25 |
| FALSE | 0.1 | 300 | 50 | 98.36 | 0.25 |
| TRUE | 0.2 | 300 | 50 | 93.5 | 0.25 |
| FALSE | 0.2 | 300 | 50 | 98.46 | 0.25 |
| TRUE | 0.25 | 300 | 50 | 92.98 | 0.25 |
| FALSE | 0.25 | 300 | 50 | 98.16 | 0.25 |
| TRUE | 0.3 | 300 | 50 | 92.42 | 0.25 |
| FALSE | 0.3 | 300 | 50 | 98.28 | 0.25 |

(continued)

| Censored | Inference Binding Probability | Number of Probes | Number of Probes Impacted | Sensitivity | True Binding Probability |
|---|---|---|---|---|---|
| TRUE | 0.5 | 300 | 50 | 93.18 | 0.25 |
| FALSE | 0.5 | 300 | 50 | 98.18 | 0.25 |
| TRUE | 0.75 | 300 | 50 | 92.98 | 0.25 |
| FALSE | 0.75 | 300 | 50 | 96.9 | 0.25 |
| TRUE | 0.9 | 300 | 50 | 92.6 | 0.25 |
| FALSE | 0.9 | 300 | 50 | 94.18 | 0.25 |
| TRUE | 0.05 | 300 | 100 | 92.7 | 0.25 |
| FALSE | 0.05 | 300 | 100 | 97.88 | 0.25 |
| TRUE | 0.1 | 300 | 100 | 93.14 | 0.25 |
| FALSE | 0.1 | 300 | 100 | 97.94 | 0.25 |
| TRUE | 0.2 | 300 | 100 | 92.94 | 0.25 |
| FALSE | 0.2 | 300 | 100 | 97.66 | 0.25 |
| TRUE | 0.25 | 300 | 100 | 92.74 | 0.25 |
| FALSE | 0.25 | 300 | 100 | 97.72 | 0.25 |
| TRUE | 0.3 | 300 | 100 | 93.06 | 0.25 |
| FALSE | 0.3 | 300 | 100 | 98.34 | 0.25 |
| TRUE | 0.5 | 300 | 100 | 92.52 | 0.25 |
| FALSE | 0.5 | 300 | 100 | 98.2 | 0.25 |
| TRUE | 0.75 | 300 | 100 | 92.26 | 0.25 |
| FALSE | 0.75 | 300 | 100 | 95.88 | 0.25 |
| TRUE | 0.9 | 300 | 100 | 91.54 | 0.25 |
| FALSE | 0.9 | 300 | 100 | 84.26 | 0.25 |
| TRUE | 0.05 | 300 | 200 | 91.6 | 0.25 |
| FALSE | 0.05 | 300 | 200 | 95.22 | 0.25 |
| TRUE | 0.1 | 300 | 200 | 93.36 | 0.25 |
| FALSE | 0.1 | 300 | 200 | 97.76 | 0.25 |
| TRUE | 0.2 | 300 | 200 | 92.96 | 0.25 |
| FALSE | 0.2 | 300 | 200 | 97.88 | 0.25 |
| TRUE | 0.25 | 300 | 200 | 93.28 | 0.25 |
| FALSE | 0.25 | 300 | 200 | 98.28 | 0.25 |
| TRUE | 0.3 | 300 | 200 | 92.7 | 0.25 |
| FALSE | 0.3 | 300 | 200 | 97.6 | 0.25 |
| TRUE | 0.5 | 300 | 200 | 92.36 | 0.25 |
| FALSE | 0.5 | 300 | 200 | 97.34 | 0.25 |
| TRUE | 0.75 | 300 | 200 | 91.22 | 0.25 |
| FALSE | 0.75 | 300 | 200 | 88.52 | 0.25 |
| TRUE | 0.9 | 300 | 200 | 90.52 | 0.25 |

(continued)

| Censored | Inference Binding Probability | Number of Probes | Number of Probes Impacted | Sensitivity | True Binding Probability |
|---|---|---|---|---|---|
| FALSE | 0.9 | 300 | 200 | 33 | 0.25 |
| TRUE | 0.05 | 300 | 300 | 91.7 | 0.25 |
| FALSE | 0.05 | 300 | 300 | 0 | 0.25 |
| TRUE | 0.1 | 300 | 300 | 92.66 | 0.25 |
| FALSE | 0.1 | 300 | 300 | 92.06 | 0.25 |
| TRUE | 0.2 | 300 | 300 | 92.78 | 0.25 |
| FALSE | 0.2 | 300 | 300 | 98.02 | 0.25 |
| TRUE | 0.25 | 300 | 300 | 93.56 | 0.25 |
| FALSE | 0.25 | 300 | 300 | 98.02 | 0.25 |
| TRUE | 0.3 | 300 | 300 | 93 | 0.25 |
| FALSE | 0.3 | 300 | 300 | 98.22 | 0.25 |
| TRUE | 0.5 | 300 | 300 | 91.6 | 0.25 |
| FALSE | 0.5 | 300 | 300 | 96.72 | 0.25 |
| TRUE | 0.75 | 300 | 300 | 90.36 | 0.25 |
| FALSE | 0.75 | 300 | 300 | 67.08 | 0.25 |
| TRUE | 0.9 | 300 | 300 | 88.72 | 0.25 |
| FALSE | 0.9 | 300 | 300 | 0.58 | 0.25 |

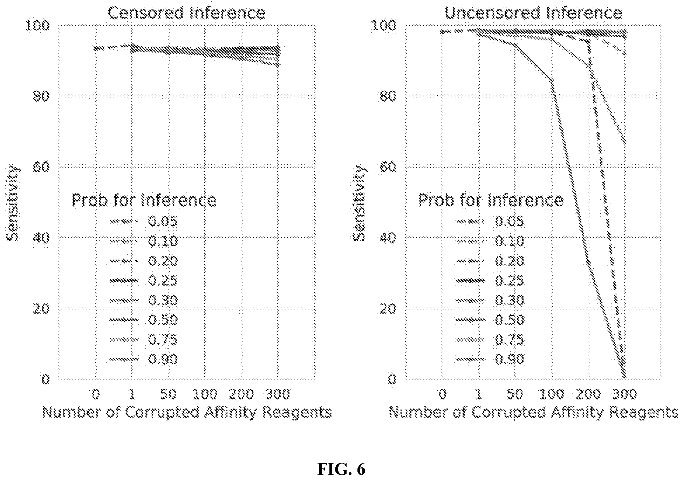

These results, which are plotted in **FIG. 6,** show that censored protein identification may be a preferred approach in some cases where binding probabilities may not be accurately estimated.

**Example 6: Performance of protein inference approaches using affinity reagents with unknown binding epitopes**

In some cases, affinity reagents may possess a number of binding sites which are unknown. The sensitivity of censored protein identification and uncensored protein identification approaches with affinity reagent binding measurements were compared using affinity reagents that each bind five trimer sites (e.g. a targeted trimer, and four random off-target sites) with probability 0.25 that are input into the protein identification algorithm. A subset of the affinity reagents (0 of 300, 1 of 300, 50 of 300, 100 of 300, 200 of 300, or 300 of 300) had either 1, 4, or 40 additional extra binding sites each against a random trimer with binding probability 0.05, 0.1 or 0.25. The results of the analysis are shown in Table 5.

**Table 5**

| Censored | Extra Sites Binding Probability | Number of Probes | Number of Probes Impacted | Sensitivity | Number of Unknown Extra Sites |
|---|---|---|---|---|---|
| TRUE | 0.05 | 300 | 0 | 93.32 | 1 |
| FALSE | 0.05 | 300 | 0 | 98.04 | 1 |
| TRUE | 0.05 | 300 | 1 | 93.14 | 1 |
| FALSE | 0.05 | 300 | 1 | 97.96 | 1 |
| TRUE | 0.05 | 300 | 1 | 92.68 | 4 |
| FALSE | 0.05 | 300 | 1 | 98.12 | 4 |
| TRUE | 0.05 | 300 | 1 | 92.32 | 40 |
| FALSE | 0.05 | 300 | 1 | 97.82 | 40 |

(continued)

| Censored | Extra Sites Binding Probability | Number of Probes | Number of Probes Impacted | Sensitivity | Number of Unknown Extra Sites |
|---|---|---|---|---|---|
| TRUE | 0.1 | 300 | 1 | 92.28 | 1 |
| FALSE | 0.1 | 300 | 1 | 98.02 | 1 |
| TRUE | 0.1 | 300 | 1 | 92.56 | 4 |
| FALSE | 0.1 | 300 | 1 | 98.34 | 4 |
| TRUE | 0.1 | 300 | 1 | 92.64 | 40 |
| FALSE | 0.1 | 300 | 1 | 97.86 | 40 |
| TRUE | 0.25 | 300 | 1 | 93.42 | 1 |
| FALSE | 0.25 | 300 | 1 | 98.46 | 1 |
| TRUE | 0.25 | 300 | 1 | 92.94 | 4 |
| FALSE | 0.25 | 300 | 1 | 98.12 | 4 |
| TRUE | 0.25 | 300 | 1 | 92.36 | 40 |
| FALSE | 0.25 | 300 | 1 | 98.1 | 40 |
| TRUE | 0.05 | 300 | 50 | 93.16 | 1 |
| FALSE | 0.05 | 300 | 50 | 97.94 | 1 |
| TRUE | 0.05 | 300 | 50 | 92.12 | 4 |
| FALSE | 0.05 | 300 | 50 | 97.44 | 4 |
| TRUE | 0.05 | 300 | 50 | 67.5 | 40 |
| FALSE | 0.05 | 300 | 50 | 96.26 | 40 |
| TRUE | 0.1 | 300 | 50 | 92.92 | 1 |
| FALSE | 0.1 | 300 | 50 | 98.34 | 1 |
| TRUE | 0.1 | 300 | 50 | 90.64 | 4 |
| FALSE | 0.1 | 300 | 50 | 97.88 | 4 |
| TRUE | 0.1 | 300 | 50 | 34.98 | 40 |
| FALSE | 0.1 | 300 | 50 | 92.24 | 40 |
| TRUE | 0.25 | 300 | 50 | 91.52 | 1 |
| FALSE | 0.25 | 300 | 50 | 98.12 | 1 |
| TRUE | 0.25 | 300 | 50 | 83.52 | 4 |
| FALSE | 0.25 | 300 | 50 | 97 | 4 |
| TRUE | 0.25 | 300 | 50 | 2.92 | 40 |
| FALSE | 0.25 | 300 | 50 | 37.52 | 40 |
| TRUE | 0.05 | 300 | 100 | 93 | 1 |
| FALSE | 0.05 | 300 | 100 | 97.84 | 1 |
| TRUE | 0.05 | 300 | 100 | 90.3 | 4 |
| FALSE | 0.05 | 300 | 100 | 97.56 | 4 |
| TRUE | 0.05 | 300 | 100 | 28.88 | 40 |
| FALSE | 0.05 | 300 | 100 | 90.12 | 40 |
| TRUE | 0.1 | 300 | 100 | 90.86 | 1 |

(continued)

| Censored | Extra Sites Binding Probability | Number of Probes | Number of Probes Impacted | Sensitivity | Number of Unknown Extra Sites |
|---|---|---|---|---|---|
| FALSE | 0.1 | 300 | 100 | 97.96 | 1 |
| TRUE | 0.1 | 300 | 100 | 88.52 | 4 |
| FALSE | 0.1 | 300 | 100 | 97.9 | 4 |
| TRUE | 0.1 | 300 | 100 | 3.14 | 40 |
| FALSE | 0.1 | 300 | 100 | 35.04 | 40 |
| TRUE | 0.25 | 300 | 100 | 88.4 | 1 |
| FALSE | 0.25 | 300 | 100 | 97.68 | 1 |
| TRUE | 0.25 | 300 | 100 | 70.06 | 4 |
| FALSE | 0.25 | 300 | 100 | 95.26 | 4 |
| TRUE | 0.25 | 300 | 100 | 0.24 | 40 |
| FALSE | 0.25 | 300 | 100 | 0.08 | 40 |
| TRUE | 0.05 | 300 | 200 | 91.68 | 1 |
| FALSE | 0.05 | 300 | 200 | 98.22 | 1 |
| TRUE | 0.05 | 300 | 200 | 86.8 | 4 |
| FALSE | 0.05 | 300 | 200 | 98.1 | 4 |
| TRUE | 0.05 | 300 | 200 | 2.14 | 40 |
| FALSE | 0.05 | 300 | 200 | 26.82 | 40 |
| TRUE | 0.1 | 300 | 200 | 89.18 | 1 |
| FALSE | 0.1 | 300 | 200 | 97.96 | 1 |
| TRUE | 0.1 | 300 | 200 | 75.24 | 4 |
| FALSE | 0.1 | 300 | 200 | 96.36 | 4 |
| TRUE | 0.1 | 300 | 200 | 0.16 | 40 |
| FALSE | 0.1 | 300 | 200 | 0.16 | 40 |
| TRUE | 0.25 | 300 | 200 | 84.8 | 1 |
| FALSE | 0.25 | 300 | 200 | 96.7 | 1 |
| TRUE | 0.25 | 300 | 200 | 30.92 | 4 |
| FALSE | 0.25 | 300 | 200 | 90.92 | 4 |
| TRUE | 0.25 | 300 | 200 | 0.02 | 40 |
| FALSE | 0.25 | 300 | 200 | 0 | 40 |
| TRUE | 0.05 | 300 | 300 | 91.72 | 1 |
| FALSE | 0.05 | 300 | 300 | 97.68 | 1 |
| TRUE | 0.05 | 300 | 300 | 79.84 | 4 |
| FALSE | 0.05 | 300 | 300 | 96.88 | 4 |
| TRUE | 0.05 | 300 | 300 | 0.64 | 40 |
| FALSE | 0.05 | 300 | 300 | 1.26 | 40 |
| TRUE | 0.1 | 300 | 300 | 88.3 | 1 |
| FALSE | 0.1 | 300 | 300 | 98.34 | 1 |

(continued)

| Censored | Extra Sites Binding Probability | Number of Probes | Number of Probes Impacted | Sensitivity | Number of Unknown Extra Sites |
|---|---|---|---|---|---|
| TRUE | 0.1 | 300 | 300 | 54.92 | 4 |
| FALSE | 0.1 | 300 | 300 | 95.32 | 4 |
| TRUE | 0.1 | 300 | 300 | 0 | 40 |
| FALSE | 0.1 | 300 | 300 | 0 | 40 |
| TRUE | 0.25 | 300 | 300 | 74.6 | 1 |
| FALSE | 0.25 | 300 | 300 | 97.26 | 1 |
| TRUE | 0.25 | 300 | 300 | 6.22 | 4 |
| FALSE | 0.25 | 300 | 300 | 58.24 | 4 |
| TRUE | 0.25 | 300 | 300 | 0 | 40 |
| FALSE | 0.25 | 300 | 300 | 0 | 40 |

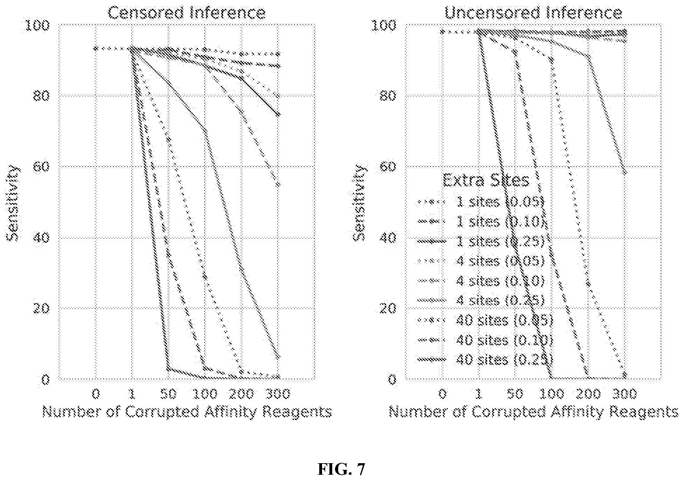

These results, which are plotted in **FIG. 7,** show that uncensored inference is more tolerant to the inclusion of additional hidden binding sites, and that the performance of both inference approaches is significantly compromised when 50 of the 300 affinity reagents contain 40 additional binding sites.

**Example 7: Performance of protein inference approaches using affinity reagents with missing binding epitopes**

In some cases, there may be improperly characterized affinity reagents with a number of annotated binding epitopes that do not exist (e.g., extra expected binding sites). That is, the model used to generate expected binding probabilities for an affinity reagent contains extra expected sites that do not exist. The sensitivity of censored protein identification and uncensored protein identification approaches with affinity reagent binding measurements were compared using affinity reagents that each bind random trimer sites (e.g. a targeted trimer, and four random off-target sites) with probability 0.25 that are input into the protein identification algorithm. A subset of the affinity reagents (0 of 300, 1 of 300, 50 of 300, 100 of 300, 200 of 300, or 300 of 300) had either 1, 4, or 40 extra expected binding sites each against a random trimer with binding probability 0.05, 0.1 or 0.25 added to the model for the affinity reagent used by the protein inference algorithm. The results of the analysis are shown in Table 6.

**Table 6**

| Censored | Extra Sites Binding Probability | Number of Extra Sites | Number of Probes | Number of Probes Impacted | Sensitivity |
|---|---|---|---|---|---|
| TRUE | 0.05 | 1 | 300 | 0 | 93.32 |
| FALSE | 0.05 | 1 | 300 | 0 | 98.04 |
| TRUE | 0.05 | 1 | 300 | 1 | 94.06 |
| FALSE | 0.05 | 1 | 300 | 1 | 98.6 |
| TRUE | 0.05 | 4 | 300 | 1 | 93.08 |
| FALSE | 0.05 | 4 | 300 | 1 | 98.6 |
| TRUE | 0.05 | 40 | 300 | 1 | 93.38 |
| FALSE | 0.05 | 40 | 300 | 1 | 98.1 |
| TRUE | 0.1 | 1 | 300 | 1 | 92.98 |
| FALSE | 0.1 | 1 | 300 | 1 | 97.88 |
| TRUE | 0.1 | 4 | 300 | 1 | 93.54 |
| FALSE | 0.1 | 4 | 300 | 1 | 98.2 |
| TRUE | 0.1 | 40 | 300 | 1 | 93.26 |

(continued)

| Censored | Extra Sites Binding Probability | Number of Extra Sites | Number of Probes | Number of Probes Impacted | Sensitivity |
|---|---|---|---|---|---|
| FALSE | 0.1 | 40 | 300 | 1 | 98.12 |
| TRUE | 0.25 | 1 | 300 | 1 | 92.98 |
| FALSE | 0.25 | 1 | 300 | 1 | 97.62 |
| TRUE | 0.25 | 4 | 300 | 1 | 92.7 |
| FALSE | 0.25 | 4 | 300 | 1 | 98.16 |
| TRUE | 0.25 | 40 | 300 | 1 | 93.06 |
| FALSE | 0.25 | 40 | 300 | 1 | 97.66 |
| TRUE | 0.05 | 1 | 300 | 50 | 92.4 |
| FALSE | 0.05 | 1 | 300 | 50 | 98.2 |
| TRUE | 0.05 | 4 | 300 | 50 | 92.66 |
| FALSE | 0.05 | 4 | 300 | 50 | 98.1 |
| TRUE | 0.05 | 40 | 300 | 50 | 91.14 |
| FALSE | 0.05 | 40 | 300 | 50 | 97.66 |
| TRUE | 0.1 | 1 | 300 | 50 | 93.22 |
| FALSE | 0.1 | 1 | 300 | 50 | 97.9 |
| TRUE | 0.1 | 4 | 300 | 50 | 92.04 |
| FALSE | 0.1 | 4 | 300 | 50 | 97.56 |
| TRUE | 0.1 | 40 | 300 | 50 | 87.74 |
| FALSE | 0.1 | 40 | 300 | 50 | 97.08 |
| TRUE | 0.25 | 1 | 300 | 50 | 92.28 |
| FALSE | 0.25 | 1 | 300 | 50 | 98.26 |
| TRUE | 0.25 | 4 | 300 | 50 | 91.8 |
| FALSE | 0.25 | 4 | 300 | 50 | 97.62 |
| TRUE | 0.25 | 40 | 300 | 50 | 87.16 |
| FALSE | 0.25 | 40 | 300 | 50 | 93.52 |
| TRUE | 0.05 | 1 | 300 | 100 | 91.9 |
| FALSE | 0.05 | 1 | 300 | 100 | 97.64 |
| TRUE | 0.05 | 4 | 300 | 100 | 92.74 |
| FALSE | 0.05 | 4 | 300 | 100 | 98.02 |
| TRUE | 0.05 | 40 | 300 | 100 | 84.18 |
| FALSE | 0.05 | 40 | 300 | 100 | 97.42 |
| TRUE | 0.1 | 1 | 300 | 100 | 92.82 |
| FALSE | 0.1 | 1 | 300 | 100 | 98.08 |
| TRUE | 0.1 | 4 | 300 | 100 | 92.46 |
| FALSE | 0.1 | 4 | 300 | 100 | 97.82 |
| TRUE | 0.1 | 40 | 300 | 100 | 76.28 |
| FALSE | 0.1 | 40 | 300 | 100 | 95.2 |

(continued)

| Censored | Extra Sites Binding Probability | Number of Extra Sites | Number of Probes | Number of Probes Impacted | Sensitivity |
|---|---|---|---|---|---|
| TRUE | 0.25 | 1 | 300 | 100 | 91.18 |
| FALSE | 0.25 | 1 | 300 | 100 | 97.84 |
| TRUE | 0.25 | 4 | 300 | 100 | 90.38 |
| FALSE | 0.25 | 4 | 300 | 100 | 97.64 |
| TRUE | 0.25 | 40 | 300 | 100 | 60.5 |
| FALSE | 0.25 | 40 | 300 | 100 | 46.34 |
| TRUE | 0.05 | 1 | 300 | 200 | 93.32 |
| FALSE | 0.05 | 1 | 300 | 200 | 98.16 |
| TRUE | 0.05 | 4 | 300 | 200 | 90.42 |
| FALSE | 0.05 | 4 | 300 | 200 | 97.68 |
| TRUE | 0.05 | 40 | 300 | 200 | 74.82 |
| FALSE | 0.05 | 40 | 300 | 200 | 92.86 |
| TRUE | 0.1 | 1 | 300 | 200 | 93.28 |
| FALSE | 0.1 | 1 | 300 | 200 | 98.2 |
| TRUE | 0.1 | 4 | 300 | 200 | 90.62 |
| FALSE | 0.1 | 4 | 300 | 200 | 98.04 |
| TRUE | 0.1 | 40 | 300 | 200 | 55.4 |
| FALSE | 0.1 | 40 | 300 | 200 | 46.62 |
| TRUE | 0.25 | 1 | 300 | 200 | 92.14 |
| FALSE | 0.25 | 1 | 300 | 200 | 97.88 |
| TRUE | 0.25 | 4 | 300 | 200 | 85.22 |
| FALSE | 0.25 | 4 | 300 | 200 | 96.68 |
| TRUE | 0.25 | 40 | 300 | 200 | 4.92 |
| FALSE | 0.25 | 40 | 300 | 200 | 0.34 |
| TRUE | 0.05 | 1 | 300 | 300 | 92.8 |
| FALSE | 0.05 | 1 | 300 | 300 | 98.34 |
| TRUE | 0.05 | 4 | 300 | 300 | 91.04 |
| FALSE | 0.05 | 4 | 300 | 300 | 97.9 |
| TRUE | 0.05 | 40 | 300 | 300 | 53.2 |
| FALSE | 0.05 | 40 | 300 | 300 | 54.84 |
| TRUE | 0.1 | 1 | 300 | 300 | 91.28 |
| FALSE | 0.1 | 1 | 300 | 300 | 97.44 |
| TRUE | 0.1 | 4 | 300 | 300 | 85.08 |
| FALSE | 0.1 | 4 | 300 | 300 | 97.08 |
| TRUE | 0.1 | 40 | 300 | 300 | 10.66 |
| FALSE | 0.1 | 40 | 300 | 300 | 1.76 |
| TRUE | 0.25 | 1 | 300 | 300 | 90.64 |

(continued)

| Censored | Extra Sites Binding Probability | Number of Extra Sites | Number of Probes | Number of Probes Impacted | Sensitivity |
|---|---|---|---|---|---|
| FALSE | 0.25 | 1 | 300 | 300 | 97.54 |
| TRUE | 0.25 | 4 | 300 | 300 | 78.6 |
| FALSE | 0.25 | 4 | 300 | 300 | 95.36 |
| TRUE | 0.25 | 40 | 300 | 300 | 0.06 |
| FALSE | 0.25 | 40 | 300 | 300 | 0 |

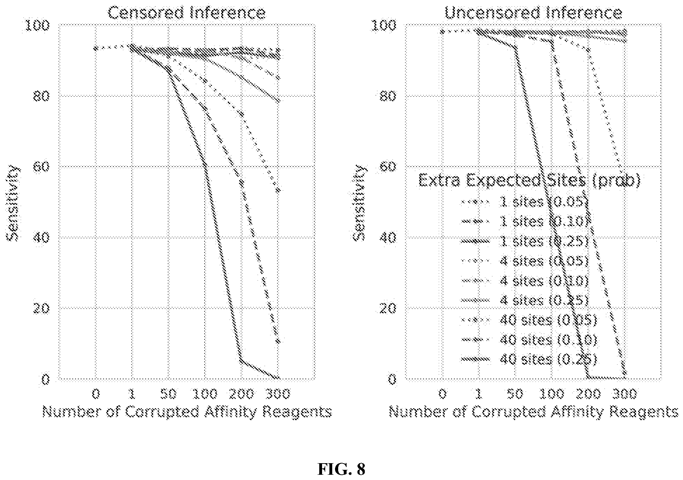

These results, which are plotted in **FIG. 8,** show that uncensored inference is more tolerant to the inclusion of extra expected binding sites included in the model of affinity reagent binding, and that the performance of both protein identification approaches is compromised to some degree when the majority of affinity reagents contain 40 extra expected binding sites.

**Example 8: Censored inference for affinity reagent binding analysis with an alternative scaling strategy**

The methods described herein may be applied to infer protein identity (e.g., identify unknown proteins) using affinity reagent binding measurements in combination with various probability scaling strategies. The censored inference approach described in **Example 3** scales the probability of an observed outcome for a protein based on the number of potential binding sites on the protein (protein length - 2) and the number of observed binding outcomes (M):

$$SL_{Protein} = \frac{P(outcome\ set\ |\ protein)}{\binom{L-2}{M}}$$

The methods described herein may be applied with alternative approaches for computing scaled likelihoods. This example applies an alternative approach for normalization that models the probability of generating N binding events for a protein of length k from the set of affinity reagents used to measure the protein, and scales based on this probability. First, for each probe, the probability of the probe binding a trimer of unknown identity in the sample is calculated:

$$P(trimer\ bind\ |probe_i) = \sum_{j=1}^{j=8000} P(trimer_j)P(probe_i\ bind\ |trimer_j)$$

where $P(trimer_j)$ is the frequency with which the trimer occurs relative to the summed count of all 8,000 trimers in the proteome. For any protein of length $k,$ the probability of a probe $i$ binding the protein may be given by:

$$P(protein\ bind\ |\ probe_i, k) = 1 - (1 - P(trimer\ bind\ |probe_i))^{k-2}$$

The number of successful binding events observed for a protein of length $k$ may follow a Poisson-Binomial distribution with $n$ trials, where $n$ is the number of probe binding measurements made for the protein and the parameters $\mathbf{p}_{probes,k}$ of the distribution indicate the probability of success for each trial:

$$\mathbf{p}_{probes,k} =$$
$$[P(bind\ |probe_1, k), P(bind\ |probe_2, k), P(bind\ |probe_3, k) \ldots P(bind\ |probe_n, k)].$$

The probability of generating N binding events from a protein of length $k$, with a particular set of probes, may be given by the probability mass function of the Poisson binomial distribution ($PMF_{PoiBin}$) parameterized by $\mathbf{p}$, evaluated

at N:

$$P(N\ binding\ events\ |\ probes, k) = PMF_{PoiBin}(N, \mathbf{p_{probes,k}})$$

The scaled likelihood of a particular outcome set is computed based on this probability:

$$SL_{protein, binding\ events} = \frac{P(outcome\ set\ |\ protein)}{P(N\ binding\ events\ |probes, k)}$$

**Example 9: Using Randomly Selected Affinity Reagents**

The methods described herein may be applied to any set of affinity reagents. For example, the protein identification approach may be applied to affinity reagents targeting the most abundant trimers in the proteome, or targeting random trimers. The results from a human protein inference analysis using affinity reagents targeting the top 300 most abundant trimers in the proteome, 300 randomly selected trimers in the proteome, or the 300 least abundant trimers in the proteome are shown in Tables 7a-7c.

**Tables 7a-c**

| Table 7a - 300 affinity reagents targeting the least-common trimers in the proteome | | | | |
|---|---|---|---|---|
| **Number of Probes** | **Probe Set ID** | **Experiment Repetition** | **Selection Type** | **Sensitivity** |
| 300 | 100 | 0 | Bottom 300 | 91.9 |
| 300 | 100 | 1 | Bottom 300 | 91.24 |
| 300 | 100 | 2 | Bottom 300 | 91.74 |
| 300 | 100 | 3 | Bottom 300 | 90.9 |
| 300 | 100 | 4 | Bottom 300 | 90.46 |
| Table 7b - 300 affinity reagents targeting random trimers in the proteome | | | | |
| **Number of Probes** | **Probe Set ID** | **Experiment Repetition** | **Selection Type** | **Sensitivity** |
| 300 | 0 | 0 | Random | 94.4 |
| 300 | 0 | 1 | Random | 94.2 |
| 300 | 0 | 2 | Random | 94.18 |
| 300 | 0 | 3 | Random | 94.64 |
| 300 | 0 | 4 | Random | 94.24 |
| 300 | 1 | 0 | Random | 94.12 |
| 300 | 1 | 1 | Random | 94.08 |
| 300 | 1 | 2 | Random | 94.12 |
| 300 | 1 | 3 | Random | 93.7 |
| 300 | 1 | 4 | Random | 93.54 |
| 300 | 2 | 0 | Random | 93.68 |
| 300 | 2 | 1 | Random | 93.68 |
| 300 | 2 | 2 | Random | 93.68 |
| 300 | 2 | 3 | Random | 93.74 |
| 300 | 2 | 4 | Random | 93.9 |
| 300 | 3 | 0 | Random | 95.12 |
| 300 | 3 | 1 | Random | 94.38 |

(continued)

| Table 7b - 300 affinity reagents targeting random trimers in the proteome | | | | |
|---|---|---|---|---|
| Number of Probes | Probe Set ID | Experiment Repetition | Selection Type | Sensitivity |
| 300 | 3 | 2 | Random | 94.76 |
| 300 | 3 | 3 | Random | 95.4 |
| Number of Probes | Probe Set ID | Experiment Repetition | Selection Type | Sensitivity |
| 300 | 3 | 4 | Random | 94.6 |
| 300 | 4 | 0 | Random | 94.46 |
| 300 | 4 | 1 | Random | 94.74 |
| 300 | 4 | 2 | Random | 95.04 |
| 300 | 4 | 3 | Random | 94.66 |
| 300 | 4 | 4 | Random | 94.76 |
| 300 | 5 | 0 | Random | 94.58 |
| 300 | 5 | 1 | Random | 94.62 |
| 300 | 5 | 2 | Random | 94.48 |
| 300 | 5 | 3 | Random | 94.48 |
| 300 | 5 | 4 | Random | 95 |
| 300 | 6 | 0 | Random | 93.18 |
| 300 | 6 | 1 | Random | 93.44 |
| 300 | 6 | 2 | Random | 93.28 |
| 300 | 6 | 3 | Random | 93.8 |
| 300 | 6 | 4 | Random | 94.26 |
| 300 | 7 | 0 | Random | 95.16 |
| 300 | 7 | 1 | Random | 94.02 |
| 300 | 7 | 2 | Random | 95 |
| 300 | 7 | 3 | Random | 95.1 |
| 300 | 7 | 4 | Random | 94.86 |
| 300 | 8 | 0 | Random | 93.56 |
| 300 | 8 | 1 | Random | 95.5 |
| 300 | 8 | 2 | Random | 94.7 |
| 300 | 8 | 3 | Random | 94.72 |
| 300 | 8 | 4 | Random | 94.94 |
| 300 | 9 | 0 | Random | 94.46 |
| 300 | 9 | 1 | Random | 95.44 |
| 300 | 9 | 2 | Random | 93.98 |
| 300 | 9 | 3 | Random | 94.58 |
| 300 | 9 | 4 | Random | 94.34 |
| 300 | 10 | 0 | Random | 94.54 |
| 300 | 10 | 1 | Random | 94.56 |
| 300 | 10 | 2 | Random | 94.78 |

(continued)

| Number of Probes | Probe Set ID | Experiment Repetition | Selection Type | Sensitivity |
|---|---|---|---|---|
| 300 | 10 | 3 | Random | 94.86 |
| 300 | 10 | 4 | Random | 95.08 |
| 300 | 11 | 0 | Random | 94.36 |
| 300 | 11 | 1 | Random | 94.86 |
| 300 | 11 | 2 | Random | 95.3 |
| 300 | 11 | 3 | Random | 94.16 |
| 300 | 11 | 4 | Random | 94.9 |
| 300 | 12 | 0 | Random | 94.92 |
| 300 | 12 | 1 | Random | 94.66 |
| 300 | 12 | 2 | Random | 94.26 |
| 300 | 12 | 3 | Random | 94.58 |
| 300 | 12 | 4 | Random | 94.02 |
| 300 | 13 | 0 | Random | 94.78 |
| 300 | 13 | 1 | Random | 94.54 |
| 300 | 13 | 2 | Random | 95.02 |
| 300 | 13 | 3 | Random | 94.94 |
| 300 | 13 | 4 | Random | 94.98 |
| 300 | 14 | 0 | Random | 95.3 |
| 300 | 14 | 1 | Random | 94.36 |
| 300 | 14 | 2 | Random | 94.76 |
| 300 | 14 | 3 | Random | 95.26 |
| 300 | 14 | 4 | Random | 94.52 |
| 300 | 15 | 0 | Random | 94.48 |
| 300 | 15 | 1 | Random | 94.6 |
| 300 | 15 | 2 | Random | 94.98 |
| 300 | 15 | 3 | Random | 94.6 |
| 300 | 15 | 4 | Random | 95.8 |
| 300 | 16 | 0 | Random | 94.58 |
| 300 | 16 | 1 | Random | 92.96 |
| 300 | 16 | 2 | Random | 94.6 |
| 300 | 16 | 3 | Random | 93.84 |
| 300 | 16 | 4 | Random | 94.38 |
| 300 | 17 | 0 | Random | 94.76 |
| 300 | 17 | 1 | Random | 94.54 |
| 300 | 17 | 2 | Random | 94.72 |
| 300 | 17 | 3 | Random | 94.24 |
| 300 | 17 | 4 | Random | 94.12 |
| 300 | 18 | 0 | Random | 94.16 |

(continued)

| Number of Probes | Probe Set ID | Experiment Repetition | Selection Type | Sensitivity |
|---|---|---|---|---|
| 300 | 18 | 1 | Random | 94.1 |
| 300 | 18 | 2 | Random | 94.86 |
| 300 | 18 | 3 | Random | 93.98 |
| 300 | 18 | 4 | Random | 95.04 |
| 300 | 19 | 0 | Random | 93.58 |
| 300 | 19 | 1 | Random | 94.94 |
| 300 | 19 | 2 | Random | 95.12 |
| 300 | 19 | 3 | Random | 94.8 |
| 300 | 19 | 4 | Random | 94.8 |
| 300 | 20 | 0 | Random | 93 |
| 300 | 20 | 1 | Random | 94.22 |
| 300 | 20 | 2 | Random | 94.4 |
| 300 | 20 | 3 | Random | 93.64 |
| 300 | 20 | 4 | Random | 94.76 |
| 300 | 21 | 0 | Random | 93.68 |
| 300 | 21 | 1 | Random | 94.18 |
| 300 | 21 | 2 | Random | 94.38 |
| 300 | 21 | 3 | Random | 94.48 |
| 300 | 21 | 4 | Random | 94.68 |
| 300 | 22 | 0 | Random | 93.66 |
| 300 | 22 | 1 | Random | 94.16 |
| 300 | 22 | 2 | Random | 94.1 |
| 300 | 22 | 3 | Random | 94.16 |
| 300 | 22 | 4 | Random | 94.1 |
| 300 | 23 | 0 | Random | 93.94 |
| 300 | 23 | 1 | Random | 94.42 |
| 300 | 23 | 2 | Random | 94.24 |
| 300 | 23 | 3 | Random | 93.9 |
| 300 | 23 | 4 | Random | 94.4 |
| 300 | 24 | 0 | Random | 95 |
| 300 | 24 | 1 | Random | 94.82 |
| 300 | 24 | 2 | Random | 94.16 |
| 300 | 24 | 3 | Random | 94.58 |
| 300 | 24 | 4 | Random | 94.54 |
| 300 | 25 | 0 | Random | 94.5 |
| 300 | 25 | 1 | Random | 95.1 |
| 300 | 25 | 2 | Random | 95.3 |
| 300 | 25 | 3 | Random | 94.54 |

(continued)

| Number of Probes | Probe Set ID | Experiment Repetition | Selection Type | Sensitivity |
|---|---|---|---|---|
| 300 | 25 | 4 | Random | 95.22 |
| 300 | 26 | 0 | Random | 94.22 |
| 300 | 26 | 1 | Random | 94.08 |
| 300 | 26 | 2 | Random | 94.52 |
| 300 | 26 | 3 | Random | 94.3 |
| 300 | 26 | 4 | Random | 94.6 |
| 300 | 27 | 0 | Random | 93.92 |
| 300 | 27 | 1 | Random | 94.24 |
| 300 | 27 | 2 | Random | 93.64 |
| 300 | 27 | 3 | Random | 93.84 |
| 300 | 27 | 4 | Random | 94.04 |
| 300 | 28 | 0 | Random | 94.08 |
| 300 | 28 | 1 | Random | 95.14 |
| 300 | 28 | 2 | Random | 94.82 |
| 300 | 28 | 3 | Random | 94.7 |
| 300 | 28 | 4 | Random | 94.92 |
| 300 | 29 | 0 | Random | 94.82 |
| 300 | 29 | 1 | Random | 93.76 |
| 300 | 29 | 2 | Random | 93.98 |
| 300 | 29 | 3 | Random | 93.14 |
| 300 | 29 | 4 | Random | 94.46 |
| 300 | 30 | 0 | Random | 94.6 |
| 300 | 30 | 1 | Random | 96.22 |
| 300 | 30 | 2 | Random | 95.06 |
| 300 | 30 | 3 | Random | 95.12 |
| 300 | 30 | 4 | Random | 94.82 |
| 300 | 31 | 0 | Random | 93.12 |
| 300 | 31 | 1 | Random | 93.92 |
| 300 | 31 | 2 | Random | 93.3 |
| 300 | 31 | 3 | Random | 94.7 |
| 300 | 31 | 4 | Random | 94.22 |
| 300 | 32 | 0 | Random | 93.7 |
| 300 | 32 | 1 | Random | 94.62 |
| 300 | 32 | 2 | Random | 94.12 |
| 300 | 32 | 3 | Random | 94.08 |
| 300 | 32 | 4 | Random | 94.72 |
| 300 | 33 | 0 | Random | 94.82 |
| 300 | 33 | 1 | Random | 93.44 |

(continued)

| Number of Probes | Probe Set ID | Experiment Repetition | Selection Type | Sensitivity |
|---|---|---|---|---|
| 300 | 33 | 2 | Random | 94.06 |
| 300 | 33 | 3 | Random | 94.54 |
| 300 | 33 | 4 | Random | 94.42 |
| 300 | 34 | 0 | Random | 94.16 |
| 300 | 34 | 1 | Random | 93.28 |
| 300 | 34 | 2 | Random | 94.9 |
| 300 | 34 | 3 | Random | 93.12 |
| 300 | 34 | 4 | Random | 94.3 |
| 300 | 35 | 0 | Random | 94.54 |
| 300 | 35 | 1 | Random | 93.56 |
| 300 | 35 | 2 | Random | 93.4 |
| 300 | 35 | 3 | Random | 93.78 |
| 300 | 35 | 4 | Random | 94.5 |
| 300 | 36 | 0 | Random | 94.34 |
| 300 | 36 | 1 | Random | 93.9 |
| 300 | 36 | 2 | Random | 94.7 |
| 300 | 36 | 3 | Random | 95.12 |
| 300 | 36 | 4 | Random | 94.8 |
| 300 | 37 | 0 | Random | 94.38 |
| 300 | 37 | 1 | Random | 95.22 |
| 300 | 37 | 2 | Random | 94.98 |
| 300 | 37 | 3 | Random | 94.12 |
| 300 | 37 | 4 | Random | 95.06 |
| 300 | 38 | 0 | Random | 94.34 |
| 300 | 38 | 1 | Random | 94.82 |
| 300 | 38 | 2 | Random | 93.8 |
| 300 | 38 | 3 | Random | 94.8 |
| 300 | 38 | 4 | Random | 95.1 |
| 300 | 39 | 0 | Random | 93.72 |
| 300 | 39 | 1 | Random | 93.7 |
| 300 | 39 | 2 | Random | 94.12 |
| 300 | 39 | 3 | Random | 94.04 |
| 300 | 39 | 4 | Random | 93.98 |
| 300 | 40 | 0 | Random | 94.42 |
| 300 | 40 | 1 | Random | 93.86 |
| 300 | 40 | 2 | Random | 93.46 |
| 300 | 40 | 3 | Random | 94.34 |
| 300 | 40 | 4 | Random | 94.12 |

(continued)

| Number of Probes | Probe Set ID | Experiment Repetition | Selection Type | Sensitivity |
|---|---|---|---|---|
| 300 | 41 | 0 | Random | 94.16 |
| 300 | 41 | 1 | Random | 95 |
| 300 | 41 | 2 | Random | 95.22 |
| 300 | 41 | 3 | Random | 95.38 |
| 300 | 41 | 4 | Random | 95.36 |
| 300 | 42 | 0 | Random | 93.36 |
| 300 | 42 | 1 | Random | 94.38 |
| 300 | 42 | 2 | Random | 94.28 |
| 300 | 42 | 3 | Random | 94.52 |
| 300 | 42 | 4 | Random | 93.94 |
| 300 | 43 | 0 | Random | 95.5 |
| 300 | 43 | 1 | Random | 95.04 |
| 300 | 43 | 2 | Random | 95.32 |
| 300 | 43 | 3 | Random | 94.84 |
| 300 | 43 | 4 | Random | 95.26 |
| 300 | 44 | 0 | Random | 94.74 |
| 300 | 44 | 1 | Random | 94.6 |
| 300 | 44 | 2 | Random | 93.8 |
| 300 | 44 | 3 | Random | 94.04 |
| 300 | 44 | 4 | Random | 94.22 |
| 300 | 45 | 0 | Random | 93.64 |
| 300 | 45 | 1 | Random | 93.78 |
| 300 | 45 | 2 | Random | 94.12 |
| 300 | 45 | 3 | Random | 94.48 |
| 300 | 45 | 4 | Random | 94.66 |
| 300 | 46 | 0 | Random | 94.48 |
| 300 | 46 | 1 | Random | 94.92 |
| 300 | 46 | 2 | Random | 95.04 |
| 300 | 46 | 3 | Random | 94.14 |
| 300 | 46 | 4 | Random | 94.6 |
| 300 | 47 | 0 | Random | 94.2 |
| 300 | 47 | 1 | Random | 93.56 |
| 300 | 47 | 2 | Random | 95.36 |
| 300 | 47 | 3 | Random | 95.64 |
| 300 | 47 | 4 | Random | 94.18 |
| 300 | 48 | 0 | Random | 94.38 |
| 300 | 48 | 1 | Random | 95.1 |
| 300 | 48 | 2 | Random | 94.24 |

(continued)

| Number of Probes | Probe Set ID | Experiment Repetition | Selection Type | Sensitivity |
|---|---|---|---|---|
| 300 | 48 | 3 | Random | 94.6 |
| 300 | 48 | 4 | Random | 94.76 |
| 300 | 49 | 0 | Random | 94.98 |
| 300 | 49 | 1 | Random | 95.9 |
| 300 | 49 | 2 | Random | 95.08 |
| 300 | 49 | 3 | Random | 94.72 |
| 300 | 49 | 4 | Random | 94.02 |
| 300 | 50 | 0 | Random | 94.72 |
| 300 | 50 | 1 | Random | 94.44 |
| 300 | 50 | 2 | Random | 95.84 |
| 300 | 50 | 3 | Random | 95 |
| 300 | 50 | 4 | Random | 94.62 |
| 300 | 51 | 0 | Random | 94.92 |
| 300 | 51 | 1 | Random | 94.26 |
| 300 | 51 | 2 | Random | 94.34 |
| 300 | 51 | 3 | Random | 94.66 |
| 300 | 51 | 4 | Random | 93.58 |
| 300 | 52 | 0 | Random | 94.98 |
| 300 | 52 | 1 | Random | 95.12 |
| 300 | 52 | 2 | Random | 94.88 |
| 300 | 52 | 3 | Random | 94.78 |
| 300 | 52 | 4 | Random | 94.88 |
| 300 | 53 | 0 | Random | 94.88 |
| 300 | 53 | 1 | Random | 95.04 |
| 300 | 53 | 2 | Random | 94.18 |
| 300 | 53 | 3 | Random | 94.04 |
| 300 | 53 | 4 | Random | 94.56 |
| 300 | 54 | 0 | Random | 94.26 |
| 300 | 54 | 1 | Random | 94.1 |
| 300 | 54 | 2 | Random | 95.32 |
| 300 | 54 | 3 | Random | 94.44 |
| 300 | 54 | 4 | Random | 94.74 |
| 300 | 55 | 0 | Random | 94.68 |
| 300 | 55 | 1 | Random | 94.68 |
| 300 | 55 | 2 | Random | 95.52 |
| 300 | 55 | 3 | Random | 94.54 |
| 300 | 55 | 4 | Random | 95.12 |
| 300 | 56 | 0 | Random | 94.58 |

(continued)

| Number of Probes | Probe Set ID | Experiment Repetition | Selection Type | Sensitivity |
|---|---|---|---|---|
| 300 | 56 | 1 | Random | 95.14 |
| 300 | 56 | 2 | Random | 94.58 |
| 300 | 56 | 3 | Random | 95.18 |
| 300 | 56 | 4 | Random | 94.84 |
| 300 | 57 | 0 | Random | 94.54 |
| 300 | 57 | 1 | Random | 93.82 |
| 300 | 57 | 2 | Random | 94.92 |
| 300 | 57 | 3 | Random | 95.14 |
| 300 | 57 | 4 | Random | 94.26 |
| 300 | 58 | 0 | Random | 94.36 |
| 300 | 58 | 1 | Random | 94.74 |
| 300 | 58 | 2 | Random | 94.92 |
| 300 | 58 | 3 | Random | 94.36 |
| 300 | 58 | 4 | Random | 94.28 |
| 300 | 59 | 0 | Random | 94.54 |
| 300 | 59 | 1 | Random | 93.92 |
| 300 | 59 | 2 | Random | 95.04 |
| 300 | 59 | 3 | Random | 95.4 |
| 300 | 59 | 4 | Random | 93.76 |
| 300 | 60 | 0 | Random | 94.8 |
| 300 | 60 | 1 | Random | 94.74 |
| 300 | 60 | 2 | Random | 93.82 |
| 300 | 60 | 3 | Random | 94.54 |
| 300 | 60 | 4 | Random | 93.86 |
| 300 | 61 | 0 | Random | 94.5 |
| 300 | 61 | 1 | Random | 94.76 |
| 300 | 61 | 2 | Random | 94.3 |
| 300 | 61 | 3 | Random | 94.68 |
| 300 | 61 | 4 | Random | 94.42 |
| 300 | 62 | 0 | Random | 93.72 |
| 300 | 62 | 1 | Random | 94.94 |
| 300 | 62 | 2 | Random | 94.12 |
| 300 | 62 | 3 | Random | 93.86 |
| 300 | 62 | 4 | Random | 95.38 |
| 300 | 63 | 0 | Random | 95.1 |
| 300 | 63 | 1 | Random | 95.4 |
| 300 | 63 | 2 | Random | 94.94 |
| 300 | 63 | 3 | Random | 94.62 |

(continued)

| Number of Probes | Probe Set ID | Experiment Repetition | Selection Type | Sensitivity |
|---|---|---|---|---|
| 300 | 63 | 4 | Random | 94.32 |
| 300 | 64 | 0 | Random | 94.96 |
| 300 | 64 | 1 | Random | 94.02 |
| 300 | 64 | 2 | Random | 94.52 |
| 300 | 64 | 3 | Random | 93.98 |
| 300 | 64 | 4 | Random | 94.48 |
| 300 | 65 | 0 | Random | 93.6 |
| 300 | 65 | 1 | Random | 94.4 |
| 300 | 65 | 2 | Random | 93.38 |
| 300 | 65 | 3 | Random | 94.54 |
| 300 | 65 | 4 | Random | 93.14 |
| 300 | 66 | 0 | Random | 94.44 |
| 300 | 66 | 1 | Random | 94.2 |
| 300 | 66 | 2 | Random | 94.9 |
| 300 | 66 | 3 | Random | 94.68 |
| 300 | 66 | 4 | Random | 94.6 |
| 300 | 67 | 0 | Random | 94.3 |
| 300 | 67 | 1 | Random | 94.08 |
| 300 | 67 | 2 | Random | 94.56 |
| 300 | 67 | 3 | Random | 93.78 |
| 300 | 67 | 4 | Random | 94.52 |
| 300 | 68 | 0 | Random | 93.24 |
| 300 | 68 | 1 | Random | 93.76 |
| 300 | 68 | 2 | Random | 94.8 |
| 300 | 68 | 3 | Random | 94.36 |
| 300 | 68 | 4 | Random | 93.76 |
| 300 | 69 | 0 | Random | 94.58 |
| 300 | 69 | 1 | Random | 94.52 |
| 300 | 69 | 2 | Random | 94.72 |
| 300 | 69 | 3 | Random | 94.88 |
| 300 | 69 | 4 | Random | 93.38 |
| 300 | 70 | 0 | Random | 95.34 |
| 300 | 70 | 1 | Random | 94.52 |
| 300 | 70 | 2 | Random | 94.38 |
| 300 | 70 | 3 | Random | 94.94 |
| 300 | 70 | 4 | Random | 93.6 |
| 300 | 71 | 0 | Random | 93.8 |
| 300 | 71 | 1 | Random | 94.38 |

(continued)

| Number of Probes | Probe Set ID | Experiment Repetition | Selection Type | Sensitivity |
|---|---|---|---|---|
| 300 | 71 | 2 | Random | 94.32 |
| 300 | 71 | 3 | Random | 93.2 |
| 300 | 71 | 4 | Random | 94.28 |
| 300 | 72 | 0 | Random | 94.76 |
| 300 | 72 | 1 | Random | 95 |
| 300 | 72 | 2 | Random | 95.64 |
| 300 | 72 | 3 | Random | 95.28 |
| 300 | 72 | 4 | Random | 95.68 |
| 300 | 73 | 0 | Random | 94.92 |
| 300 | 73 | 1 | Random | 94.52 |
| 300 | 73 | 2 | Random | 94.36 |
| 300 | 73 | 3 | Random | 94.38 |
| 300 | 73 | 4 | Random | 94.56 |
| 300 | 74 | 0 | Random | 94.62 |
| 300 | 74 | 1 | Random | 94.18 |
| 300 | 74 | 2 | Random | 94.38 |
| 300 | 74 | 3 | Random | 94.38 |
| 300 | 74 | 4 | Random | 93.5 |
| 300 | 75 | 0 | Random | 95.32 |
| 300 | 75 | 1 | Random | 95.42 |
| 300 | 75 | 2 | Random | 94.9 |
| 300 | 75 | 3 | Random | 94.96 |
| 300 | 75 | 4 | Random | 94.1 |
| 300 | 76 | 0 | Random | 94.9 |
| 300 | 76 | 1 | Random | 95.46 |
| 300 | 76 | 2 | Random | 94.72 |
| 300 | 76 | 3 | Random | 94.54 |
| 300 | 76 | 4 | Random | 94.16 |
| 300 | 77 | 0 | Random | 94.14 |
| 300 | 77 | 1 | Random | 93.94 |
| 300 | 77 | 2 | Random | 94.28 |
| 300 | 77 | 3 | Random | 94.62 |
| 300 | 77 | 4 | Random | 94.38 |
| 300 | 78 | 0 | Random | 93.8 |
| 300 | 78 | 1 | Random | 93.84 |
| 300 | 78 | 2 | Random | 94.56 |
| 300 | 78 | 3 | Random | 94.18 |
| 300 | 78 | 4 | Random | 93.76 |

(continued)

| Number of Probes | Probe Set ID | Experiment Repetition | Selection Type | Sensitivity |
|---|---|---|---|---|
| 300 | 79 | 0 | Random | 94.28 |
| 300 | 79 | 1 | Random | 93.66 |
| 300 | 79 | 2 | Random | 93.76 |
| 300 | 79 | 3 | Random | 94.6 |
| 300 | 79 | 4 | Random | 95.76 |
| 300 | 80 | 0 | Random | 94.52 |
| 300 | 80 | 1 | Random | 94.82 |
| 300 | 80 | 2 | Random | 93.82 |
| 300 | 80 | 3 | Random | 94.9 |
| 300 | 80 | 4 | Random | 94.3 |
| 300 | 81 | 0 | Random | 94.84 |
| 300 | 81 | 1 | Random | 94.82 |
| 300 | 81 | 2 | Random | 94.76 |
| 300 | 81 | 3 | Random | 94.54 |
| 300 | 81 | 4 | Random | 94.74 |
| 300 | 82 | 0 | Random | 95.26 |
| 300 | 82 | 1 | Random | 94.32 |
| 300 | 82 | 2 | Random | 94.04 |
| 300 | 82 | 3 | Random | 94.98 |
| 300 | 82 | 4 | Random | 94.56 |
| 300 | 83 | 0 | Random | 94.9 |
| 300 | 83 | 1 | Random | 94.76 |
| 300 | 83 | 2 | Random | 94.06 |
| 300 | 83 | 3 | Random | 94.46 |
| 300 | 83 | 4 | Random | 94.8 |
| 300 | 84 | 0 | Random | 93.66 |
| 300 | 84 | 1 | Random | 93.28 |
| 300 | 84 | 2 | Random | 94.64 |
| 300 | 84 | 3 | Random | 93.58 |
| 300 | 84 | 4 | Random | 93.86 |
| 300 | 85 | 0 | Random | 94.16 |
| 300 | 85 | 1 | Random | 93.06 |
| 300 | 85 | 2 | Random | 94.02 |
| 300 | 85 | 3 | Random | 93.1 |
| 300 | 85 | 4 | Random | 94.3 |
| 300 | 86 | 0 | Random | 94.18 |
| 300 | 86 | 1 | Random | 95.02 |
| 300 | 86 | 2 | Random | 93.9 |

(continued)

| Number of Probes | Probe Set ID | Experiment Repetition | Selection Type | Sensitivity |
|---|---|---|---|---|
| 300 | 86 | 3 | Random | 94.58 |
| 300 | 86 | 4 | Random | 94.8 |
| 300 | 87 | 0 | Random | 95.18 |
| 300 | 87 | 1 | Random | 95.52 |
| 300 | 87 | 2 | Random | 95.38 |
| 300 | 87 | 3 | Random | 95.7 |
| 300 | 87 | 4 | Random | 94.72 |
| 300 | 88 | 0 | Random | 94.52 |
| 300 | 88 | 1 | Random | 93.7 |
| 300 | 88 | 2 | Random | 94.36 |
| 300 | 88 | 3 | Random | 94.14 |
| 300 | 88 | 4 | Random | 95.1 |
| 300 | 89 | 0 | Random | 93.62 |
| 300 | 89 | 1 | Random | 94.8 |
| 300 | 89 | 2 | Random | 94.1 |
| 300 | 89 | 3 | Random | 94.96 |
| 300 | 89 | 4 | Random | 94.68 |
| 300 | 90 | 0 | Random | 94.6 |
| 300 | 90 | 1 | Random | 94.04 |
| 300 | 90 | 2 | Random | 94.14 |
| 300 | 90 | 3 | Random | 94.36 |
| 300 | 90 | 4 | Random | 94.24 |
| 300 | 91 | 0 | Random | 94.12 |
| 300 | 91 | 1 | Random | 94.32 |
| 300 | 91 | 2 | Random | 93.7 |
| 300 | 91 | 3 | Random | 94.56 |
| 300 | 91 | 4 | Random | 94.68 |
| 300 | 92 | 0 | Random | 95.06 |
| 300 | 92 | 1 | Random | 94.06 |
| 300 | 92 | 2 | Random | 95.48 |
| 300 | 92 | 3 | Random | 95.48 |
| 300 | 92 | 4 | Random | 95.24 |
| 300 | 93 | 0 | Random | 93.46 |
| 300 | 93 | 1 | Random | 94.4 |
| 300 | 93 | 2 | Random | 93.62 |
| 300 | 93 | 3 | Random | 94.72 |
| 300 | 93 | 4 | Random | 95.16 |
| 300 | 94 | 0 | Random | 95 |

(continued)

| Number of Probes | Probe Set ID | Experiment Repetition | Selection Type | Sensitivity |
|---|---|---|---|---|
| 300 | 94 | 1 | Random | 94.74 |
| 300 | 94 | 2 | Random | 94.1 |
| 300 | 94 | 3 | Random | 94.26 |
| 300 | 94 | 4 | Random | 95.02 |
| 300 | 95 | 0 | Random | 94.94 |
| 300 | 95 | 1 | Random | 94.6 |
| 300 | 95 | 2 | Random | 93.9 |
| 300 | 95 | 3 | Random | 95.16 |
| 300 | 95 | 4 | Random | 94.14 |
| 300 | 96 | 0 | Random | 95.08 |
| 300 | 96 | 1 | Random | 94.54 |
| 300 | 96 | 2 | Random | 94.6 |
| 300 | 96 | 3 | Random | 95.14 |
| 300 | 96 | 4 | Random | 93.88 |
| 300 | 97 | 0 | Random | 93.66 |
| 300 | 97 | 1 | Random | 94.32 |
| 300 | 97 | 2 | Random | 93.76 |
| 300 | 97 | 3 | Random | 94.1 |
| 300 | 97 | 4 | Random | 93.64 |
| 300 | 98 | 0 | Random | 95.48 |
| 300 | 98 | 1 | Random | 94.34 |
| 300 | 98 | 2 | Random | 94.96 |
| 300 | 98 | 3 | Random | 94.74 |
| 300 | 98 | 4 | Random | 95.28 |
| 300 | 99 | 0 | Random | 93.86 |
| 300 | 99 | 1 | Random | 94.2 |
| 300 | 99 | 2 | Random | 94.98 |
| 300 | 99 | 3 | Random | 94.38 |
| 300 | 99 | 4 | Random | 94.44 |

*Table 7c - 300 affinity reagents targeting the most-common trimers in the proteome*

| Number of Probes | Probe Set ID | Experiment Repetitions | Selection Type | Sensitivity |
|---|---|---|---|---|
| 300 | 101 | 0 | Top 300 | 97.98 |
| 300 | 101 | 1 | Top 300 | 97.24 |
| 300 | 101 | 2 | Top 300 | 97.94 |
| 300 | 101 | 3 | Top 300 | 98.18 |
| 300 | 101 | 4 | Top 300 | 97.12 |

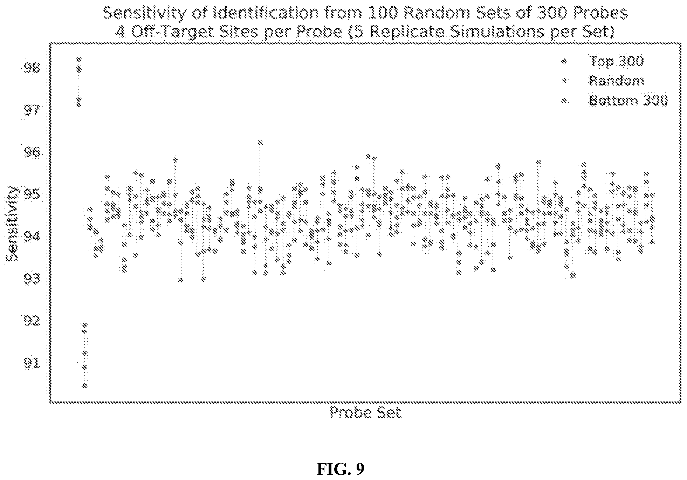

These results are plotted in **FIG. 9.** In all cases, each affinity reagent had a binding probability of 0.25 to the targeted trimer, and a binding probability of 0.25 to 4 additional randomly selected trimers. The performance of each affinity reagent set is measured based on sensitivity (e.g., the percentage of proteins identified). Each affinity reagent set was assessed in 5 replicates, with the performance of each replicate plotted as a dot, and a vertical line connecting replicate measurements from the same set of affinity reagents. The results from the affinity reagent set consisting of the top 300 most abundant affinity reagents is in blue, the bottom 300 in green. A total of 100 different sets of 300 affinity reagents targeting random trimers were generated and assessed. Each of those sets is represented by a set of 5 grey points (one for each replicate) connected by a vertical grey line. According to the uncensored inference used in this analysis, targeting more abundant trimers improves identification performance as compared to targeting random trimers.

**Example 10: Affinity reagents with biosimilar off target sites**

The methods described herein may be applied to affinity reagent binding experiment with affinity reagents having different types of off-target binding sites (epitopes). In this example, performance with two classes of affinity reagents are compared: random, and "biosimilar" affinity reagents. The results from these assessments are shown in Tables 8a-8d.

**Tables 8a-d**

| *Table 8a - Performance of Censored Inference with Affinity Reagents having Biosimilar Off-Target Sites and Targeting the 300 Most-Abundant Trimers in the Proteome* | | | |
|---|---|---|---|
| **Censored** | **Number of Cycles** | **Probe Type** | **Sensitivity** |
| TRUE | 100 | Biosimilar | 0.00634 |
| TRUE | 200 | Biosimilar | 31.97667 |
| TRUE | 300 | Biosimilar | 68.73336 |
| *Table 8b - Performance of Uncensored Inference with Affinity Reagents having Biosimilar Off Target Sites and Targeting the 300 Most-Abundant Trimers in the Proteome* | | | |
| **Censored** | **Number of Cycles** | **Probe Type** | **Sensitivity** |
| FALSE | 100 | Biosimilar | 75.67516 |
| FALSE | 200 | Biosimilar | 97.68607 |
| FALSE | 300 | Biosimilar | 99.06809 |
| *Table 8c - Performance of Censored Inference with Affinity Reagents having Random Off-Target Sites and Targeting the 300 Most-Abundant Trimers in the Proteome* | | | |
| **Censored** | **Number of Cycles** | **Probe Type** | **Sensitivity** |
| TRUE | 100 | Random | 0.082414 |
| TRUE | 200 | Random | 74.68619 |
| TRUE | 300 | Random | 93.13427 |
| *Table 8d - Performance of Uncensored Inference with Affinity Reagents having Random Off-Target Sites and Targeting the 300 Most Abundant Trimers in the Proteome* | | | |
| **Censored** | **Number of Cycles** | **Probe Type** | **Sensitivity** |
| FALSE | 100 | Random | 60.02916 |
| FALSE | 200 | Random | 95.47356 |
| FALSE | 300 | Random | 98.51021 |

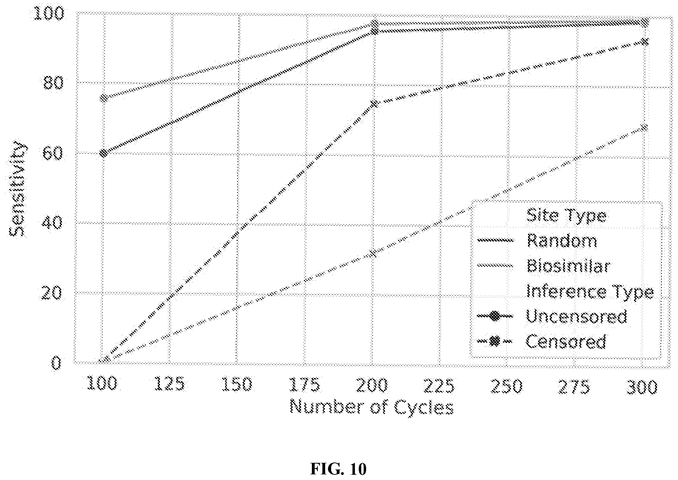

Unlike the random affinity reagents, the biosimilar affinity reagents have off-target binding sites that are biochemically similar to the targeted epitope. Both the random and biosimilar affinity reagents recognize their target epitope (e.g., a trimer) with binding probability 0.25. Each of the random class of affinity reagents has 4 randomly selected off-target trimer binding sites with binding probability 0.25. In contrast, the 4 off-target binding sites for the "biosimilar" affinity reagents are the four trimers most similar to the trimer targeted by the affinity reagent, which are bound with probability 0.25. For these biosimilar affinity reagents, the similarity between trimer sequences is computed by summing the BLOSUM62 coefficient for the amino acid pair at each sequence location. Both the random and biosimilar affinity reagent sets target the top 300 most abundant trimers in the human proteome, where abundance is measured as the number of unique proteins containing one or more instances of the trimer. **FIG. 10** shows the performance of the censored (dashed lines) and uncensored (solid lines) protein inference approaches in terms of the percent of proteins identified in a human sample when affinity reagents with random (blue) or biosimilar (orange) off-target sites are used.

In this comparison, uncensored inference outperforms censored inference, with uncensored inference performing better in the case of biosimilar affinity reagents, and censored inference performing better in the case of random affinity reagents.

Alternatively, rather than using affinity reagents targeting the most abundant trimers in the proteome, an optimal set of trimer targets may be chosen for a particular approach based on the candidate proteins that may be measured (for example, the human proteome), the type of protein inference being performed (censored or uncensored), and the type of affinity reagents being used (random or biosimilar). A "greedy" algorithm, as described below, may be used to select a set of optimal affinity reagents:

1) Initialize an empty list of selected affinity reagents (AR).
2) Initialize a set of candidate ARs (e.g., a collection of 8,000 ARs, each targeting a unique trimer with random off-target sites).
3) Select a set of protein sequences to optimize against (e.g., all human proteins in the Uniprot reference proteome).
4) Repeat the following until the desired number of ARs has been selected:

   a. For each candidate AR:

      i. Simulate binding of the candidate AR against the protein set.
      ii. Perform protein inference for each protein using the simulated binding measurements from the candidate AR and the simulated binding measurements from all previously selected ARs.
      iii. Calculate a score for the candidate AR by summing up the probability of the correct protein identification for each protein determined by protein inference.

   b. Add the AR with the highest score to the set of selected ARs, and remove it from the candidate AR list.

The greedy approach was used to select 300 optimal affinity reagents from either the collection of random affinity reagents or biosimilar affinity reagents targeting the top 4,000 most abundant trimers in the human proteome. The optimization was performed for both censored protein inference and uncensored protein inference. The results from these optimizations are provided in Tables 9a-9d.

**Tables 9a-d**

| Table 9a - Performance of Censored Inference with Affinity Reagents having Biosimilar Off-Target Sites and Targeting the 300 Optimal Trimers in the Proteome | | | |
|---|---|---|---|
| **Censored** | **Number of Cycles** | **Probe Type** | **Sensitivity** |
| TRUE | 100 | Biosimilar | 25.58007 |
| TRUE | 200 | Biosimilar | 87.82173 |
| TRUE | 300 | Biosimilar | 95.15025 |

| Table 9b - Performance of Uncensored Inference with Affinity Reagents having Biosimilar Off Target Sites and Targeting the 300 Optimal Trimers in the Proteome | | | |
|---|---|---|---|
| **Censored** | **Number of Cycles** | **Probe Type** | **Sensitivity** |
| FALSE | 100 | Biosimilar | 76.76556 |
| FALSE | 200 | Biosimilar | 97.2106 |
| FALSE | 300 | Biosimilar | 99.03005 |

| Table 9c - Performance of Censored Inference with Affinity Reagents having Random Off-Target Sites and Targeting the 300 Optimal Trimers in the Proteome | | | |
|---|---|---|---|
| **Censored** | **Number of Cycles** | **Probe Type** | **Sensitivity** |
| TRUE | 100 | Random | 24.93343 |

(continued)

| Table 9c - Performance of Censored Inference with Affinity Reagents having Random Off-Target Sites and Targeting the 300 Optimal Trimers in the Proteome | | | |
|---|---|---|---|
| **Censored** | **Number of Cycles** | **Probe Type** | **Sensitivity** |
| TRUE | 200 | Random | 88.06263 |
| TRUE | 300 | Random | 95.8476 |

| Table 9d - Performance of Uncensored Inference with Affinity Reagents having Random Off-Target Sites and Targeting the 300 Optimal Trimers in the Proteome | | | |
|---|---|---|---|
| **Censored** | **Number of Cycles** | **Probe Type** | **Sensitivity** |
| FALSE | 100 | Random | 65.72841 |
| FALSE | 200 | Random | 96.38012 |
| FALSE | 300 | Random | 98.56092 |

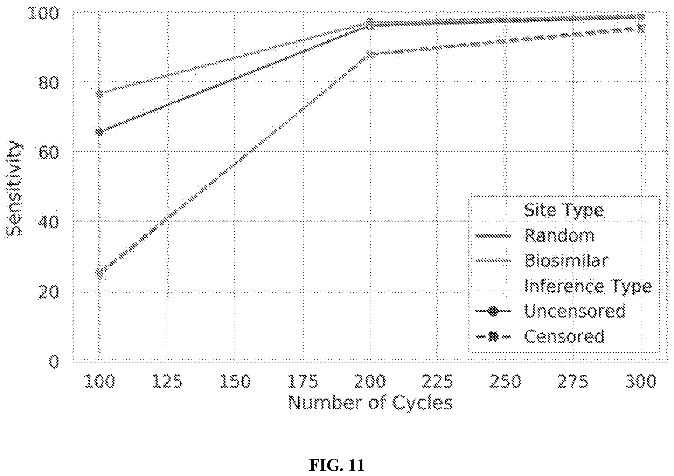

The performance of the optimized probe sets for both censored protein inference and uncensored protein inference are plotted in **FIG. 11.**

Using the set of affinity reagents selected by the greedy optimization algorithm improves the performance of both random and biosimilar affinity reagent sets using both censored protein inference and uncensored protein inference approaches. Additionally, random affinity reagents sets perform almost identically to biosimilar affinity reagents sets when the greedy approach is used to select affinity reagents.

**Example 11: Protein inference using binding of mixtures of affinity reagents**

The methods described herein may be applied to analyze and/or identify proteins that have been measured using mixtures of affinity reagents. The probability of a specific protein generating a binding outcome when assayed by a mixture of affinity reagents may be computed as follows:

1) Calculate $\overline{p_{ns}}$, the average probability of non-specific epitope binding of each affinity reagent in the mixture.

2) Calculate the number of binding sites on the protein based on the length of the protein (L) and the length of the affinity reagent epitopes (K): Num binding sites = L - K + 1 . The probability of no non-specific binding events occurring is $(1 - \overline{p_{ns}})^{L-K+1}$.

3) For each affinity reagent in the mixture, calculate the probability of no epitope-specific binding events occurring:

$$P\_no\_spec\_bind(AR)$$
$$= \prod_{epitope} (1 - epitope\ binding\ probabilty)^{epitope\ count\ in\ protein}$$

4) The probability of the mixture generating a non-binding outcome for the protein is:

$$P(no\ bind \mid protein) = (1 - \overline{p_{ns}})^{L-K+1} \prod_{AR} P\_no\_spec\_bind(AR)$$

5) The probability of the mixture generating a binding outcome is:

$$P(bind \mid protein) = 1 - P(no\ bind \mid protein)$$

This approach for calculating the probability of a binding or non-binding outcome from a protein mixture was used in combination with the methods described herein to analyze the performance of mixtures of affinity reagents for protein identification. Each individual affinity reagent in the analysis binds to its targeted trimer epitope with a probability of 0.25 and the 4 most similar trimers to that epitope target with a probability of 0.25. For these affinity reagents, trimer similarity is calculated by summing the coefficients from the BLOSUM62 substitution matrix for the amino acids at each sequence location in the trimers being compared. Additionally, each affinity reagent binds 20 additional off-target sites with binding probability scaled depending on the sequence similarity between the off-target site and the targeted trimer calculated using the BLOSUM62 substitution matrix. The probability for these additional off target sites is: $0.25 * 1.5^{S_{OT}-S_{self}}$ where $S_{OT}$ is the BLOSUM62 similarity between the off-target site and the targeted site, and $S_{self}$ is the BLOSUM62 similarity between the targeted sequence and itself. Any off-target sites with binding probability below $2.45 \times 10^8$ are adjusted to have binding probability $2.45 \times 10^8$. The non-specific epitope binding probability is $2.45 \times 10^8$ in this example.

An optimal set of 300 mixtures of affinity reagents were generated for both censored and uncensored protein inference using a greedy approach:

1) Initialize an empty list of selected affinity reagent (AR) mixtures.
2) Initialize a list of candidate affinity reagents (in this example, consisting of the 300 most optimal computed using the greedy approach detailed in **Example 10**).
3) Select a set of protein sequences to optimize against (e.g., all human proteins in the Uniprot reference proteome).
4) Repeat the following until the desired number of AR mixtures has been generated:

   a. Initialize an empty mixture.
   b. For each candidate AR:

      i. Simulate binding outcomes using the current mixture with the candidate AR added to it.
      ii. Perform protein inference for each protein using the simulated binding measurements from i. and simulated binding measurements from previously generated mixtures.
      iii. Calculate a score for the mixture with this candidate AR by summing up the probability of the correct protein identification for each protein as determined by protein inference.

   c. Add the highest scoring candidate AR to the mixture.
   d. For each candidate AR not already in the mixture, score the mixture with the addition of the AR, as in i-iii, and if the highest scoring candidate has a higher score than the previous candidate added to the mixture, add it to the mixture and repeat this step. The mixture is complete when the best scoring candidate AR reduces the score of the mixture relative to the previously added candidate or when all candidate ARs have been added to the mixture.

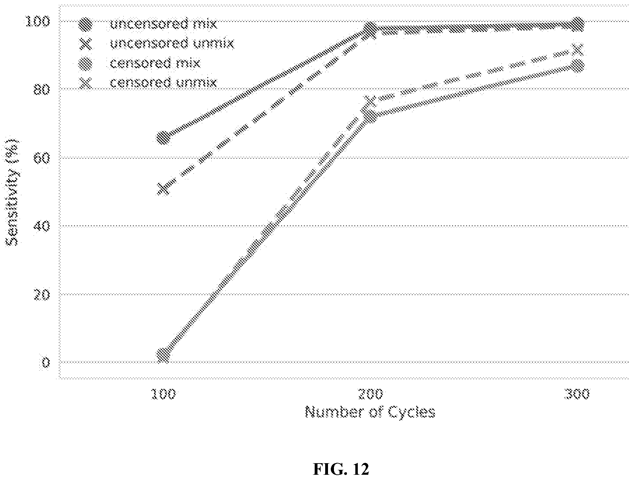

**FIG. 12** shows the protein identification sensitivity when the unmixed candidate affinity reagents are used with censored protein inference and uncensored protein inference, and when mixtures are used. The data plotted in FIG. 12 is shown in Tables 10a-10b.

**Tables 10a-b**

| Table 10a - Performance of Censored Inference with Measurements Made on Individual Probe Binding (unmix) or Mixtures of Probes (mix) | | | | |
|---|---|---|---|---|
| **Censored** | **Mix Type** | **Number of Cycles** | **Probe Type** | **Sensitivity** |
| TRUE | mix | 100 | Biosimilar | 2.244199 |
| TRUE | unmix | 100 | Biosimilar | 1.363002 |
| TRUE | mix | 200 | Biosimilar | 72.16939 |
| TRUE | unmix | 200 | Biosimilar | 76.51198 |
| TRUE | mix | 300 | Biosimilar | 86.91518 |
| TRUE | unmix | 300 | Biosimilar | 91.5684 |

| Table 10b - Performance of Uncensored Inference with Measurements Made on Individual Probe Binding (unmix) or Mixtures of Probes (mix) | | | | |
|---|---|---|---|---|
| **Censored** | **Mix Type** | **Number of Cycles** | **Probe Type** | **Sensitivity** |
| FALSE | mix | 100 | Biosimilar | 65.76011 |

(continued)

| Table 10b - Performance of Uncensored Inference with Measurements Made on Individual Probe Binding (unmix) or Mixtures of Probes (mix) | | | | |
|---|---|---|---|---|
| Censored | Mix Type | Number of Cycles | Probe Type | Sensitivity |
| FALSE | unmix | 100 | Biosimilar | 50.79244 |
| FALSE | mix | 200 | Biosimilar | 97.81286 |
| FALSE | unmix | 200 | Biosimilar | 96.30404 |
| FALSE | mix | 300 | Biosimilar | 99.14416 |
| FALSE | unmix | 300 | Biosimilar | 98.56726 |

The use of mixtures improves performance when uncensored inference is used but may negatively impact performance if censored inference is used.

**Example 12 - Glycan identification with a database of 7 candidate glycans**

Consider a situation where a database contains 7 candidate glycans:

| ID | Structure |
|---|---|
| 19 | Galb1-4GlcNAcb1-6(Galb1-4GlcNAcb1-3)GalNAc |
| 52 | GlcNAcb1-2Mana1-6(GlcNAcb1-2Mana1-3)Manb1-4GlcNAcb1-4GlcNAc |
| 344 | GlcNAca1-4Galb1-3GalNAc |
| 378 | Neu5Aca2-3Galb1-4(Fuca1-3)GlcNAcb1-3GalNAc |
| 430 | Fuca1-3GlcNAcb1-6(Galb1-4GlcNAcb1-3)Galb1-4Glc |
| 519 | GalNAca1-3(Fuca1-2)Galb1-4GlcNAcb1-6GalNAc |
| 534 | Neu5Aca2-3Galb1-4(Fuca1-3)GlcNAcb1-2Man |

Additionally, the experiment is performed with 4 affinity reagents (AR), each of which has a 25% likelihood of binding a given disaccharide. The other disaccharides these reagents bind to are not found in any glycan in the database.
A hit table is constructed for the affinity reagents to each sequence in the database (Row = affinity reagents #1 to #4, Col = SEQ ID)

| AR Target | 19 | 52 | 344 | 378 | 430 | 519 | 534 |
|---|---|---|---|---|---|---|---|
| Neu5Aca2-3Gal | | | | 1 | | | 1 |
| GlcNAcb1-2Man | | 2 | | | | | 1 |
| Fuca1-3GlcNAc | | | | 1 | 1 | | 1 |
| Galb1-4GlcNAc | 2 | | | 1 | 1 | 1 | 1 |

Notably, this information arrives incrementally, and therefore may be computed iteratively. From the hit table, $P(glycan\_i \mid AR\_j)$ is evaluated to generate a probability matrix, as shown below. Note that for a given entry, if hit table >= 1, then use P_landing_AR_n = true landing rate = 0.25 ; else if hit table = 0, use P(detector error) = 0.00001

| | 19 | 52 | 344 | 378 | 430 | 519 | 534 |
|---|---|---|---|---|---|---|---|
| Neu5Aca2-3 Gal | 1.00E-05 | 1.00E-05 | 1.00E-05 | 0.25 | 1.00E-05 | 1.00E-05 | 0.25 |
| GlcNAcb1-2Man | 1.00E-05 | 0.25 | 1.00E-05 | 1.00E-05 | 1.00E-05 | 1.00E-05 | 0.25 |
| Fuca1-3GlcNAc | 1.00E-05 | 1.00E-05 | 1.00E-05 | 0.25 | 0.25 | 1.00E-05 | 0.25 |

(continued)

| | 19 | 52 | 344 | 378 | 430 | 519 | 534 |
|---|---|---|---|---|---|---|---|
| Galb1-4GlcNAc | 0.25 | 1.00E-05 | 1.00E-05 | 0.25 | 0.25 | 0.25 | 0.25 |

Note that many of the cells contain a 0.00001 probability. This small probability accounts for possible detector error. The initial, un-normalized probability of a glycan is calculated as the product of the probabilities for each candidate glycan:

| 19 | 52 | 344 | 378 | 430 | 519 | 534 |
|---|---|---|---|---|---|---|
| 2.5E-16 | 2.5E-16 | 1E-20 | 1.5625E-07 | 6.25E-12 | 2.5E-16 | 0.00390625 |

Next, the size normalization is computed, which refers to the number of ways some number of affinity reagents may land on a given glycan, as a function of the number of potential binding sites of the glycan. The size normalization is given by the Choose(sites_i, n) term. For example, candidate ID 52 has 6 disaccharide sites and a size normalization of [6 choose 4] which is 15. If there are more binding events than the number of available disaccharide sites, the size normalization factor is set to 1. The un-normalized probabilities of each glycan are normalized to take into account this size correction by dividing by the size normalization which gives:

| 19 | 52 | 344 | 378 | 430 | 519 | 534 |
|---|---|---|---|---|---|---|
| 2.5E-16 | 1.6667E-17 | 1E-20 | 1.5625E-07 | 1.25E-12 | 2.5E-16 | 0.00390625 |

Next, the probabilities are normalized such that the entire set of probabilities over the entire database sums up to one. This is achieved by summing the size-normalized probabilities to 0.00390641 and dividing each of the size-normalized probabilities by this normalization to achieve the final balanced probabilities:

| 19 | 52 | 344 | 378 | 430 | 519 | 534 |
|---|---|---|---|---|---|---|
| 6.39974E-14 | 4.2665E-15 | 2.5599E-18 | 3.9998E-05 | 3.1999E-10 | 6.3997E-14 | 0.99996 |

CLAUSES

1. A computer-implemented method for iteratively identifying candidate proteins within a sample of unknown proteins, the method comprising:

(a) receiving, by said computer, information of binding measurements of each of a plurality of affinity reagent probes to said unknown proteins in said sample, each affinity reagent probe configured to selectively bind to one or more candidate proteins among a plurality of candidate proteins;
(b) comparing, by said computer, at least a portion of said information of binding measurements against a database comprising a plurality of protein sequences, each protein sequence corresponding to a candidate protein among said plurality of candidate proteins; and
(c) for each of one or more candidate proteins in said plurality of candidate proteins, iteratively generating, by said computer, a probability that said each of one or more candidate proteins is present in said sample based on said comparison of said at least a portion of said information of binding measurements of said each of one or more candidate proteins against said database comprising said plurality of protein sequences.

2. The method of clause 1, wherein generating said plurality of probabilities further comprises iteratively receiving additional information of binding measurements of each of a plurality of additional affinity reagent probes, each additional affinity reagent probe configured to selectively bind to one or more candidate proteins among said plurality of candidate proteins.

3. The method of clause 1, further comprising generating, for said each of one or more candidate proteins, a confidence level that said candidate protein matches one of said unknown proteins in said sample.

4. The method of clause 1, wherein generating said probability comprises taking into account a detector error rate associated with said information of binding measurements.

5. The method of clause 4, wherein said detector error rate is obtained from specifications of one or more detectors used to acquire said information of binding measurements.

6. The method of clause 4, wherein said detector error rate is set to an estimated detector error rate.

7. The method of clause 6, wherein said estimated detector error rate is set by a user of said computer.

8. The method of clause 6, wherein said estimated detector error rate is about 0.001.

9. The method of clause 1, wherein iteratively generating said plurality of probabilities further comprises removing one or more candidate proteins from said plurality of candidate proteins from subsequent iterations, thereby reducing a number of iterations necessary to perform said iterative generation of said probabilities.

10. The method of clause 9, wherein removing said one or more candidate proteins is based at least on a predetermined criterion of said binding measurements associated with said candidate proteins.

11. The method of clause 10, wherein said predetermined criterion comprises said one or more candidate proteins having binding measurements to a first plurality among said plurality of affinity reagent probes below a predetermined threshold.

12. The method of clause 1, wherein each of said probabilities is normalized to a length of said candidate protein.

13. The method of clause 1, wherein each of said probabilities are normalized to a total sum of probabilities of said plurality of candidate proteins.

14. The method of clause 1, wherein said plurality of affinity reagent probes comprises no more than 50 affinity reagent probes.

15. The method of clause 1, wherein said plurality of affinity reagent probes comprises no more than 100 affinity reagent probes.

16. The method of clause 1, wherein said plurality of affinity reagent probes comprises no more than 500 affinity reagent probes.

17. The method of clause 1, wherein said plurality of affinity reagent probes comprises more than 500 affinity reagent probes.

18. The method of clause 1, wherein said probabilities are iteratively generated until a predetermined condition is satisfied.

19. The method of clause 18, wherein said predetermined condition comprises generating each of the plurality of probabilities with a confidence of at least 90%.

20. The method of clause 19, wherein said predetermined condition comprises generating each of said plurality of probabilities with a confidence of at least 95%.

21. The method of clause 20, wherein said predetermined condition comprises generating each of said plurality of probabilities with a confidence of at least 99%.

22. The method of clause 1, further comprising generating a paper or electronic report identifying one or more unknown proteins in said sample.

23. The method of clause 1, wherein said sample comprises a biological sample.

24. The method of clause 23, wherein said biological sample is obtained from a subject.

25. The method of clause 24, further comprising identifying a disease state in said subject based at least on said plurality of probabilities.

26. A computer-implemented method for identifying candidate proteins within a sample of unknown proteins, the method comprising:

(a) receiving, by said computer, information of binding measurements of each of a plurality of affinity reagent probes to said unknown proteins in said sample, each affinity reagent probe configured to selectively bind to one or more candidate proteins among a plurality of candidate proteins;
(b) comparing, by said computer, at least a portion of said information of binding measurements against a database comprising a plurality of protein sequences, each protein sequence corresponding to a candidate protein among said plurality of candidate proteins; and
(c) removing one or more candidate proteins from said plurality of candidate proteins based at least on said comparison of said at least a portion of said information of binding measurements against said database comprising said plurality of protein sequences.

27. The method of clause 26, wherein removing said one or more candidate proteins is based at least on a predetermined criterion of said binding measurements associated with said candidate proteins.

28. The method of clause 27, wherein said predetermined criterion comprises said one or more candidate proteins having binding measurements to a first plurality among said plurality of affinity reagent probes below a predetermined threshold.

29. The method of clause 26, wherein said plurality of affinity reagent probes comprises no more than 50 affinity reagent probes.

30. The method of clause 26, wherein said plurality of affinity reagent probes comprises no more than 100 affinity reagent probes.

31. The method of clause 26, wherein said plurality of affinity reagent probes comprises no more than 500 affinity reagent probes.

32. The method of clause 26, wherein said plurality of affinity reagent probes comprises more than 500 affinity reagent probes.

33. The method of clause 26, further comprising generating a paper or electronic report identifying one or more unknown proteins in said sample.

34. The method of clause 26, wherein said sample comprises a biological sample.

35. The method of clause 34, wherein said biological sample is obtained from a subject.

36. The method of clause 35, further comprising identifying a disease state in said subject based at least on said identified candidate proteins.

37. A computer-implemented method for iteratively identifying candidate glycans within a sample of unknown glycans, the method comprising:

(a) receiving, by said computer, binding measurements of each of a plurality of affinity reagent probes to said unknown glycans in said sample, each affinity reagent probe configured to selectively bind to one or more candidate glycans among a plurality of candidate glycans;

(b) comparing, by said computer, binding measurements against a database comprising a plurality of glycan sequences, each glycan sequence corresponding to a candidate glycan among said plurality of candidate glycans; and

(c) for each of one or more candidate glycans in said plurality of candidate glycans, iteratively generating, by said computer, a probability that said each of one or more candidate glycans is present in said sample based on said comparison of said binding measurements against said database comprising a plurality of glycan sequences that each correspond to a candidate glycan among said plurality of candidate glycans.

38. The method of clause 37, wherein generating said plurality of probabilities further comprises iteratively receiving additional information of binding measurements of each of a plurality of additional affinity reagent probes, each additional affinity reagent probe configured to selectively bind to one or more candidate glycans among said plurality of candidate glycans.

39. The method of clause 37, further comprising generating, for said each of one or more candidate glycans, a confidence level that said candidate glycan matches one of said unknown glycans in said sample.

40. The method of clause 37, wherein generating said probability comprises taking into account a detector error rate associated with said information of binding measurements.

41. The method of clause 40, wherein said detector error rate is obtained from specifications of one or more detectors used to acquire said information of binding measurements.

42. The method of clause 40, wherein said detector error rate is set to an estimated detector error rate.

43. The method of clause 42, wherein said estimated detector error rate is set by a user of said computer.

44. The method of clause 42, wherein said estimated detector error rate is about 0.001.

45. The method of clause 37, wherein iteratively generating said plurality of probabilities further comprises removing one or more candidate glycans from said plurality of candidate glycans from subsequent iterations, thereby reducing a number of iterations necessary to perform said iterative generation of said probabilities.

46. The method of clause 45, wherein removing said one or more candidate glycans is based at least on a predetermined criterion of said binding measurements associated with said candidate glycans.

47. The method of clause 46, wherein said predetermined criterion comprises said one or more candidate glycans having binding measurements to a first plurality among said plurality of affinity reagent probes below a predetermined threshold.

48. The method of clause 37, wherein each of said probabilities is normalized to a number of potential binding sites of said candidate glycan.

49. The method of clause 37, wherein each of said probabilities are normalized to a total sum of probabilities of said plurality of candidate glycans.

50. The method of clause 37, wherein said plurality of affinity reagent probes comprises no more than 50 affinity reagent probes.

51. The method of clause 37, wherein said plurality of affinity reagent probes comprises no more than 100 affinity reagent probes.

52. The method of clause 37, wherein said plurality of affinity reagent probes comprises no more than 500 affinity reagent probes.

53. The method of clause 37, wherein said plurality of affinity reagent probes comprises more than 500 affinity reagent probes.

54. The method of clause 37, wherein said probabilities are iteratively generated until a predetermined condition is satisfied.

55. The method of clause 54, wherein said predetermined condition comprises generating each of the plurality of probabilities with a confidence of at least 90%.

56. The method of clause 55, wherein said predetermined condition comprises generating each of said plurality of probabilities with a confidence of at least 95%.

57. The method of clause 56, wherein said predetermined condition comprises generating each of said plurality of probabilities with a confidence of at least 99.999%.

58. The method of clause 37, further comprising generating a paper or electronic report identifying one or more unknown glycans in said sample.

59. The method of clause 37, wherein said sample comprises a biological sample.

60. The method of clause 59, wherein said biological sample is obtained from a subject.

61. The method of clause 60, further comprising identifying a disease state in said subject based at least on said plurality of probabilities.

62. A computer-implemented method for identifying candidate glycans within a sample of unknown glycans, the method comprising:

> (a) receiving, by said computer, binding measurements of each of a plurality of affinity reagent probes to said unknown glycans in said sample, each affinity reagent probe configured to selectively bind to one or more candidate glycans among a plurality of candidate glycans;
> (b) comparing, by said computer, at least a portion of said binding measurements against a database comprising a plurality of glycan sequences, each glycan sequence corresponding to a candidate glycan among said plurality of candidate glycans; and
> (c) removing one or more candidate glycans from said plurality of candidate glycans based at least on said comparison of said at least a portion of said information of binding measurements against said database comprising said plurality of glycan sequences.

63. The method of clause 62, wherein removing said one or more candidate glycans is based at least on a predetermined criterion of said binding measurements associated with said candidate glycans.

64. The method of clause 63, wherein said predetermined criterion comprises said one or more candidate glycans having binding measurements to a first plurality among said plurality of affinity reagent probes below a predetermined threshold.

65. The method of clause 62, wherein said plurality of affinity reagent probes comprises no more than 50 affinity reagent probes.

66. The method of clause 62, wherein said plurality of affinity reagent probes comprises no more than 100 affinity reagent probes.

67. The method of clause 62, wherein said plurality of affinity reagent probes comprises no more than 500 affinity reagent probes.

68. The method of clause 62, wherein said plurality of affinity reagent probes comprises more than 500 affinity reagent probes.

69. The method of clause 62, further comprising generating a paper or electronic report identifying one or more unknown glycans in said sample.

70. The method of clause 62, wherein said sample comprises a biological sample.

71. The method of clause 70, wherein said biological sample is obtained from a subject.

72. The method of clause 71, further comprising identifying a disease state in said subject based at least on said identified candidate glycans.

73. The method of any of the previous clauses, wherein binding measurements comprise measurements of binding affinity reagents to glycans.

74. The method of any of the previous clauses, wherein binding measurements comprises measurements of non-binding affinity reagents to glycans.

75. The method of clause 57, wherein said predetermined condition comprises generating each of said plurality of probabilities with a confidence of at least 99.999999999999%.

76. The method of clause 57, wherein said predetermined condition comprises generating each of said plurality of probabilities with a confidence of at least 99.9999999999999%.

77. The method of clause 57, wherein said predetermined condition comprises generating each of said plurality of probabilities with a confidence of at least 99.99999999999999%.

78. A computer-implemented method for iteratively identifying candidate metabolites within a sample of unknown metabolites, the method comprising:

(a) receiving, by said computer, binding measurements of each of a plurality of affinity reagent probes to said unknown metabolites in said sample, each affinity reagent probe configured to selectively bind to one or more candidate metabolites among a plurality of candidate metabolites;

(b) comparing, by said computer, binding measurements against a database comprising a plurality of metabolite structures, each metabolite structure corresponding to a candidate metabolite among said plurality of candidate metabolites; and

(c) for each of one or more candidate metabolites in said plurality of candidate metabolites, iteratively generating, by said computer, a probability that said each of one or more candidate metabolites is present in said sample based on said comparison of said binding measurements against said database comprising a plurality of metabolite structures that each correspond to a candidate metabolite among said plurality of candidate metabolites.

79. The method of clause 78, wherein generating said plurality of probabilities further comprises iteratively receiving additional information of binding measurements of each of a plurality of additional affinity reagent probes, each additional affinity reagent probe configured to selectively bind to one or more candidate metabolites among said plurality of candidate metabolites.

80. The method of clause 78, further comprising generating, for said each of one or more candidate metabolites, a confidence level that said candidate metabolite matches one of said unknown metabolites in said sample.

81. The method of clause 78, wherein generating said probability comprises taking into account a detector error rate associated with said information of binding measurements.

82. The method of clause 81, wherein said detector error rate is obtained from specifications of one or more detectors used to acquire said information of binding measurements.

83. The method of clause 81, wherein said detector error rate is set to an estimated detector error rate.

84. The method of clause 83, wherein said estimated detector error rate is set by a user of said computer.

85. The method of clause 83, wherein said estimated detector error rate is about 0.001.

86. The method of clause 78, wherein iteratively generating said plurality of probabilities further comprises removing one or more candidate metabolites from said plurality of candidate metabolites from subsequent iterations, thereby reducing a number of iterations necessary to perform said iterative generation of said probabilities.

87. The method of clause 86, wherein removing said one or more candidate metabolites is based at least on a predetermined criterion of said binding measurements associated with said candidate metabolites.

88. The method of clause 87, wherein said predetermined criterion comprises said one or more candidate metabolites having binding measurements to a first plurality among said plurality of affinity reagent probes below a predetermined threshold.

89. The method of clause 78, wherein each of said probabilities is normalized to a number of potential binding sites of said candidate metabolite.

90. The method of clause 78, wherein each of said probabilities are normalized to a total sum of probabilities of said plurality of candidate metabolites.

91. The method of clause 78, wherein said plurality of affinity reagent probes comprises no more than 50 affinity reagent probes.

92. The method of clause 78, wherein said plurality of affinity reagent probes comprises no more than 100 affinity reagent probes.

93. The method of clause 78, wherein said plurality of affinity reagent probes comprises no more than 500 affinity reagent probes.

94. The method of clause 78, wherein said plurality of affinity reagent probes comprises more than 500 affinity reagent probes.

95. The method of clause 78, wherein said probabilities are iteratively generated until a predetermined condition is satisfied.

96. The method of clause 95, wherein said predetermined condition comprises generating each of the plurality of probabilities with a confidence of at least 90%.

97. The method of clause 96, wherein said predetermined condition comprises generating each of said plurality of probabilities with a confidence of at least 95%.

98. The method of clause 97, wherein said predetermined condition comprises generating each of said plurality of probabilities with a confidence of at least 99.999%.

99. The method of clause 78, further comprising generating a paper or electronic report identifying one or more unknown metabolites in said sample.

23. The method of clause 78, wherein said sample comprises a biological sample.

24. The method of clause 100, wherein said biological sample is obtained from a subj ect.

25. The method of clause 101, further comprising identifying a disease state in said subject based at least on said plurality of probabilities.

26. A computer-implemented method for identifying candidate metabolites within a sample of unknown metabolites, the method comprising:

(a) receiving, by said computer, binding measurements of each of a plurality of affinity reagent probes to said unknown metabolites in said sample, each affinity reagent probe configured to selectively bind to one or more candidate metabolites among a plurality of candidate metabolites;
(b) comparing, by said computer, at least a portion of said binding measurements against a database comprising a plurality of metabolite structures, each metabolite structure corresponding to a candidate metabolite among said plurality of candidate metabolites; and
(c) removing one or more candidate metabolites from said plurality of candidate metabolites based at least on said comparison of said at least a portion of said information of binding measurements against said database comprising said plurality of metabolite structures.

104. The method of clause 103, wherein removing said one or more candidate metabolites is based at least on a predetermined criterion of said binding measurements associated with said candidate metabolites.

105. The method of clause 104, wherein said predetermined criterion comprises said one or more candidate metabolites having binding measurements to a first plurality among said plurality of affinity reagent probes below a predetermined threshold.

106. The method of clause 103, wherein said plurality of affinity reagent probes comprises no more than 50 affinity reagent probes.

107. The method of clause 103, wherein said plurality of affinity reagent probes comprises no more than 100 affinity reagent probes.

108. The method of clause 103, wherein said plurality of affinity reagent probes comprises no more than 500 affinity reagent probes.

109. The method of clause 103, wherein said plurality of affinity reagent probes comprises more than 500 affinity reagent probes.

110. The method of clause 103, further comprising generating a paper or electronic report identifying one or more unknown metabolites in said sample.

111. The method of clause 103, wherein said sample comprises a biological sample.

112. The method of clause 111, wherein said biological sample is obtained from a subject.

113. The method of clause 112, further comprising identifying a disease state in said subject based at least on said identified candidate metabolites.

114. The method of any of the previous clauses, wherein binding measurements comprise measurements of binding affinity reagents to metabolites.

115. The method of any of the previous clauses, wherein binding measurements comprises measurements of non-binding affinity reagents to metabolites.

116. The method of clause 98, wherein said predetermined condition comprises generating each of said plurality of probabilities with a confidence of at least 99.99999%.

117. The method of clause 98, wherein said predetermined condition comprises generating each of said plurality of probabilities with a confidence of at least 99.999999%.

118. The method of clause 98, wherein said predetermined condition comprises generating each of said plurality of probabilities with a confidence of at least 99.9999999%.

119. The method of clause 98, wherein said predetermined condition comprises generating each of said plurality of probabilities with a confidence of at least 99.99999999%.

120. The method of clause 98, wherein said predetermined condition comprises generating each of said plurality of probabilities with a confidence of at least 99.99999999%.

121. The method of clause 98, wherein said predetermined condition comprises generating each of said plurality of probabilities with a confidence of at least 99.999999999%.

122. The method of clause 98, wherein said predetermined condition comprises generating each of said plurality of probabilities with a confidence of at least 99.9999999999%.

123. The method of clause 98, wherein said predetermined condition comprises generating each of said plurality of probabilities with a confidence of at least 99.99999999999%.

124. The method of clause 98, wherein said predetermined condition comprises generating each of said plurality of probabilities with a confidence of at least 99.999999999999%.

125. The method of clause 98, wherein said predetermined condition comprises generating each of said plurality of probabilities with a confidence of at least 99.9999999999999%.

126. The method of clause 98, wherein said predetermined condition comprises generating each of said plurality of probabilities with a confidence of at least 99.99999999999999%.

127. A computer-implemented method for iteratively identifying candidate glycans within a sample of unknown glycans, the method comprising:

(a) receiving, by said computer, binding measurements of each of a plurality of affinity reagent probes to said unknown glycans in said sample, each affinity reagent probe configured to selectively bind to one or more candidate glycans among a plurality of candidate glycans;

(b) comparing, by said computer, binding measurements against a database comprising a plurality of glycan structures, each glycan structure corresponding to a candidate glycan among said plurality of candidate glycans; and

(c) for each of one or more candidate glycans in said plurality of candidate glycans, iteratively generating, by said computer, a probability that said each of one or more candidate glycans is present in said sample based on said comparison of said binding measurements against said database comprising a plurality of glycan structures that each correspond to a candidate glycan among said plurality of candidate glycans.

128. The method of clause 127, wherein generating said plurality of probabilities further comprises iteratively receiving additional information of binding measurements of each of a plurality of additional affinity reagent probes, each additional affinity reagent probe configured to selectively bind to one or more candidate glycans among said plurality of candidate glycans.

129. The method of clause 127, further comprising generating, for said each of one or more candidate glycans, a confidence level that said candidate glycan matches one of said unknown glycans in said sample.

130. The method of clause 127, wherein generating said probability comprises taking into account a detector error rate associated with said information of binding measurements.

131. The method of clause 130, wherein said detector error rate is obtained from specifications of one or more detectors used to acquire said information of binding measurements.

132. The method of clause 130, wherein said detector error rate is set to an estimated detector error rate.

133. The method of clause 132, wherein said estimated detector error rate is set by a user of said computer.

134. The method of clause 132, wherein said estimated detector error rate is about 0.001.

135. The method of clause 127, wherein iteratively generating said plurality of probabilities further comprises removing one or more candidate glycans from said plurality of candidate glycans from subsequent iterations, thereby reducing a number of iterations necessary to perform said iterative generation of said probabilities.

136. The method of clause 135, wherein removing said one or more candidate glycans is based at least on a predetermined criterion of said binding measurements associated with said candidate glycans.

137. The method of clause 136, wherein said predetermined criterion comprises said one or more candidate glycans having binding measurements to a first plurality among said plurality of affinity reagent probes below a predetermined threshold.

138. The method of clause 127, wherein each of said probabilities is normalized to a number of potential binding sites of said candidate glycan.

139. The method of clause 127, wherein each of said probabilities are normalized to a total sum of probabilities of said plurality of candidate glycans.

140. The method of clause 127, wherein said plurality of affinity reagent probes comprises no more than 50 affinity reagent probes.

141. The method of clause 127, wherein said plurality of affinity reagent probes comprises no more than 100 affinity reagent probes.

142. The method of clause 127, wherein said plurality of affinity reagent probes comprises no more than 500 affinity reagent probes.

143. The method of clause 127, wherein said plurality of affinity reagent probes comprises more than 500 affinity reagent probes.

144. The method of clause 127, wherein said probabilities are iteratively generated until a predetermined condition is satisfied.

145. The method of clause 144, wherein said predetermined condition comprises generating each of the plurality of probabilities with a confidence of at least 90%.

146. The method of clause 145, wherein said predetermined condition comprises generating each of said plurality of probabilities with a confidence of at least 95%.

147. The method of clause 146, wherein said predetermined condition comprises generating each of said plurality of probabilities with a confidence of at least 99.999%.

148. The method of clause 127, further comprising generating a paper or electronic report identifying one or more unknown glycans in said sample.

149. The method of clause 127, wherein said sample comprises a biological sample.

150. The method of clause 149, wherein said biological sample is obtained from a subj ect.

151. The method of clause 150, further comprising identifying a disease state in said subject based at least on said plurality of probabilities.

152. A computer-implemented method for identifying candidate glycans within a sample of unknown glycans, the method comprising:

(a) receiving, by said computer, binding measurements of each of a plurality of affinity reagent probes to said unknown glycans in said sample, each affinity reagent probe configured to selectively bind to one or more candidate glycans among a plurality of candidate glycans;
(b) comparing, by said computer, at least a portion of said binding measurements against a database comprising a plurality of glycan structures, each glycan structure corresponding to a candidate glycan among said plurality of candidate glycans; and
(c) removing one or more candidate glycans from said plurality of candidate glycans based at least on said comparison of said at least a portion of said information of binding measurements against said database comprising said plurality of glycan structures.

153. The method of clause 152, wherein removing said one or more candidate glycans is based at least on a predetermined criterion of said binding measurements associated with said candidate glycans.

154. The method of clause 153, wherein said predetermined criterion comprises said one or more candidate glycans having binding measurements to a first plurality among said plurality of affinity reagent probes below a predetermined threshold.

155. The method of clause 152, wherein said plurality of affinity reagent probes comprises no more than 50 affinity reagent probes.

156. The method of clause 152, wherein said plurality of affinity reagent probes comprises no more than 100 affinity reagent probes.

157. The method of clause 152, wherein said plurality of affinity reagent probes comprises no more than 500 affinity reagent probes.

158. The method of clause 152, wherein said plurality of affinity reagent probes comprises more than 500 affinity reagent probes.

159. The method of clause 152, further comprising generating a paper or electronic report identifying one or more unknown glycans in said sample.

160. The method of clause 152, wherein said sample comprises a biological sample.

161. The method of clause 160, wherein said biological sample is obtained from a subj ect.

162. The method of clause 161, further comprising identifying a disease state in said subject based at least on said identified candidate glycans.

163. The method of any of the previous clauses, wherein binding measurements comprise measurements of binding affinity reagents to glycans.

164. The method of any of the previous clauses, wherein binding measurements comprises measurements of non-binding affinity reagents to glycans.

165. The method of clause 147, wherein said predetermined condition comprises generating each of said plurality of probabilities with a confidence of at least 99.99999%.

166. The method of clause 147, wherein said predetermined condition comprises generating each of said plurality of probabilities with a confidence of at least 99.999999%.

167. The method of clause 147, wherein said predetermined condition comprises generating each of said plurality of probabilities with a confidence of at least 99.9999999%.

168. The method of clause 147, wherein said predetermined condition comprises generating each of said plurality of probabilities with a confidence of at least 99.99999999%.

169. The method of clause 147, wherein said predetermined condition comprises generating each of said plurality of probabilities with a confidence of at least 99.99999999%.

170. The method of clause 147, wherein said predetermined condition comprises generating each of said plurality of probabilities with a confidence of at least 99.999999999%.

171. The method of clause 147, wherein said predetermined condition comprises generating each of said plurality of probabilities with a confidence of at least 99.9999999999%.

172. The method of clause 147, wherein said predetermined condition comprises generating each of said plurality of probabilities with a confidence of at least 99.99999999999%.

173. The method of clause 147, wherein said predetermined condition comprises generating each of said plurality of probabilities with a confidence of at least 99.999999999999%.

174. The method of clause 147, wherein said predetermined condition comprises generating each of said plurality of probabilities with a confidence of at least 99.9999999999999%.

175. The method of clause 147, wherein said predetermined condition comprises generating each of said plurality of probabilities with a confidence of at least 99.99999999999999%.

176. A computer-implemented method for iteratively identifying candidate proteins within a sample of unknown proteins, the method comprising:

(a) receiving, by said computer, binding measurements of each of a plurality of affinity reagent probes to said unknown proteins in said sample, each affinity reagent probe configured to selectively bind to one or more candidate proteins among a plurality of candidate proteins;

(b) comparing, by said computer, binding measurements against a database comprising a plurality of protein sequences, each protein sequence corresponding to a candidate protein among said plurality of candidate proteins; and

(c) for each of one or more candidate proteins in said plurality of candidate proteins, iteratively generating, by said computer, a probability that said each of one or more candidate proteins is present in said sample based on said comparison of said binding measurements against said database comprising a plurality of protein sequences that each correspond to a candidate protein among said plurality of candidate proteins.

177. The method of clause 176, wherein generating said plurality of probabilities further comprises iteratively receiving additional information of binding measurements of each of a plurality of additional affinity reagent probes, each additional affinity reagent probe configured to selectively bind to one or more candidate proteins among said plurality of candidate proteins.

178. The method of clause 176, further comprising generating, for said each of one or more candidate proteins, a confidence level that said candidate protein matches one of said unknown proteins in said sample.

179. The method of clause 176, wherein generating said probability comprises taking into account a detector error rate associated with said information of binding measurements.

180. The method of clause 179, wherein said detector error rate is obtained from specifications of one or more detectors used to acquire said information of binding measurements.

181. The method of clause 179, wherein said detector error rate is set to an estimated detector error rate.

182. The method of clause 181, wherein said estimated detector error rate is set by a user of said computer.

183. The method of clause 181, wherein said estimated detector error rate is about 0.001.

184. The method of clause 176, wherein iteratively generating said plurality of probabilities further comprises removing one or more candidate proteins from said plurality of candidate proteins from subsequent iterations, thereby reducing a number of iterations necessary to perform said iterative generation of said probabilities.

185. The method of clause 184, wherein removing said one or more candidate proteins is based at least on a predetermined criterion of said binding measurements associated with said candidate proteins.

186. The method of clause 185, wherein said predetermined criterion comprises said one or more candidate proteins having binding measurements to a first plurality among said plurality of affinity reagent probes below a predetermined threshold.

187. The method of clause 176, wherein each of said probabilities is normalized to a length of said candidate protein.

188. The method of clause 176, wherein each of said probabilities are normalized to a total sum of probabilities of said plurality of candidate proteins.

189. The method of clause 176, wherein said plurality of affinity reagent probes comprises no more than 50 affinity reagent probes.

190. The method of clause 176, wherein said plurality of affinity reagent probes comprises no more than 100 affinity reagent probes.

191. The method of clause 176, wherein said plurality of affinity reagent probes comprises no more than 500 affinity reagent probes.

192. The method of clause 176, wherein said plurality of affinity reagent probes comprises more than 500 affinity reagent probes.

193. The method of clause 176, wherein said probabilities are iteratively generated until a predetermined condition is satisfied.

194. The method of clause 193, wherein said predetermined condition comprises generating each of the plurality of probabilities with a confidence of at least 90%.

195. The method of clause 194, wherein said predetermined condition comprises generating each of said plurality of probabilities with a confidence of at least 95%.

196. The method of clause 195, wherein said predetermined condition comprises generating each of said plurality of probabilities with a confidence of at least 99.999%.

197. The method of clause 176, further comprising generating a paper or electronic report identifying one or more unknown proteins in said sample.

198. The method of clause 176, wherein said sample comprises a biological sample.

199. The method of clause 198, wherein said biological sample is obtained from a subject.

200. The method of clause 199, further comprising identifying a disease state in said subject based at least on said plurality of probabilities.

201. A computer-implemented method for identifying candidate proteins within a sample of unknown proteins, the method comprising:

(a) receiving, by said computer, binding measurements of each of a plurality of affinity reagent probes to said unknown proteins in said sample, each affinity reagent probe configured to selectively bind to one or more candidate proteins among a plurality of candidate proteins;

(b) comparing, by said computer, at least a portion of said binding measurements against a database comprising a plurality of protein sequences, each protein sequence corresponding to a candidate protein among said plurality of candidate proteins; and

(c) removing one or more candidate proteins from said plurality of candidate proteins based at least on said comparison of said at least a portion of said information of binding measurements against said database comprising said plurality of protein sequences.

202. The method of clause 201, wherein removing said one or more candidate proteins is based at least on a predetermined criterion of said binding measurements associated with said candidate proteins.

203. The method of clause 202, wherein said predetermined criterion comprises said one or more candidate proteins having binding measurements to a first plurality among said plurality of affinity reagent probes below a predetermined threshold.

204. The method of clause 201, wherein said plurality of affinity reagent probes comprises no more than 50 affinity reagent probes.

205. The method of clause 201, wherein said plurality of affinity reagent probes comprises no more than 100 affinity reagent probes.

206. The method of clause 201, wherein said plurality of affinity reagent probes comprises no more than 500 affinity reagent probes.

207. The method of clause 201, wherein said plurality of affinity reagent probes comprises more than 500 affinity reagent probes.

208. The method of clause 201, further comprising generating a paper or electronic report identifying one or more unknown proteins in said sample.

209. The method of clause 201, wherein said sample comprises a biological sample.

210. The method of clause 209, wherein said biological sample is obtained from a subject.

211. The method of clause 210, further comprising identifying a disease state in said subject based at least on said identified candidate proteins.

212. The method of any of the previous clauses 176 to 211, wherein binding measurements comprise measurements of binding affinity reagents to proteins.

213. The method of any of the previous clauses 176 to 212, wherein binding measurements comprises measurements of non-binding affinity reagents to proteins.

214. The method of clause 196, wherein said predetermined condition comprises generating each of said plurality of probabilities with a confidence of at least 99.99999%.

215. The method of clause 196, wherein said predetermined condition comprises generating each of said plurality of probabilities with a confidence of at least 99.999999%.

216. The method of clause 196, wherein said predetermined condition comprises generating each of said plurality of probabilities with a confidence of at least 99.9999999%.

217. The method of clause 196, wherein said predetermined condition comprises generating each of said plurality of probabilities with a confidence of at least 99.99999999%.

218. The method of clause 196, wherein said predetermined condition comprises generating each of said plurality of probabilities with a confidence of at least 99.99999999%.

219. The method of clause 196, wherein said predetermined condition comprises generating each of said plurality of probabilities with a confidence of at least 99.999999999%.

220. The method of clause 196, wherein said predetermined condition comprises generating each of said plurality of probabilities with a confidence of at least 99.9999999999%.

221. The method of clause 196, wherein said predetermined condition comprises generating each of said plurality of probabilities with a confidence of at least 99.99999999999%.

222. The method of clause 196, wherein said predetermined condition comprises generating each of said plurality of probabilities with a confidence of at least 99.999999999999%.

223. The method of clause 196, wherein said predetermined condition comprises generating each of said plurality of probabilities with a confidence of at least 99.9999999999999%.

224. The method of clause 196, wherein said predetermined condition comprises generating each of said plurality of probabilities with a confidence of at least 99.99999999999999%.

225. A method of reinforcing coupling between an affinity reagent and a protein, the method comprising:

attaching one or more DNA tags having a first sequence to an affinity reagent;
attaching one or more DNA tags having a second sequence to a protein;
hybridizing the affinity reagent to the protein;
hybridizing at least one DNA linker to the affinity reagent and to the protein, the DNA linker having a first region that hybridizes to the first sequence and having a second region that hybridizes to the second sequence.

226. The method of clause 225, wherein the affinity reagent has one DNA tag.

227. The method of clause 225, wherein the affinity reagent has two DNA tags.

228. The method of clause 225, wherein the affinity reagent has more than two DNA tags.

229. The method of clause 225, wherein the protein has one DNA tag.

230. The method of clause 225, wherein the protein has two DNA tags.

231. The method of clause 225, wherein the protein has more than two DNA tags.

232. The method of clause 225, wherein the protein has more than ten DNA tags.

233. The method of clause 225, wherein the affinity reagent and protein moiety is exposed to DNA linkers in a concentration between 5 picomolar and 500 nanomolar.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following clauses define the scope of the invention and that methods and structures within the scope of these clauses and their equivalents be covered thereby.

## Claims

1. A computer-implemented method for iteratively identifying candidate proteins within a sample of unknown proteins, the method comprising:

(a) receiving, by said computer, binding measurements of each of a plurality of affinity reagent probes to said unknown proteins in said sample, each affinity reagent probe configured to selectively bind to one or more candidate proteins among a plurality of candidate proteins;
(b) comparing, by said computer, binding measurements against a database comprising a plurality of protein sequences, each protein sequence corresponding to a candidate protein among said plurality of candidate proteins; and
(c) for each candidate protein in said plurality of candidate proteins, iteratively generating, by said computer, a probability that said candidate protein is present in said sample based on said comparison of said binding measurements against said database comprising a plurality of protein sequences that each correspond to a candidate protein among said plurality of candidate proteins, wherein each of said probabilities is normalized to a length of said candidate protein.

2. The method of claim 1, wherein each of said probabilities is normalized to a total sum of probabilities of said plurality, of candidate proteins.

3. The method of any preceding claim, further comprising quantifying proteins in said sample by counting the number of identifications made for each protein candidate.

4. The method of any preceding claim, further comprising generating, for each of said candidate proteins, a confidence level that said candidate protein matches one of said unknown proteins in said sample.

5. The method of any preceding claim, further comprising generating a paper or electronic report identifying one or more unknown proteins in said sample.

6. The method of any preceding claim, wherein said sample comprises a biological sample.

7. The method of claim 6, wherein said biological sample is obtained from a subject, and further comprising identifying a disease state in said subject based at least on said plurality of probabilities.

8. The method of any preceding claim, wherein proteins of the sample are treated to remove post-translational modifications.

9. The method of any preceding claim, wherein the affinity reagent probes each recognize several different epitopes.

10. The method of any preceding claim, wherein one or more of the affinity reagent probes bind amino acid motifs of a given length of 2, 3, 4, 5, 6, 7, 8, 9 or 10 amino acids.

11. The method of claim 10, wherein one or more of the affinity reagent probes bind amino acid motifs of a given length of 3 amino acids.

12. The method of any preceding claim, wherein the plurality of candidate proteins comprises at least 1000 different candidate proteins.

Receive information of binding
measurements of each a plurality of
affinity reagent probes to the unknown
proteins in the sample

~ 105

Compare information of binding
measurements against a database of
candidate protein sequences

~ 110

100

For each candidate protein,
calculate a probability that the
candidate protein is present in the
sample

~ 115

**FIG. 1**

**FIG. 2**

**FIG. 3**

**FIG. 4**

**FIG. 5**

**FIG. 6**

**FIG. 7**

**FIG. 8**

Sensitivity of Identification from 100 Random Sets of 300 Probes
4 Off-Target Sites per Probe (5 Replicate Simulations per Set)

**FIG. 9**

**FIG. 10**

**FIG. 11**

**FIG. 12**

**Step 1: Affinity Reagent Hybridization**

**Step 2: DNA Linker Hybridization**

FIG. 13

EP 4 372 383 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62575976